# EUROPEAN PATENT APPLICATION

(11) **EP 4 752 148 A1**
(43) Date of publication of application: **03.06.2026**
(21) Application number: 24461640.5
(22) Date of filing: 27.11.2024
(51) Int. Cl.: C07D 498/04, A61P 35/00, A61K 31/496, A61K 31/498, A61K 31/506, A61K 31/536, A61K 31/551

(54) **PRMT5 INHIBITORS**

(71) Applicant: Ryvu Therapeutics S.A., 30-394 Kraków (PL)
(72) Inventor: The designation of the inventor has not yet been filed
(74) Representative: Maiwald GmbH

(57) **Abstract**

The present invention relates to compounds of formula (I) and salts, stereoisomers, atrop-isomers, rotamers, tautomers or N-oxides thereof that are useful as PRMT5 inhibitors. The present invention further relates to the compounds of formula (I) for use as a medicament and to pharmaceutical compositions comprising said compounds.

## Description

### Field of the Invention

The present invention relates to compounds of formula (I) and salts, stereoisomers, atrop-isomers, rotamers, tautomers or N-oxides thereof that are useful as PRMT5 inhibitors. The present invention further relates to the compounds of formula (I) for use as a medicament and to pharmaceutical compositions comprising said compounds.

### Background of the Invention

Protein arginine methyltransferases are enzymes that catalyze the transfer of methyl groups from S-adenosylmethionine (SAM) to the arginine residues on histones and other proteins. Protein methyltransferase 5 (PRMT5) is an arginine methyltransferase that can catalyze the formation of omega-N monomethylarginine (MMA) and symmetrical dimethylarginine (SDMA) (Blanc and Richard 2017). PRMT5 conjugated with WD-repeat containing proteins (MEP50/WDR77) forms methylosome, which regulates essential cellular functions via symmetric dimethylation of target proteins involved in regulation of gene expression, RNA splicing, signal transduction, metabolism and other functions (Koh, Bezzi and Guccione 2015; Wu et al., 2021).

Homozygous deletions of p16/CDKN2a (cyclin dependent kinase inhibitor 2A) locus are prevalent in cancer and often involve co-deletion of adjacent genes. Metabolic gene MTAP (methylthioadenosine phosphorylase) is localized at the 9p21 chromosome in the close proximity to p16/CDKN2A tumor-suppressor locus. Co-deletion of MTAP may be observed in 80-90% of all tumors harboring homozygous deletion of CDKN2A, which represents 10 - 15% of all human tumors (Gao et al., 2013, Marjon et al., 2016; Firestone and Schramm 2017,). Many of these tumor types, such as non-small cell lung cancer, pancreatic adenocarcinoma, glioblastoma or mesothelioma are associated with poor prognosis, representing a significant unmet medical need.

MTAP, a critical enzyme in the methionine salvage pathway, is the only enzyme capable of degrading methylthioadenosine (MTA). Data from several genome-wide shRNA drop out screens performed on wide panels of cancer cell lines have revealed a selective requirement for PRMT5 activity in MTAP-deleted cells (Kruykov et al., 2016; Marjon et al, 2016; Markarov et al., 2016). Accumulation of MTA in cells with MTAP deletion causes a partial inhibition of the methylation activity of PRMT5, making those cells vulnerable to further PRMT5 inhibition, which in turn reduces the level of symmetric arginine dimethylation of the whole proteome, and thus an increased sensitivity of cells to modulation of the methylosome activity.

PRMT5 is a well-known essential gene regulating many cellular processes including cell growth and proliferation, apoptosis and DNA damage response. Conditional PRMT5 knockout or siRNA mediated knockdown studies indicate that significant liabilities could be associated with inhibiting PRMT5 in normal tissues. Therefore, novel approaches are required to target PRMT5 in cancerous cells while not affecting viability of normal cells (MTAP WT). MTA-cooperative PRMT5 inhibitors could provide an improved therapeutic window, by preferential binding to MTA-bound PRMT5, which is enriched in MTAP deficient tumor cells.

In view of the above, compounds acting as PRMT5 inhibitors are useful for treating one or more diseases selected from the group consisting of cancer and pre-cancerous syndromes.

Accordingly, there is a need for compounds acting as PRMT5 inhibitors, preferably for MTA-bound PRMT5, and thus provide a therapeutic impact in the treatment of diseases, in which the inhibition of PRMT5 is beneficial.

### Objects and Summary of the Invention

It is therefore an object of the present invention to provide compounds, which act as PRMT5 inhibitors, preferably with a high activity.

It is another object of the present invention to provide compounds, which are suitable for use as a medicament. It is another object of the present invention to provide compounds, which are suitable for use in the treatment of one or more diseases, in which the inhibition of PRMT5 is beneficial. It is yet another object to provide compounds, which are suitable for use in the treatment of one or more diseases selected from the group consisting of cancer and pre-cancerous syndromes.

The above objects can be achieved by the compounds of formula (I) as defined herein as well as pharmaceutical compositions comprising the same, and by the medical uses thereof.

The inventors of the present invention *inter alia* surprisingly found that the compounds of formula (I) as defined herein act as PRMT5 inhibitors, in particular for MTA-bound PRMT5. Accordingly, the compounds of formula (I) can be used as a medicament, in particular for the treatment of one or more diseases selected from the group consisting of cancer and pre-cancerous syndromes.

In a first aspect, the present invention therefore relates to a compound of formula (la*) 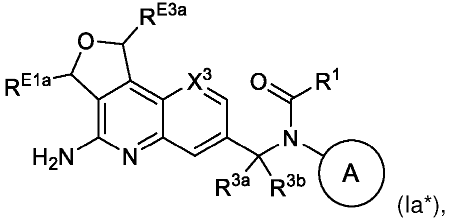 or a salt, stereoisomer, atropisomer, rotamer, tautomer, or N-oxide thereof; wherein
- A: is a moiety selected from 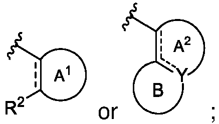
wherein
the wavy line in each case marks the connection to the N-atom of the remainder of the molecule;
and wherein
- A¹: is a 5- or 6-membered saturated, partially or fully unsaturated, or aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned rings is independently unsubstituted or substituted with one or more, same or different substituents R^{A1};
- A²: is a 5- or 6-membered saturated, partially or fully unsaturated, or aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned rings is independently unsubstituted or substituted with one or more, same or different substituents R^{A2};
and wherein the dashed lines in the rings A¹ and A² denote that an additional bond may be present, so that a double bond is formed;
and wherein
- B: is a fused morpholine ring, wherein each substitutable carbon atom of said morpholine ring is independently unsubstituted or substituted with one or more, same or different substituents R^{B};
- Y: is C;
- X³: is CH, CR^{X3}, or N;
- R¹: is a 6-membered aromatic carbocyclyl or heterocyclyl, wherein the aforementioned heterocyclic ring comprises one or more, same or different heteroatoms selected from O, N, or S, wherein said N- and/or S-atoms are independently oxidized or non-oxidized, and wherein the aforementioned rings are independently unsubstituted or substituted with one or more, preferably one, same or different substituents R^{Y};
- R²: is halogen, CN, S(=O)₂R^{S}, S(=O)(=NH)R^{S}, C(=O)R^{C}, NHC(=O)R^{C}, C(=O)NR^{N4}R^{N5}, C₁-C₄-alkoxy, C₁-C₄-haloalkyl, C₁-C₄-haloalkoxy, C₁-C₆-hydroxyalkyl,;
- R^{3a}: is H, or D;
- R^{3b}: is H, or D;
and wherein
- R^{A1}: is halogen, CN, OH, C₁-C₄-alkyl, C₁-C₄-alkoxy, C₁-C₄-haloalkyl, or 3- to 7-membered saturated carbocyclyl or heterocyclyl, wherein the aforementioned heterocyclyl ring comprises one or more, same or different heteroatoms selected from O, N, or S, wherein said N- and/or S-atoms are independently oxidized or non-oxidized; and wherein each substitutable carbon or heteroatom in the aforementioned rings is independently unsubstituted or substituted with one or more, same or different substituents R^{A3};
- R^{A2}: is halogen, CN, OH, C₁-C₄-alkyl, C₁-C₄-alkoxy, or C₁-C₄-haloalkyl;
- R^{A3}: is halogen, CN, OH, C₁-C₄-alkyl, C₁-C₄-alkoxy, or C₁-C₄-haloalkyl;
or two R^{A3} attached to the same atom form cyclopropyl;
- R^{B}: is halogen, CN, OH, C₁-C₄-alkyl, or C₁-C₄-haloalkyl;
or two R^{B} attached to the same atom form an oxo group;
- R^{C}: is H, or C₁-C₄-alkyl;
- R^{E1a}: is H, C₁-C₂-alkyl, or C₁-C₂-haloalkyl;
- R^{E3a}: is H, C₁-C₂-alkyl, or C₁-C₂-haloalkyl;
- R^{N4}: is H, or C₁-C₄-alkyl;
- R^{N5}: is H, or C₁-C₄-alkyl;
- R^{S}: is C₁-C₄-alkyl, C₁-C₄-alkoxy, C₁-C₄-haloalkyl, C₃-C₅-cycloalkyl, or NR^{N4}R^{N5};
- R^{X3}: is halogen;
- R^{Y}: is halogen, CN, OH, NR^{N4}R^{N5}, C₁-C₄-alkyl, C₁-C₄-alkoxy, C₁-C₄-haloalkyl, C₁-C₄-haloal-koxy, C₁-C₄-hydroxyalkyl, C₁-C₄-alkoxy-C₁-C₄-alkoxy, or 3- to 7-membered saturated, partially or fully unsaturated, or aromatic carbocyclyl, carbocyclyl-C₁-C₂-alkyl, carbocyclyloxy, heterocyclyl, heterocyclyloxy, or heterocyclyl-C₁-C₂-alkyl, or 5- to 10-membered saturated carbobicyclyl or hetereobicyclyl, wherein the aforementioned heterocyclyl or heterobicyclyl rings comprise one or more, same or different heteroatoms selected from O, N, or S, wherein said N- and/or S-atoms are independently oxidized or non-oxidized, and wherein each substitutable carbon or heteroatom in the aforementioned groups is independently unsubstituted or substituted with one or more, same or different substituents R^{Z};
or two R^{Y} attached to the same atom form an oxo group;
or two R^{Y} together with the atoms to which they are bonded form a fused 5- or 6-membered saturated, partially or fully unsaturated, or aromatic carbocyclyl, or heterocyclyl, wherein the aforementioned heterocyclyl ring comprises one or more, same or different heteroatoms selected from O, N, or S, wherein said N- and/or S-atoms are independently oxidized or non-oxidized;
- R^{Z}: is halogen, CN, C₁-C₄-alkyl, C₁-C₄-haloalkyl, C₁-C₄-alkoxy, or 3- to 6-membered saturated carbocyclyl, carbocyclyloxy, heterocyclyl, or heterocyclyloxy, wherein the afore-mentioned heterocyclyl rings comprise one or more, same or different heteroatoms selected from O, N, or S, wherein said N- and/or S-atoms are independently oxidized or non-oxidized;
or two R^{Z} attached to the same atom form an oxo group or cyclopropyl;
with the proviso that one of the following i) to iv) is true:
i) A is 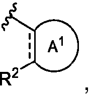 wherein A¹ is substituted with at least one substituent R^{A1}; wherein one of said at least one substituent R^{A1} is piperazinyl, piperidinyl, or 1,4-diazepane, wherein each substitutable carbon atom of the piperazinyl, piperidinyl, or 1,4-diazepane is independently unsubstituted or substituted with one or more, same or different substituents R^{A3};
ii) A is 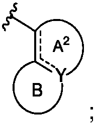 wherein B is a fused morpholine ring, wherein each substitutable carbon atom of said morpholine ring is independently unsubstituted or substituted with one or more, same or different substituents R^{B};
iii) R¹ is substituted with at least one substituent R^{Y};
   wherein one of said at least one substituent R^{Y} is piperazinyl or 1,4-diazepane, wherein each substitutable carbon atom of the piperazinyl or 1,4-diazepane is independently unsubstituted or substituted with one or more, same or different substituents R^{Z};
iv) A

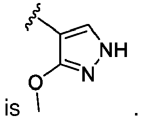

In a preferred embodiment,
i) A is 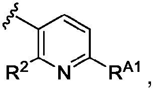 wherein
   R^{A1} is piperazinyl, wherein each substitutable carbon atom of the piperazinyl is independently unsubstituted or substituted with one or more, same or different substituents RA3;
   wherein preferably
   R^{A3} is CF₃;
      or
ii) A is 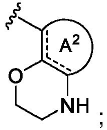 or
iii) R¹ is 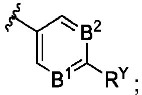 wherein
   - B¹: is N or CH;
   - B²: is N or CH; and
   - R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
   wherein preferably
   - R^{Z}: is F or CF₃,
   or two R^{Z} attached to the same carbon atom form cyclopropyl;or
iv) A is 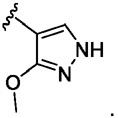 In another preferred embodiment,
   the compound is a compound of formula (la*-1) 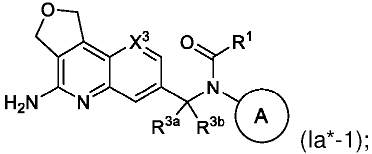 wherein
   - X³: is CH.

In another preferred embodiment,
- R²: is halogen, S(=O)₂R^{S}, C₁-C₂-alkoxy, or C₁-C₂-haloalkyl;
wherein preferably
- R^{S}: is C₁-alkyl.

In an even more preferred embodiment,
- R²: is Cl, CF₃, or OCH₃.

In another preferred embodiment,
- A¹: is pyridinyl or pyrazolyl;
wherein the pyridinyl or pyrazolyl is independently unsubstituted or substituted with one or more, same or different, substituents R^{A1}; wherein
- R^{A1}: is C₁-C₂-alkyl or piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3};
and
- A²: is unsubstituted phenyl; and
- B: is a fused unsubstituted morpholine ring, wherein each substitutable carbon atom of said morpholine ring is independently unsubstituted or substituted with one or more, same or different substituents R^{B}.

In an even more preferred embodiment,
- A: is a moiety selected from the group consisting of 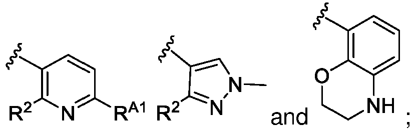
wherein
- R^{A1}: is piperazinyl, wherein each substitutable carbon atom of the piperazinyl is independently unsubstituted or substituted with one or more, same or different substituents R^{A3};
and wherein preferably
- R^{A3}: is CF₃.

In an even more preferred embodiment,
- A: is a moiety selected from the group consisting of

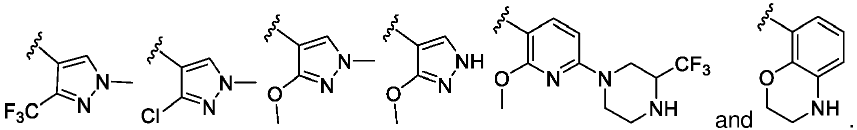

In another preferred embodiment,
- R¹: is 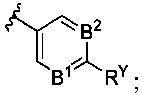
wherein
- B¹: is N;
- B²: is CH or N; and
- R^{Y}: is F, CN, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃,
or two R^{Z} attached to the same carbon atom form cyclopropyl.

In another preferred embodiment,
- A: is 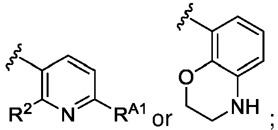
wherein
- R^{A1}: is piperazinyl, wherein the piperazinyl is unsubstituted or one substitutable carbon atom of the piperazinyl is substituted with one or more, same or different substituents R^{A3}; and
- R¹: is 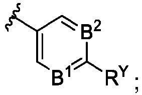
wherein
- B¹: is N;
- B²: is CH or N; and
- R^{Y}: is F, CN, CF₃, or cyclopropyl;
and wherein preferably
- R^{A3}: is CF₃;
or
- A: is and 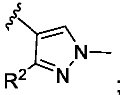
- R¹: is 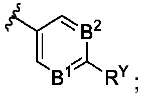
wherein
- B¹: is N;
- B²: is CH or N; and
- R^{Y}: piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃,
or two R^{Z} attached to the same carbon atom form cyclopropyl.

In one even more preferred embodiment,
- R¹: is

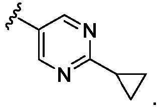

In another even more preferred embodiment,
- R¹: is 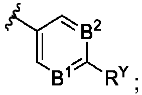
wherein
- B¹: is N;
- B²: is CH; and
- R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃,
or two R^{Z} attached to the same carbon atom form cyclopropyl.

In another preferred embodiment,
the compound of formula (I) is selected from the group consisting of:
N-({4-amino-1H,3H-furo[3,4-c]quinolin-7-yl}methyl)-N-[1-methyl-3-(trifluoromethyl)-1H-pyrazol-4-yl]-6-(piperazin-1-yl)pyridine-3-carboxamide;
N-({4-amino-1H,3H-furo[3,4-c]quinolin-7-yl}methyl)-6-{4,7-diazaspiro[2.5]octan-7-yl}-N-[1-methyl-3-(trifluoromethyl)-1H-pyrazol-4-yl]pyridine-3-carboxamide;
N-({4-amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide;
N-({4-amino-1H,3H-furo[3,4-c]quinolin-7-yl}methyl)-2-cyclopropyl-N-(3,4-dihydro-2H-1,4-ben-zoxazin-8-yl)pyrimidine-5-carboxamide;
N-({4-amino-1H,3H-furo[3,4-c]quinolin-7-yl}methyl)-N-(3-methoxy-1-methyl-1H-pyrazol-4-yl)-6-[3-(trifluoromethyl)piperazin-1-yl]pyridine-3-carboxamide;
N-({4-amino-1H,3H-furo[3,4-c]quinolin-7-yl}methyl)-6-(6,6-difluoro-1,4-diazepan-1-yl)-N-(3-methoxy-1-methyl-1H-pyrazol-4-yl)pyridine-3-carboxamide;
N-({4-amino-1H,3H-furo[3,4-c]quinolin-7-yl}methyl)-N-(3-chloro-1-methyl-1H-pyrazol-4-yl)-6-[3-(trifluoromethyl)piperazin-1-yl]pyridine-3-carboxamide;
N-({4-amino-1H,3H-furo[3,4-c]quinolin-7-yl}methyl)-2-cyclopropyl-N-(3-methoxy-1H-pyrazol-4-yl)pyrimidine-5-carboxamide; and
N-({4-amino-1H,3H-furo[3,4-c]quinolin-7-yl}methyl)-N-(3-methoxy-1-methyl-1H-pyrazol-4-yl)-6-[2-(trifluoromethyl)piperazin-1-yl]pyridine-3-carboxamide.

In a further aspect, the present invention relates to a pharmaceutical composition comprising a pharmaceutically effective amount of the compound of formula (I) as defined herein, and optionally a pharmaceutically acceptable carrier, diluent or excipient.

In yet another aspect, the present invention relates to a compound of formula (I) as defined herein or a pharmaceutical composition comprising the same as defined herein for use in medicine. In particular, the present invention relates to a compound of formula (I) as defined herein or a pharmaceutical composition comprising the same as defined herein for use in modulating PRMT5, in particular (partial) inhibition of PRMT5.

In one embodiment, the compound of the present invention or a pharmaceutical composition comprising the same is for use in the treatment of a disease selected from the group consisting of cancer and pre-cancerous syndromes.

In further aspects, the present invention relates to methods of treatment comprising the administration of a compound of formula (I) as defined herein or a pharmaceutical composition comprising the same as defined herein to a human or animal body.

### Detailed Description

In the following, preferred embodiments of the substituents in the above formula (I) are described in further detail. It is to be understood that each preferred embodiment is relevant on its own as well as in combination with other preferred embodiments. Furthermore, it is to be understood that the preferences in each case also apply to the salts, stereoisomers, tautomers, atro-pisomers, rotamers and N-oxides of the compounds of the invention.

As indicated above, in the compound of formula (la*) 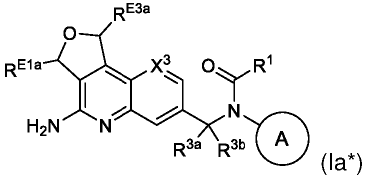
- X³: is CH, CR^{X3}, or N; wherein
- R^{X3}: is halogen.

In a particularly preferred embodiment,
- X³: is CH.

Further, as indicated above in connection with the compounds of formula (la*),
R^{E1a} and R^{E3a} are independently H, C₁-C₄-alkyl, or C₁-C₄-haloalkyl.

In a preferred embodiment,
R^{E1a} and R^{E3a} are independently H, C₁-C₂-alkyl, or C₁-C₂-haloalkyl.

In a more preferred embodiment,
R^{E1a} and R^{E3a} are independently H or C₁-C₂-alkyl.

In an even more preferred embodiment,
R^{E1a} and R^{E3a} are independently H or CH₃.

In an even more preferred embodiment,
R^{E3a} is H; and
R^{E1a} is H, C₁-C₂-alkyl, or C₁-C₂-haloalkyl, preferably H or C₁-C₂-alkyl, more preferably H or CH₃. In one particularly preferred embodiment,
R^{E1a} and R^{E3a} are H.

Thus, in a more preferred embodiment, the compound of formula (la*) is a compound of formula (Ia**), preferably a compound of formula (la**-1)

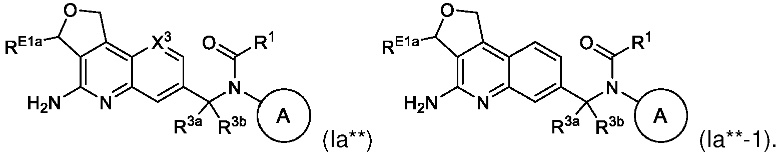

More preferably, the compound of formula (la*), in particular the compound of formula (la**) or (la**-1), is a compound of formula (la*-1) or (la*-2) 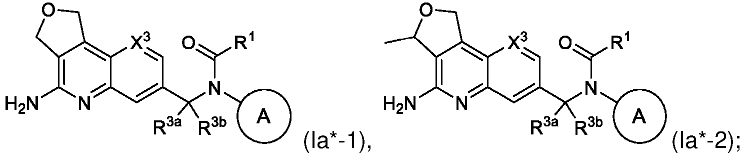 wherein preferably
- X³: is CH or CR^{X3}, more preferably CH.

Thus, the compound of formula (la*) is even more preferably a compound of formula (Ia*-1*) or (la*-2*)

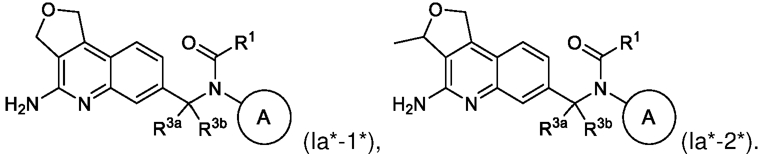

In one particularly preferred embodiment, the compound of formula (I) is a compound of formula (Ia*-1), preferably a compound of formula (Ia*-1*).

In connection with the compounds of formula (la*), as well as in connection with the compounds of formula (Ia**), (Ia**-1), (Ia*-1), (Ia*-1*), (Ia*-2), and (Ia*-2*), the following preferred embodiments regarding the substituent R¹ are relevant.

As indicated above, in connection with the compounds of the present invention,
- R¹: is a 6-membered aromatic carbocyclyl or heterocyclyl, wherein the aforementioned heterocyclic ring comprises one or more, same or different heteroatoms selected from O, N, or S, wherein said N- and/or S-atoms are independently oxidized or non-oxidized, and wherein the aforementioned rings are independently unsubstituted or substituted with one or more, preferably one, same or different substituents R^{Y};
wherein R^{Y} is as defined above in connection with the compounds of formula (I), and wherein preferably
- R^{Y}: is halogen, CN, C₁-C₂-alkyl, C₁-C₂-haloalkyl, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl;
more preferably
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl,
even more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl.

In an even more preferred embodiment,
- R¹: is pyridinyl or pyrimidinyl;
wherein the pyridinyl or pyrimidinyl is independently unsubstituted or substituted with one or more, preferably one, same or different substituents R^{Y};
wherein R^{Y} is as defined above in connection with the compounds of formula (I), and wherein
preferably
- R^{Y}: is halogen, CN, C₁-C₂-alkyl, C₁-C₂-haloalkyl, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl;
more preferably
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl,
even more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl.

In one even more preferred embodiment,
- R¹: is pyridinyl; wherein the pyridinyl is independently unsubstituted or substituted with one or more, preferably one, same or different substituents R^{Y}; wherein
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃;
or two R^{Z} attached to the same carbon atom form cyclopropyl,
and wherein more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃;
or two R^{Z} attached to the same carbon atom form cyclopropyl.

In another even more preferred embodiment,
- R¹: is pyrimidinyl;
wherein the pyrimidinyl is independently unsubstituted or substituted with one or more, preferably one, same or different substituents R^{Y}; wherein
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃;
or two R^{Z} attached to the same carbon atom form cyclopropyl,
and wherein more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃;
or two R^{Z} attached to the same carbon atom form cyclopropyl.

Thus, in a particularly preferred embodiment,
- R¹: is 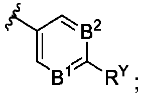
wherein
- B¹: is CH or N, and
- B²: is CH or N
wherein R^{Y} is as defined above in connection with the compounds of formula (I), and wherein
preferably
- R^{Y}: is halogen, CN, C₁-C₂-alkyl, C₁-C₂-haloalkyl, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl; more preferably
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl,
even more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl.

In a more preferred embodiment,
- R¹: is 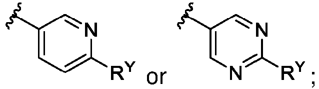
wherein R^{Y} is as defined above in connection with the compounds of formula (I), and wherein
preferably
- R^{Y}: is halogen, CN, C₁-C₂-alkyl, C₁-C₂-haloalkyl, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl;
more preferably
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl,
even more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl.

In one particularly preferred embodiment,
- R¹: is 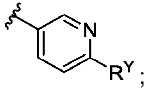
wherein
- R^{Y}: is halogen, CN, C₁-C₂-alkyl, C₁-C₂-haloalkyl, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl;
more preferably
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl,
even more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl.

In another particularly preferred embodiment,
- R¹: is 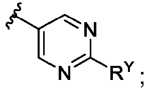
wherein
- R^{Y}: is halogen, CN, C₁-C₂-alkyl, C₁-C₂-haloalkyl, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl;
more preferably
- R^{Y}: is F, CN, CH₃, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl,
even more preferably
- R^{Y}: is cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is halogen or C₁-haloalkyl; preferably F or CF₃;
or two R^{Z} attached to the same carbon atom form cyclopropyl.

In particularly preferred embodiments,
- R¹: is selected from the group consisting of

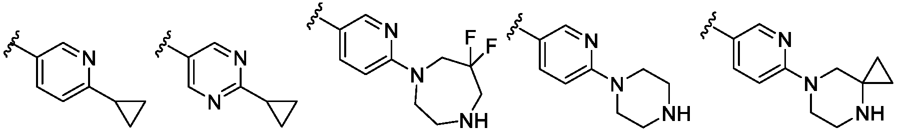

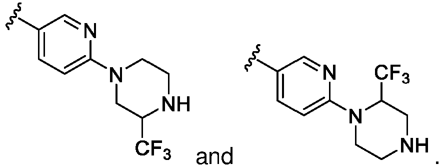

As indicated above, in particular embodiments of the present invention,
i) A is 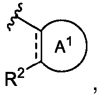 wherein A¹ is substituted with at least one substituent R^{A1};
   wherein one of said at least one substituent R^{A1} is piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3}; or
ii) A is 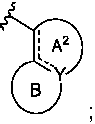 wherein
   - B: is a fused morpholine ring, wherein each substitutable carbon atom of said morpholine ring is independently unsubstituted or substituted with one or more, same or different substituents R^{B}.

In these contexts,
- R¹: is not substituted with a piperazinyl or 1,4-diazepane, wherein each substitutable carbon atom of the aforementioned piperazinyl or 1,4-diazepane is independently unsubstituted or substituted with one or more, same or different substituents R^{Z}.

Therefore, in these contexts, the same preferred embodiments regarding R¹ defined above apply, but without the options of R^{Y} being piperazinyl or 1,4-diazepane.

Thus, in a particularly preferred embodiment,
- R¹: is 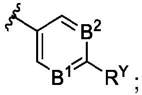
wherein
- B¹: is CH or N, and
- B²: is CH or N
wherein R^{Y} is as defined above in connection with the compounds of formula (I), and wherein
preferably
- R^{Y}: is halogen, CN, C₁-C₂-alkyl, C₁-C₂-haloalkyl, or cyclopropyl;
more preferably
- R^{Y}: is F, CN, CH₃, CF₃, or cyclopropyl,
even more preferably
- R^{Y}: is cyclopropyl.

In a more preferred embodiment,
- R¹: is 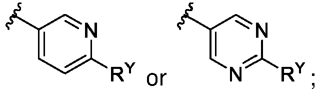
wherein R^{Y} is as defined above in connection with the compounds of formula (I), and wherein
preferably
- R^{Y}: is halogen, CN, C₁-C₂-alkyl, C₁-C₂-haloalkyl, or cyclopropyl;
more preferably
- R^{Y}: is F, CN, CH₃, CF₃, or cyclopropyl,
even more preferably
- R^{Y}: is cyclopropyl.

In one particularly preferred embodiment,
- R¹: is 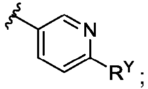
wherein
- R^{Y}: is halogen, CN, C₁-C₂-alkyl, C₁-C₂-haloalkyl, or cyclopropyl;
more preferably
- R^{Y}: is F, CN, CH₃, CF₃, or cyclopropyl,
even more preferably
- R^{Y}: is cyclopropyl.

In another particularly preferred embodiment,
- R¹: is 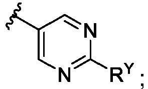
wherein
- R^{Y}: is halogen, CN, C₁-C₂-alkyl, C₁-C₂-haloalkyl, or cyclopropyl;
more preferably
- R^{Y}: is F CN, CH₃, CF₃, or cyclopropyl,
even more preferably
- R^{Y}: is cyclopropyl.

In one particularly preferred embodiment,
- R¹: is 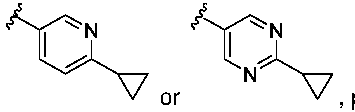 preferably

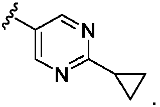

As indicated above, in other particular embodiments of the present invention,
iii) R¹ is substituted with at least one substituent R^{Y}; wherein one of said at least one substituent R^{Y} is piperazinyl or 1,4-diazepane, wherein each substitutable carbon atom of the aforementioned piperazinyl or 1,4-diazepane is independently unsubstituted or substituted with one or more, same or different substituents R^{Z}.

In this context, it is preferred that
- R¹: is substituted with one substituent R^{Y}; wherein
- R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z}.

Therefore, in this context, the same preferred embodiments regarding R¹ defined above apply, but wherein
- R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
as defined in the preferred embodiments regarding R¹ defined above.

Thus, in a preferred embodiment,
- R¹: is 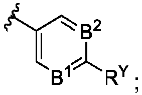
wherein
- B¹: is N or CH;
- B²: is N or CH; and
- R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
wherein preferably
- R^{Z}: is F or CF₃,
or two R^{Z} attached to the same carbon atom form cyclopropyl. In a more preferred embodiment,
- R¹: is 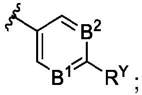
wherein
- B¹: is N;
- B²: is N or CH; and
- R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃.
or two R^{Z} attached to the same carbon atom form cyclopropyl.

In an even more preferred embodiment,
- R¹: is 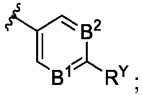
wherein
- B¹: is N;
- B²: is N or CH; and
- R^{Y}: is piperazinyl or 1,4-diazepane, wherein the aforementioned piperazinyl or 1,4-diazepane is independently unsubstituted or one substitutable carbon atom of the piperazinyl or 1,4-diazepane is substituted with one or two, same or different substituents R^{Z};
wherein
- R^{Z}: is F or CF₃,
or two R^{Z} attached to the same carbon atom form cyclopropyl.

In an even more preferred embodiment,
- R¹: is 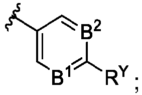
wherein
- B¹: is N;
- B²: is CH; and
- R^{Y}: is piperazinyl or 1,4-diazepane, wherein the aforementioned piperazinyl or 1,4-diazepane is independently unsubstituted or one substitutable carbon atom of the piperazinyl or 1,4-diazepane is substituted with one or two, same or different substituents R^{Z};
wherein
- R^{Z}: is F or CF₃,
or two R^{Z} attached to the same carbon atom form cyclopropyl.

In particularly preferred embodiments,
- R¹: is selected from the group consisting of 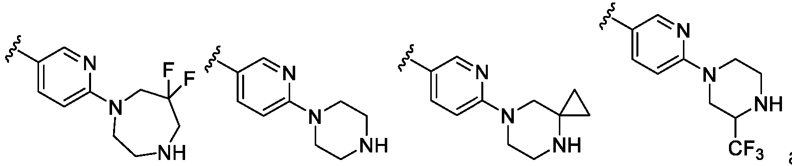 and

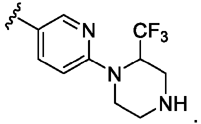

In connection with the compounds of formula (la*), as well as in connection with the compounds of formula (Ia**), (Ia**-1), (Ia*-1), (Ia*-1*), (Ia*-2), and (Ia*-2*), in particular in connection with the preferred embodiments regarding R¹ defined above, the following preferred embodiments regarding the A moiety are relevant.

As indicated above, in connection with the compounds of the present invention,
- A: is a moiety selected from

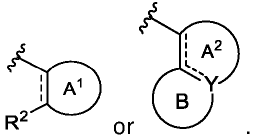

In a preferred embodiment,
- A: is a moiety selected from

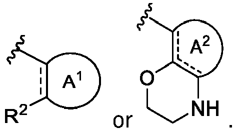

In a more preferred embodiment,
- A: is a moiety selected from

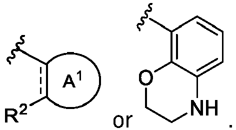

In connection with the compounds of formula (la*) as well as in connection with the compounds of formula (Ia**), (Ia**-1), (Ia*-1), (Ia*-1*), (Ia*-2), and (Ia*-2*), in particular in connection with the preferred embodiments regarding R¹ defined above, the following preferences regarding A¹ and R² are relevant.

As indicated above, in connection with the compounds of the present invention
- A¹: is a 5- or 6-membered saturated, partially or fully unsaturated, or aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned rings is independently unsubstituted or substituted with one or more, same or different substituents R^{A1}.

In a preferred embodiment,
- A¹: is a 5- or 6-membered aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned aromatic rings is independently unsubstituted or substituted with one or more, same or different substituents R^{A1}.

In a more preferred embodiment,
- A¹: is phenyl or a 5- or 6-membered aromatic heterocyclic ring, wherein the aforementioned heterocyclic ring comprises one or two heteroatoms selected from N or S, wherein said N-atoms are independently oxidized or non-oxidized, and wherein each substitutable carbon or N in the aforementioned aromatic rings is independently unsubstituted or substituted with one or more, same or different substituents R^{A1}.

In an even more preferred embodiment,
- A¹: is phenyl, pyridinyl, pyrazolyl, or thiophenyl, wherein the phenyl, pyridinyl, pyrazolyl, or thiophenyl is independently unsubstituted or substituted with one or more, same or different substituents R^{A1}.

In an even more preferred embodiment,
- A¹: is phenyl, pyridinyl, or pyrazolyl, wherein the phenyl, pyridinyl, or pyrazolyl is independently unsubstituted or substituted with one or more, same or different substituents RA1.

In an even more preferred embodiment,
- A¹: is pyridinyl or pyrazolyl, wherein the pyridinyl or pyrazolyl is independently unsubstituted or substituted with one or more, same or different substituents R^{A1}.

In one particularly preferred embodiment,
- A¹: is pyridinyl, wherein the pyridinyl is independently unsubstituted or substituted with one piperazinyl, wherein each substitutable carbon atom of the piperazinyl is independently unsubstituted or substituted with one or more, same or different substituents R^{A3}.

In another particularly preferred embodiment,
- A¹: is pyrazolyl, wherein the pyrazolyl is independently unsubstituted or substituted with one or more, preferably one, same or different substituents selected from C₁-C₂-alkyl, preferably CH₃.

In a particularly preferred embodiment,
- A¹: is selected from the group consisting of 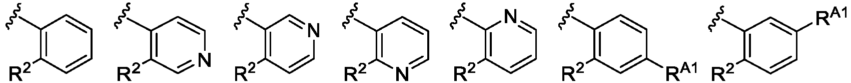 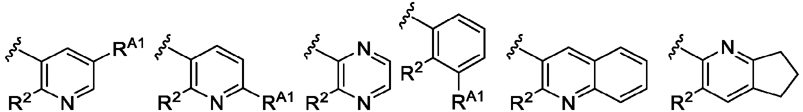 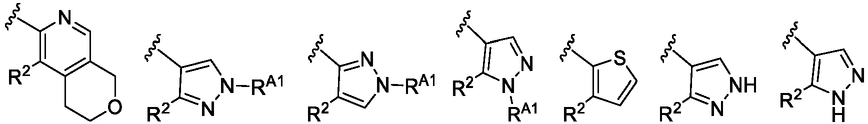 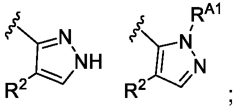
preferably from the group consisting of 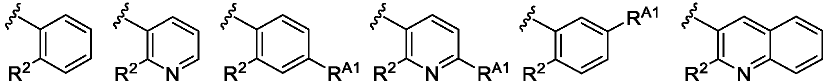 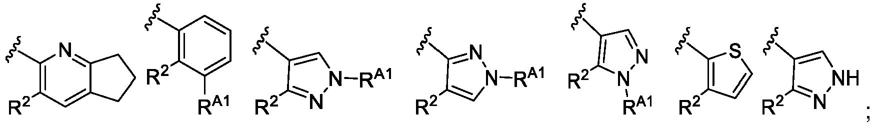 more preferably from the group consisting of 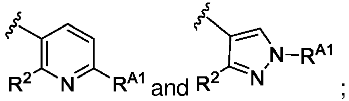 even more preferably from the group consisting of

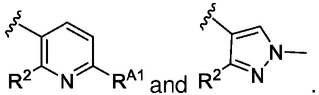

In connection with the compounds of formula (la*) as well as in connection with the compounds of formula (Ia**), (Ia**-1), (Ia*-1), (Ia*-1*), (Ia*-2), and (Ia*-2*), in particular in connection with the preferred embodiments regarding R¹ and A¹ defined above, preferably, A¹ is unsubstituted or substituted with one, two, or three, same or different substituents R^{A1}, more preferably A¹ is unsubstituted or substituted with one or two, same or different substituents R^{A1}, even more preferably A¹ is unsubstituted or substituted with only one substituent R^{A1}. In a particularly preferred embodiment, A¹ is substituted with one substituent R^{A1}. It is to be understood that when ring A¹ or any embodiment thereof is referred to herein as being unsubstituted, this refers to said ring A¹ or embodiment thereof not being substituted with a substituent R^{A1}, i.e., not having a further substituent in addition to R².

Further, in connection with the compounds of formula (I) as well as in connection with the compounds of formula (Ia**), (Ia**-1), (Ia*-1), (Ia*-1*), (Ia*-2), and (Ia*-2*), in particular with the preferred embodiments regarding R¹ and A¹ as defined above, the following preferences regarding R² and, if present, R^{A1} and, if present, R^{A3} are relevant.

As indicated above, in connection with the compounds of the present invention,
- R²: is halogen, CN, S(=O)₂R^{S}, S(=O)(=NH)R^{S}, C(=O)R^{C}, NHC(=O)R^{C}, C(=O)NR^{N4}R^{N5}, C₁-C₄-alkoxy, C₁-C₄-haloalkyl, C₁-C₄-haloalkoxy, C₁-C₆-hydroxyalkyl; wherein
- R^{C}: is H, or C₁-C₄-alkyl;
- R^{N1}: is H, or C₁-C₄-alkyl;
- R^{N2}: is H, or C₁-C₄-alkyl;
- R^{S}: is C₁-C₄-alkyl, C₁-C₄-alkoxy, C₁-C₄-haloalkyl, C₃-C₅-cycloalkyl, or NR^{N4}R^{N5}.

In a preferred embodiment,
- R²: is halogen, CN, S(=O)₂R^{S}, C(=O)R^{C}, C(=O)NR^{N4}R^{N5}, C₁-C₂-alkoxy, C₁-C₂-haloalkyl, C₁-C₂-haloalkoxy, C₁-C₂-alkoxy-C₁-C₂-alkyl, or C₁-C₂-hydroxyalkyl;
and wherein preferably
- R^{C}: is H, or C₁-C₂-alkyl;
- R^{N1}: is H, or C₁-C₂-alkyl;
- R^{N2}: is H, or C₁-C₂-alkyl;
- R^{S}: is C₁-C₂-alkyl, C₁-C₂-alkoxy, C₃-C₅-cycloalkyl, or NR^{N4}R^{N5}.

In a more preferred embodiment,
- R²: is halogen, CN, S(=O)₂R^{S}, C(=O)R^{C}, C(=O)NR^{N4}R^{N5}, C₁-C₂-alkoxy, C₁-C₂-haloalkyl, C₁-C₂-haloalkoxy, C₁-C₂-alkoxy-C₁-C₂-alkyl, or C₁-C₂-hydroxyalkyl;
and wherein preferably
- R^{C}: is H, or C₁-alkyl;
- R^{N1}: is H, or C₁-alkyl;
- R^{N2}: is H, or C₁-alkyl;
- R^{S}: is C₁-C₂-alkyl, C₃-C₅-cycloalkyl, or NR^{N4}R^{N5}.

In an even more preferred embodiment,
- R²: is halogen, CN, S(=O)₂R^{S}, C(=O)NR^{N1}R^{N2}, C₁-C₂-alkoxy, C₁-C₂-haloalkyl, or C₁-C₂-haloalkoxy;
and wherein
- R^{N1}: is H, or C₁-alkyl;
- R^{N2}: is H, or C₁-alkyl;
- R^{S}: is C₁-C₂-alkyl.

In an even more preferred embodiment,
- R²: is halogen, S(=O)₂R^{S}, C₁-C₂-alkoxy, or C₁-C₂-haloalkyl;
and wherein
- R^{S}: is C₁-alkyl.

In an even more preferred embodiment,
- R²: is Cl, F, S(=O)₂R^{S}, C₁-alkoxy, CHF₂, or CF₃;
and wherein
- R^{S}: is C₁-alkyl.

In an even more preferred embodiment, R² is Cl, CF₃, or OCH₃.

Further, as indicated above, in connection with the compounds of the present invention,
- R^{A1}: is halogen, CN, OH, C₁-C₄-alkyl, C₁-C₄-alkoxy, C₁-C₄-haloalkyl, or 3- to 7-membered saturated carbocyclyl or heterocyclyl, wherein the aforementioned heterocyclyl ring comprises one or more, same or different heteroatoms selected from O, N, or S, wherein said N- and/or S-atoms are independently oxidized or non-oxidized; and wherein each substitutable carbon or heteroatom in the aforementioned rings is independently unsubstituted or substituted with one or more, same or different substituents R^{A3}.

In a preferred embodiment,
- R^{A1}: is halogen, CN, C₁-C₂-alkyl, C₁-C₂-haloalkyl, C₁-C₂-alkoxy, cyclopropyl, or a 6- or 7-membered saturated heterocyclyl, wherein the aforementioned heterocyclyl ring comprises one or more, same or different heteroatoms selected from O, N, or S, preferably N, wherein said N- and/or S-atoms are independently oxidized or non-oxidized; and wherein each of the aforementioned heterocyclyl rings is independently unsubstituted or substituted with one or more, preferably one, same or different substituents R^{A3}.

In a more preferred embodiment,
- R^{A1}: is halogen, CN, C₁-C₂-alkyl, C₁-C₂-haloalkyl, C₁-C₂-alkoxy, cyclopropyl, piperazinyl, piperidinyl, or 1,4-diazepane, wherein each substitutable carbon atom of the piperazinyl, piperidinyl, or 1,4-diazepane is independently unsubstituted or substituted with one or more, same or different substituents R^{A3}.

In a more preferred embodiment,
- R^{A1}: is halogen, C₁-C₂-alkyl, C₁-C₂-haloalkyl, C₁-C₂-alkoxy, cyclopropyl, or piperazinyl, wherein each substitutable carbon atom of the piperazinyl is independently unsubstituted or substituted with one or more, same or different substituents R^{A3}.

In an even more preferred embodiment,
- R^{A1}: is halogen, C₁-C₂-alkyl, C₁-haloalkyl, cyclopropyl, or piperazinyl, wherein each substitutable carbon atom of the piperazinyl is independently unsubstituted or substituted with one or more, same or different substituents R^{A3}.

In an even more preferred embodiment,
- R^{A1}: is halogen, C₁-C₂-alkyl, cyclopropyl, or piperazinyl, wherein each substitutable carbon atom of the piperazinyl is independently unsubstituted or substituted with one or more, same or different substituents R^{A3}.

In an even more preferred embodiment,
- R^{A1}: is C₁-C₂-alkyl or piperazinyl, wherein each substitutable carbon atom of the piperazinyl is independently unsubstituted or substituted with one or more, same or different substituents R^{A3}.

It is to be understood that the preferred embodiments for R^{A1} may vary depending on whether R^{A1} is attached to a carbon atom or heteroatom of ring A¹. For example, if R^{A1} is attached to a carbon atom of ring A¹, R^{A1} is preferably halogen, C₁-alkyl, or piperazinyl, wherein each substitutable carbon atom of the piperazinyl is independently unsubstituted or substituted with one or more, same or different substituents R^{A3}. If R^{A1} is attached to a heteroatom of ring A¹, such as N, R^{A1} is preferably C₁-C₂-alkyl or cyclopropyl, more preferably CH₃.

Further, in connection with the compounds of the present invention, in particular in connection with the preferred embodiments of R^{A1} defined above, the following preferences regarding R^{A3} are relevant.

As indicated above, in connection with the compounds of the present invention,
- R^{A3}: is halogen, CN, OH, C₁-C₄-alkyl, C₁-C₄-alkoxy, or C₁-C₄-haloalkyl;
or two R^{A3} attached to the same atom form cyclopropyl.

In a preferred embodiment,
- R^{A3}: is halogen, or C₁-C₂-haloalkyl;
or two R^{A3} attached to the same atom form cyclopropyl.

In a more preferred embodiment,
- R^{A3}: is halogen, or C₁-haloalkyl;
or two R^{A3} attached to the same atom form cyclopropyl.

In an even more preferred embodiment,
- R^{A3}: is F or CF₃;
or two R^{A3} attached to the same atom form cyclopropyl.

In a particularly preferred embodiment,
- R^{A3}: is CF₃

As indicated above, in particular embodiments of the present invention,
i) A is 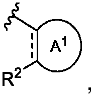 wherein A¹ is substituted with at least one substituent R^{A1}; wherein one of said at least one substituent R^{A1} is piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3}.

Thus, in this context, it is preferred that
- A: is wherein 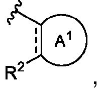
- A¹: is pyridinyl or pyrazolyl;
wherein the pyridinyl or pyrazolyl is independently substituted with one, same or different, substituents R^{A1}; wherein
- R^{A1}: is piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3};
- R²: is Cl, CF₃, or OCH₃; and
wherein preferably,
- R^{A3}: is halogen, or C₁-haloalkyl;
or two R^{A3} attached to the same atom form cyclopropyl.
more preferably,
- R^{A3}: is F or CF₃;
or two R^{A3} attached to the same atom form cyclopropyl.
particularly preferably,
- R^{A3}: is CF₃.

In one more preferred embodiment,
- A: is wherein 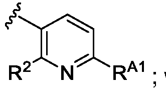
- R^{A1}: is piperazinyl, wherein each substitutable carbon atom of the piperazinyl is independently unsubstituted or substituted with one or more, same or different substituents RA3;
- R²: is Cl, CF₃, or OCH₃; and
wherein preferably,
- R^{A3}: is halogen, or C₁-haloalkyl;
or two R^{A3} attached to the same atom form cyclopropyl.
more preferably,
- R^{A3}: is F or CF₃;
or two R^{A3} attached to the same atom form cyclopropyl.
particularly preferably,
- R^{A3}: is CF₃.

In a more preferred embodiment,
- A: is 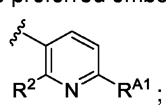 wherein
- R^{A1}: is piperazinyl, wherein the piperazinyl is unsubstituted or one substitutable carbon atom of the piperazinyl is substituted with one or more, same or different substituents R^{A3};
- R²: is Cl, CF₃, or OCH₃; and
wherein preferably,
- R^{A3}: is halogen, or C₁-haloalkyl;
or two R^{A3} attached to the same atom form cyclopropyl.
more preferably,
- R^{A3}: is F or CF₃;
or two R^{A3} attached to the same atom form cyclopropyl.
particularly preferably,
- R^{A3}: is CF₃.

In an even more preferred embodiment,
- A: is 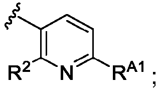 wherein
- R²: is OCH₃; and
- R^{A1}: is piperazinyl, wherein one substitutable carbon atom of the piperazinyl is substituted with CF₃.

In a particularly preferred embodiment,
- A: is

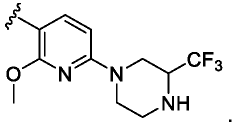

As indicated above, in particular other embodiments of the present invention,
iii) R¹ is substituted with at least one substituent R^{Y}; wherein one of said at least one substituent R^{Y} is piperazinyl or 1,4-diazepane, wherein each substitutable carbon atom of the aforementioned piperazinyl or 1,4-diazepane is independently unsubstituted or substituted with one or more, same or different substituents R^{Z}.

In this context, it is particularly preferred that
- A: is 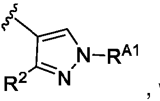 wherein
- R^{A1}: is C₁-C₂-alkyl or cyclopropyl;

In a more preferred embodiment,
- A: is

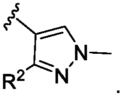

In particularly preferred embodiments,
- A: is selected from the group consisting of

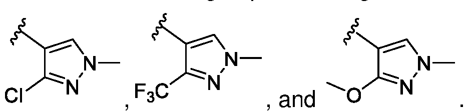

In connection with the compounds of formula (la*) as well as in connection with the compounds of formula (Ia**), (Ia**-1), (Ia*-1), (Ia*-1*), (Ia*-2), and (Ia*-2*), in particular in connection with the preferred embodiments regarding R¹ defined above, the following preferences regarding Y, A², and B are relevant.

As indicated above, in connection with the compounds of the present invention
- A²: is a 5- or 6-membered saturated, partially or fully unsaturated, or aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned rings is independently unsubstituted or substituted with one or more, same or different substituents R^{A2}.

In a preferred embodiment,
- A²: is a 5- or 6-membered aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned aromatic rings is independently unsubstituted or substituted with one or more, same or different substituents R^{A2}.

In a more preferred embodiment,
- A²: is phenyl or a 5- or 6-membered aromatic heterocyclic ring, wherein the aforementioned heterocyclic ring comprises one or two N-atoms, wherein said N-atoms are independently oxidized or non-oxidized, and wherein each substitutable carbon or N in the aforementioned aromatic rings is independently unsubstituted or substituted with one or more, same or different substituents R^{A2}.

In an even more preferred embodiment,
- A²: is phenyl, pyridinyl, or pyrazolyl; wherein the phenyl, pyridinyl, or pyrazolyl is independently unsubstituted or substituted with one or more, preferably one or two, more preferably one, same or different substituents R^{A2}; wherein preferably the phenyl, pyridinyl, or pyrazolyl is unsubstituted.

In an even more preferred embodiment,
- A²: is phenyl or pyridinyl; wherein the phenyl or pyridinyl is independently unsubstituted or substituted with one or more, preferably one or two, more preferably one, same or different substituents R^{A2}; wherein preferably the phenyl or pyridinyl is unsubstituted.

In a particularly preferred embodiment,
- A²: is phenyl; wherein the phenyl is independently unsubstituted or substituted with one or more, preferably one or two, more preferably one, same or different substituents R^{A2}; wherein preferably the phenyl is unsubstituted.

In connection with the compounds of formula (la*) as well as in connection with the compounds of formula (Ia**), (Ia**-1), (Ia*-1), (Ia*-1*), (Ia*-2), and (Ia*-2*), in particular the preferred embodiments regarding A² defined above, preferably, A² is unsubstituted or substituted with one, two, or three, same or different substituents R^{A2}, more preferably A² is unsubstituted or substituted with one or two, same or different substituents R^{A2}, even more preferably A² is unsubstituted or substituted with only one substituent R^{A2}. In a particularly preferred embodiment, A² is unsubstituted. It is to be understood that when ring A² or any embodiment thereof is referred to herein as being unsubstituted, this refers to said ring A² or embodiment thereof not being substituted with a substituent R^{A2}, i.e., not having a further substituent in addition to ring B. Further, as indicated above in connection with the compounds of the present invention,
- B: is a fused morpholine ring, wherein each substitutable carbon atom of said morpholine ring is independently unsubstituted or substituted with one or more, same or different substituents R^{B};

In a preferred embodiment,
- B: is a fused unsubstituted morpholine ring.

Thus, in a particular preferred embodiment, the bicyclic moiety 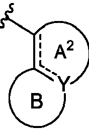 is

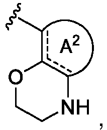

Wherein A² is defined as for the compounds of formula (la*), preferably defined as for the preferred embodiments of A² defined above.

In an even more preferred embodiment, the bicyclic moiety 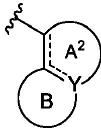 is

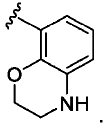

As indicated above, in particular embodiments of the present invention, ii)A is 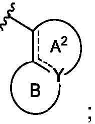 wherein
B is a fused morpholine ring, wherein each substitutable carbon atom of said morpholine ring is independently unsubstituted or substituted with one or more, same or different substituents R^{B}.

Thus, in this context, it is preferred that
- A: is

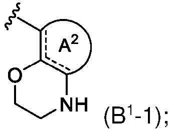

In a particularly preferred embodiment,
- A: is

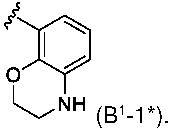

In connection with the compounds of formula (la*) as well as in connection with the compounds of formula (Ia**), (Ia**-1), (Ia*-1), (Ia*-1*), (Ia*-2), and (Ia*-2*), in particular in connection with the preferred embodiments regarding R¹ and B defined above, preferably, B is unsubstituted or substituted with one, two, or three, same or different substituents R^{B}, more preferably B is unsubstituted or substituted with one or two, same or different substituents R^{B}. It is particularly preferred that B is unsubstituted.

It is to be understood that when ring B or any embodiment thereof is referred to herein as being unsubstituted, this refers to said ring B or embodiment thereof not being substituted with a substituent R^{B}, i.e., not having a further substituent in addition to ring A².

Further, in connection with the compounds of formula (Ia*) as well as in connection with the compounds of formula (Ia**), (Ia**-1), (Ia*-1), (Ia*-1*), (Ia*-2), and (Ia*-2*), in particular in connection with the preferred embodiments regarding R¹, A², R², R^{A1}, and B as defined above, the following preferences regarding R^{A2} and R^{B}, if present, are relevant.

As indicated above, in connection with the compounds of the present invention,
- R^{A2}: is halogen, CN, OH, C₁-C₄-alkyl, or C₁-C₄-haloalkyl.

In a preferred embodiment,
- R^{A2}: is halogen, CN, C₁-C₂-alkyl, or C₁-C₂-haloalkyl.

In a more preferred embodiment,
- R^{A2}: is halogen or C₁-C₂-alkyl.

In a particularly preferred embodiment,
- R^{A2}: is Cl, F, or C₁-alkyl.

As indicated above, in connection with the compounds of the present invention,
- R^{B}: is halogen, CN, OH, C₁-C₄-alkyl, or C₁-C₄-haloalkyl; or two R^{B} attached to the same atom form an oxo group.

In a preferred embodiment,
- R^{B}: is halogen, CN, C₁-C₂-alkyl, or C₁-C₂-haloalkyl; or two R^{B} attached to the same atom form an oxo group.

In a more preferred embodiment,
- R^{B}: is halogen or C₁-C₂-alkyl; or two R^{B} attached to the same atom form an oxo group.

Overall, in a preferred embodiment,
- A¹: is phenyl, pyridinyl, or pyrazolyl;
wherein the phenyl, pyridinyl, or pyrazolyl is independently unsubstituted or substituted with one or more, preferably one, same or different, substituents R^{A1}; wherein preferably
- R^{A1}: is halogen, C₁-C₂-alkyl, cyclopropyl, piperazinyl, piperidinyl, or 1,4-diazepane, wherein each substitutable carbon atom of the piperazinyl, piperidinyl, or 1,4-diazepane, is independently unsubstituted or substituted with one or more, same or different substituents RA3;
and
- A²: is phenyl, pyridinyl, or pyrazolyl;
wherein the phenyl, pyridinyl, or pyrazolyl is independently unsubstituted or substituted with one or more, same or different, substituents R^{A2}; wherein preferably the phenyl, pyridinyl, or pyrazolyl is unsubstituted; and
- B: is a fused morpholine ring, wherein each substitutable carbon atom of said morpholine ring is independently unsubstituted or substituted with one or more, same or different substituents R^{B}, wherein preferably the fused morpholine is unsubstituted;
and wherein
- R²: is halogen, S(=O)₂R^{S}, C₁-C₂-alkoxy, or C₁-C₂-haloalkyl; preferably CI, CF₃, or OCH₃.

In a more preferred embodiment,
- A¹: is pyridinyl or pyrazolyl;
wherein the pyridinyl or pyrazolyl is independently unsubstituted or substituted with one or more, preferably one, same or different, substituents R^{A1}; wherein
- R^{A1}: is C₁-C₂-alkyl, cyclopropyl, piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3};
wherein
- R^{A3}: is F or CF₃,
or two R^{Z} attached to the same carbon atom form cyclopropyl; and wherein preferably
- R^{A3}: is CF₃;
and
- A²: is unsubstituted phenyl, pyridinyl, or pyrazolyl; preferably unsubstituted phenyl; and
- B: is a fused morpholine ring, wherein each substitutable carbon atom of said morpholine ring is independently unsubstituted or substituted with one or more, same or different substituents R^{B}, wherein preferably the fused morpholine is unsubstituted;
and wherein
- R²: is halogen, S(=O)₂R^{S}, C₁-C₂-alkoxy, or C₁-C₂-haloalkyl; preferably CI, CF₃, or OCH₃.

In one even more preferred embodiment,
- A: is a moiety selected from 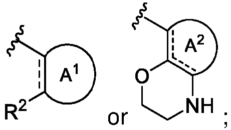 wherein
- A¹: is pyridinyl or pyrazolyl;
wherein the pyridinyl or pyrazolyl is independently unsubstituted or substituted with one or more, preferably one, same or different, substituents R^{A1}; wherein
- R^{A1}: is halogen, C₁-C₂-alkyl, cyclopropyl, piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3}; wherein
- R^{A3}: is F or CF₃,
or two R^{Z} attached to the same carbon atom form cyclopropyl; and wherein preferably
- R^{A3}: is CF₃;
and
- A²: is unsubstituted phenyl, pyridinyl, or pyrazolyl; preferably unsubstituted phenyl;
- R²: is halogen, S(=O)₂R^{S}, C₁-C₂-alkoxy, or C₁-C₂-haloalkyl; preferably CI, CF₃, or OCH₃.

In one even more preferred embodiment,
- A: is a moiety selected from 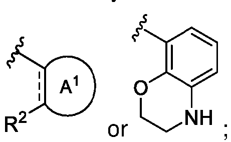 wherein
- A¹: is pyridinyl or pyrazolyl; wherein the pyridinyl or pyrazolyl is independently unsubstituted or substituted with one substituent R^{A1}; wherein
- R^{A1}: is halogen, C₁-C₂-alkyl, cyclopropyl, piperazinyl, piperidinyl, or 1,4-diazepane, wherein each substitutable carbon atom of the piperazinyl, piperidinyl, or 1,4-diazepane, is independently unsubstituted or substituted with one or more, preferably one or two, same or different substituents R^{A3}; wherein
- R^{A3}: is F or CF₃,
or two R^{Z} attached to the same carbon atom form cyclopropyl; and wherein preferably
- R^{A3}: is CF₃;
and
- R²: is halogen, C₁-C₂-alkoxy, or C₁-C₂-haloalkyl; preferably CI, CF₃, or OCH₃.

In an even more preferred embodiment,
- A: is a moiety selected from the group consisting of 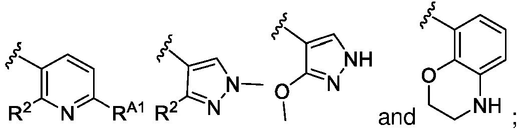
wherein
- R^{A1}: is piperazinyl or 1,4-diazepane, wherein each substitutable carbon atom of the piperazinyl or 1,4-diazepane is independently unsubstituted or substituted with one or more, preferably one or two, same or different substituents R^{A3}; wherein
- R²: is halogen, C₁-C₂-alkoxy, or C₁-C₂-haloalkyl; preferably CI, CF₃, or OCH₃; and
- R^{A3}: is F or CF₃,
or two R^{Z} attached to the same carbon atom form cyclopropyl; and wherein preferably
- R^{A3}: is CF₃.

In an even more preferred embodiment,
- A: is a moiety selected from the group consisting of 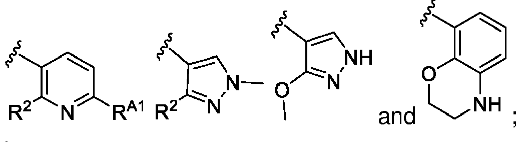
wherein
- R^{A1}: is piperazinyl, wherein the piperazinyl is unsubstituted or one carbon atom is substituted with one substituent R^{A3}; wherein
- R²: is halogen, S(=O)₂R^{S}, C₁-C₂-alkoxy, or C₁-C₂-haloalkyl; preferably CI, CF₃, or OCH₃; and
- R^{A3}: is F or CF₃,
or two R^{Z} attached to the same carbon atom form cyclopropyl; and wherein preferably
- R^{A3}: is CF₃.

In an even more preferred embodiment,
- A: is a moiety selected from the group consisting of 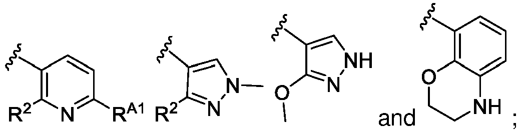
wherein
- R^{A1}: is piperazinyl, wherein the piperazinyl is unsubstituted or one carbon atom is substituted with one substituent R^{A3}; wherein
- R²: is halogen, S(=O)₂R^{S}, C₁-C₂-alkoxy, or C₁-C₂-haloalkyl; preferably CI, CF₃, or OCH₃; and
- R^{A3}: is CF₃.

In particularly preferred embodiments,
- A: is a moiety selected from the group consisting of

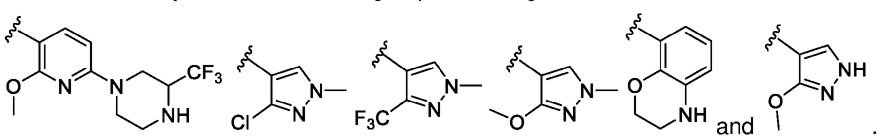

In particularly preferred embodiments,
- A: is

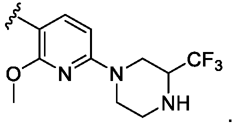

In other particularly preferred embodiments,
- A: is a moiety selected from the group consisting of

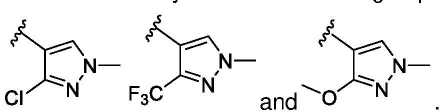

In other particularly preferred embodiments,
- A: is

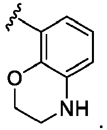

In other particularly preferred embodiments,
- A: is

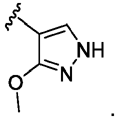

As indicated above, in connection with the compounds of formula (Ia*) of the present invention, in particular in connection with the compounds of formula (Ia**), (Ia**-1), (Ia*-1), (Ia*-1*), (Ia*-2), and (Ia*-2*); in particular in connection with the preferred embodiments regarding R¹, R², A¹, A², Y, Z, B, R^{A1}, R^{A2}, R^{A3}, R^{Y}, R^{Z}, and R^{B} defined above, one of the following i) to iv) is true:
i) A is 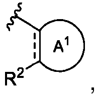 wherein A¹ is substituted with at least one, preferably one, substituent R^{A1}; wherein one of said at least one substituent R^{A1} is piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3};
ii) A is 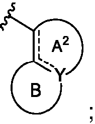 wherein B is a fused morpholine ring, wherein each substitutable carbon atom of said morpholine ring is independently unsubstituted or substituted with one or more, same or different substituents R^{B};
iii) R¹ is substituted with at least one substituent R^{Y}; wherein one of said at least one substituent R^{Y} is piperazinyl or 1,4-diazepane, wherein each substitutable carbon atom of the piperazinyl or 1,4-diazepane is independently unsubstituted or substituted with one or more, same or different substituents R^{Z};
iv) A is

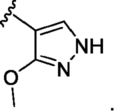

In a preferred embodiment, one of the following i) to iv) is true:
i) A is 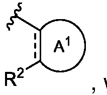 wherein A¹ is substituted with at least one, preferably one, substituent R^{A1}; wherein one of said at least one, preferably one, substituent R^{A1} is piperazinyl, wherein each substitutable carbon atom of the piperazinyl is independently unsubstituted or substituted with one or more, same or different substituents R^{A3};
ii) A is 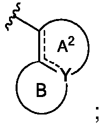 wherein B is a fused morpholine ring, wherein each substitutable carbon atom of said morpholine ring is independently unsubstituted or substituted with one or more, same or different substituents R^{B};
iii) R¹ is substituted with at least one substituent R^{Y}; wherein one of said at least one substituent R^{Y} is piperazinyl or 1,4-diazepane, wherein each substitutable carbon atom of the piperazinyl or 1,4-diazepane is independently unsubstituted or substituted with one or more, same or different substituents R^{Z};
iv) A is

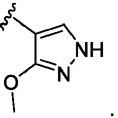

In one more preferred embodiment, one of the following i) to iv) is true:
i) A is 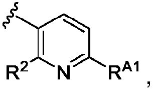 wherein
   - R^{A1}: is piperazinyl, wherein each substitutable carbon atom of the piperazinyl is independently unsubstituted or substituted with one or more, same or different substituents RA3;
   wherein preferably
   - R^{A3}: is F or CF₃,
   or two R^{A3} attached to the same carbon atom form cyclopropyl;
ii) A is 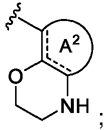 wherein preferably
   A² is unsubstituted phenyl;
iii) R¹ is 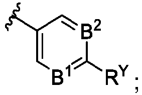
wherein
- B¹: is N or CH; preferably N;
- B²: is N or CH; and
- R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
wherein preferably
- R^{Z}: is F or CF₃,
or two R^{Z} attached to the same carbon atom form cyclopropyl; iv) A is

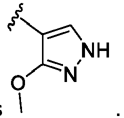

In one more preferred embodiment, one of the following i) to iv) is true:
i) A is 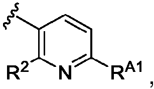 wherein
   - R^{A1}: is piperazinyl, wherein each substitutable carbon atom of the piperazinyl is independently unsubstituted or substituted with one or more, same or different substituents RA3;
   wherein
   - R^{A3}: is F or CF₃,
   or two R^{A3} attached to the same carbon atom form cyclopropyl;
ii) A is 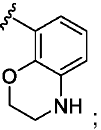
iii) R¹ is 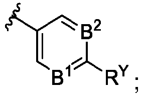 wherein
   - B¹: is N;
   - B²: is N or CH; and
   - R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
   wherein
   - R^{Z}: is F or CF₃,
   or two R^{Z} attached to the same carbon atom form cyclopropyl;
iv) A is

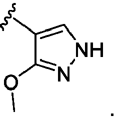

In one more preferred embodiment, one of the following i) to iv) is true:
i) A is 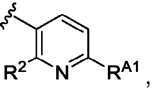 wherein
   - R^{A1}: is piperazinyl, wherein each substitutable carbon atom of the piperazinyl is independently unsubstituted or substituted with one or more, same or different substituents RA3;
   - R²: is halogen, S(=O)₂R^{S}, C₁-C₂-alkoxy, or C₁-C₂-haloalkyl, preferably CI, CF₃, or OCH₃; particularly preferably OCH₃;
   - R^{A3}: is F or CF₃, preferably CF₃.
   or two R^{A3} attached to the same carbon atom form cyclopropyl;
ii) A is 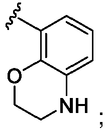
iii) R¹ is 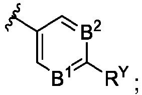 wherein
   - B¹: is N;
   - B²: is N or CH; and
   - R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
   wherein
   - R^{Z}: is F or CF₃,
   or two R^{Z} attached to the same carbon atom form cyclopropyl;
iv) A is

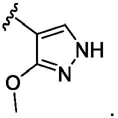

In an even more preferred embodiment, one of the following i) to iv) is true:
i) A is 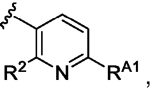 wherein
   - R^{A1}: is piperazinyl, wherein the piperazinyl is unsubstituted or one substitutable carbon atom of the piperazinyl is substituted with one or more, same or different substituents R^{A3};
   - R²: is CI, CF₃, or OCH₃; particularly preferably OCH₃;
   - R^{A3}: is CF₃,
   or two R^{A3} attached to the same carbon atom form cyclopropyl;
ii) A is 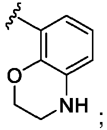
iii) R¹ is 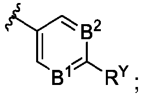 wherein
   - B¹: is N;
   - B²: is N or CH; preferably CH; and
   - R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
   wherein
   - R^{Z}: is F or CF₃,
   or two R^{Z} attached to the same carbon atom form cyclopropyl;
iv) A is

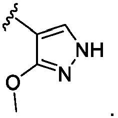

In an even more preferred embodiment, one of the following i) to iv) is true:
i) A is 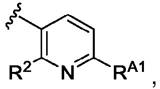 wherein
   - R^{A1}: is piperazinyl, wherein the piperazinyl is unsubstituted or one substitutable carbon atom of the piperazinyl is substituted with CF₃;
   - R²: is CI, CF₃, or OCH₃; particularly preferably OCH₃;
ii) A is 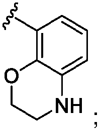
iii) R¹ is 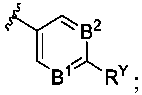 wherein
   - B¹: is N;
   - B²: is CH; and
   - R^{Y}: is piperazinyl or 1,4-diazepane, wherein each substitutable carbon atom of the aforementioned piperazinyl or 1,4-diazepane is independently unsubstituted or substituted with one or more, same or different substituents R^{Z};
   wherein
   - R^{Z}: is F or CF₃,
   or two R^{Z} attached to the same carbon atom form cyclopropyl;
iv) A is

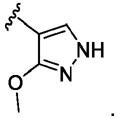

In a particularly preferred embodiment, one of the following i) to iv) is true:
i) A is 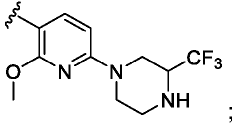
ii) A is 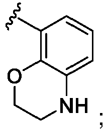
iii) R¹ is selected from the group consisting of 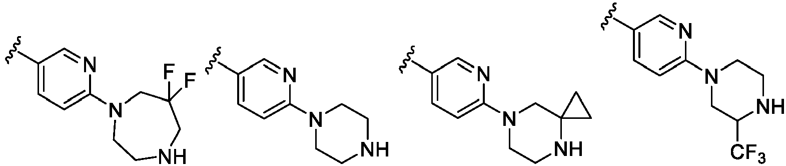 and 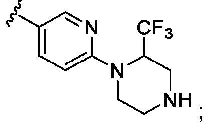
iv) A is

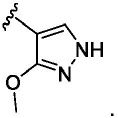

In one particular embodiment, i) as defined above is true. In another particular embodiment, ii) as defined above is true. In another particular embodiment, iii) as defined above is true. In another particular embodiment, iv) as defined above is true.

In a preferred embodiment, one of i) to iii) as defined above is true. In another particular embodiment, one of i) and ii) as defined above is true. In another particular embodiment, i) as defined above is true.

Thus, in particular preferred embodiments of the compounds of the present invention,
- A: is 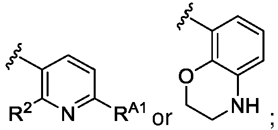
wherein
- R^{A1}: is piperazinyl, wherein the piperazinyl is unsubstituted or one substitutable carbon atom of the piperazinyl is substituted with one or more, same or different substituents R^{A3}; wherein
- R^{A3}: is F or CF₃,
or two R^{A3} attached to the same carbon atom form cyclopropyl;
and wherein preferably
- R^{A3}: is CF₃;
and
- R¹: is 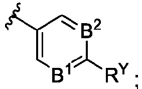
wherein
- B¹: is N;
- B²: is CH or N; and
- R^{Y}: is F, CN, CF₃, or cyclopropyl; preferably cyclopropyl;
and wherein
- R²: is halogen, S(=O)₂R^{S}, C₁-C₂-alkoxy, or C₁-C₂-haloalkyl, more preferably CI, CF₃, or OCH₃, particularly preferably OCH₃;
or
- A: is 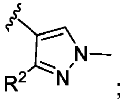 and
- R¹: is 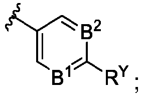
wherein
- B¹: is N;
- B²: is CH or N; and
- R^{Y}: piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃,
or two R^{Z} attached to the same carbon atom form cyclopropyl
and wherein
R² is halogen, S(=O)₂R^{S}, C₁-C₂-alkoxy, or C₁-C₂-haloalkyl, more preferably CI, CF₃, or OCH₃.

In more preferred embodiments,
- A: is 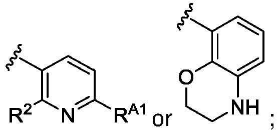
wherein
- R^{A1}: is piperazinyl, wherein the piperazinyl is unsubstituted or one substitutable carbon atom of the piperazinyl is substituted with one or more, same or different substituents R^{A3}; wherein
- R^{A3}: is F or CF₃,
or two R^{A3} attached to the same carbon atom form cyclopropyl;
and wherein preferably
- R^{A3}: is CF₃;
and
- R¹: is 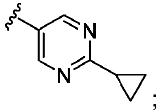
and wherein
- R²: is halogen, S(=O)₂R^{S}, C₁-C₂-alkoxy, or C₁-C₂-haloalkyl, more preferably CI, CF₃, or OCH₃, particularly preferably OCH₃;
or
- A: is 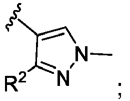 and
- R¹: is 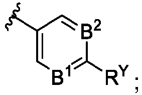
wherein
- B¹: is N;
- B²: is CH or N; and
- R^{Y}: piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃,
or two R^{Z} attached to the same carbon atom form cyclopropyl; and wherein
- R²: is halogen, S(=O)₂R^{S}, C₁-C₂-alkoxy, or C₁-C₂-haloalkyl, more preferably CI, CF₃, or OCH₃.

In one particularly preferred embodiment,
- A: is 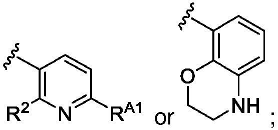
wherein
- R^{A1}: is piperazinyl, wherein the piperazinyl is unsubstituted or one substitutable carbon atom of the piperazinyl is substituted with one or more, same or different substituents R^{A3}; wherein
- R^{A3}: is F or CF₃,
or two R^{A3} attached to the same carbon atom form cyclopropyl;
and wherein preferably
- R^{A3}: is CF₃;
and
- R¹: is 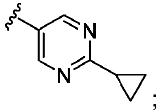
and wherein
- R²: is halogen, S(=O)₂R^{S}, C₁-C₂-alkoxy, or C₁-C₂-haloalkyl, more preferably CI, CF₃, or OCH₃, particularly preferably OCH₃.

In another particularly preferred embodiment,
- A: is 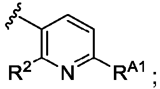
wherein
- R^{A1}: is piperazinyl, wherein the piperazinyl is unsubstituted or one substitutable carbon atom of the piperazinyl is substituted with one or more, same or different substituents R^{A3}; wherein
- R^{A3}: is F or CF₃,
or two R^{A3} attached to the same carbon atom form cyclopropyl;
and wherein more preferably
- R^{A3}: is CF₃;
and
- R¹: is 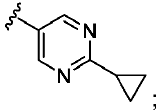
and wherein preferably,
- R²: is halogen, S(=O)₂R^{S}, C₁-C₂-alkoxy, or C₁-C₂-haloalkyl, more preferably CI, CF₃, or OCH₃, particularly preferably OCH₃.

In another particularly preferred embodiment,
- A: is 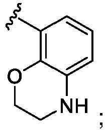 and
- R¹: is

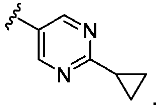

In another particularly preferred embodiment,
- A: is 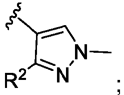 wherein
- R²: is Cl, CF₃, or OCH₃;
and
- R¹: is 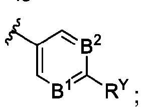
wherein
- B¹: is N;
- B²: is CH or N; and
- R^{Y}: piperazinyl, or 1,4-diazepane, wherein each substitutable carbon atom of the aforementioned piperazinyl or 1,4-diazepane is independently unsubstituted or substituted with one or more, same or different substituents R^{Z};
wherein
- R^{Z}: is F or CF₃,
or two R^{Z} attached to the same carbon atom form cyclopropyl.

In other particularly preferred embodiments, the compound of formula (I) is a compound selected from the group consisting of:
N-({4-amino-1H,3H-furo[3,4-c]quinolin-7-yl}methyl)-N-[1-methyl-3-(trifluoromethyl)-1H-pyrazol-4-yl]-6-(piperazin-1-yl)pyridine-3-carboxamide;
N-({4-amino-1H,3H-furo[3,4-c]quinolin-7-yl}methyl)-6-{4,7-diazaspiro[2.5]octan-7-yl}-N-[1-methyl-3-(trifluoromethyl)-1H-pyrazol-4-yl]pyridine-3-carboxamide;
N-({4-amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide;
N-({4-amino-1H,3H-furo[3,4-c]quinolin-7-yl}methyl)-2-cyclopropyl-N-(3,4-dihydro-2H-1,4-benzoxazin-8-yl)pyrimidine-5-carboxamide;
N-({4-amino-1H,3H-furo[3,4-c]quinolin-7-yl}methyl)-N-(3-methoxy-1-methyl-1H-pyrazol-4-yl)-6-[3-(trifluoromethyl)piperazin-1-yl]pyridine-3-carboxamide;
N-({4-amino-1H,3H-furo[3,4-c]quinolin-7-yl}methyl)-6-(6,6-difluoro-1,4-diazepan-1-yl)-N-(3-methoxy-1-methyl-1H-pyrazol-4-yl)pyridine-3-carboxamide;
N-({4-amino-1H,3H-furo[3,4-c]quinolin-7-yl}methyl)-N-(3-chloro-1-methyl-1H-pyrazol-4-yl)-6-[3-(trifluoromethyl)piperazin-1-yl]pyridine-3-carboxamide;
N-({4-amino-1H,3H-furo[3,4-c]quinolin-7-yl}methyl)-2-cyclopropyl-N-(3-methoxy-1H-pyrazol-4-yl)pyrimidine-5-carboxamide; and
N-({4-amino-1H,3H-furo[3,4-c]quinolin-7-yl}methyl)-N-(3-methoxy-1-methyl-1H-pyrazol-4-yl)-6-[2-(trifluoromethyl)piperazin-1-yl]pyridine-3-carboxamide.

### Definitions

The term "compound(s) of the present invention" is to be understood as equivalent to the term "compound(s) according to the invention", and also covers a salt, stereoisomer, rotamer, atropisomer, tautomer or N-oxide thereof.

The compounds according to the invention may be amorphous or may exist in one or more different crystalline states (polymorphs), which may have different macroscopic properties such as stability or show different biological properties such as activities. The present invention relates to amorphous and crystalline forms of compounds of formula (I), mixtures of different crystalline states of the compounds of formula (I), as well as amorphous or crystalline salts thereof.

Salts of the compounds according to the invention are preferably pharmaceutically acceptable salts, such as those containing counterions present in drug products listed in the US FDA Orange Book database. They can be formed in a customary manner, e.g., by reacting the compound with an acid of the anion in question, if the compounds according to the invention have a basic functionality, or by reacting acidic compounds according to the invention with a suitable base.

Suitable cationic counterions are in particular the ions of the alkali metals, preferably lithium, sodium and potassium, of the alkaline earth metals, preferably calcium, magnesium and barium, and of the transition metals, preferably manganese, copper, silver, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxy-ethylammonium, 2-(2-hydroxyethoxy)ethyl-ammonium, bis(2-hydroxyethyl)ammonium, benzyltrimethylammonium and benzyltriethylammonium, furthermore the cations of 1,4-piperazine, meglumine, benzathine and lysine.

Suitable anionic counterions are in particular chloride, bromide, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate, furthermore lactate, gluconate, and the anions of poly acids such as succinate, oxalate, maleate, fumarate, malate, tartrate and citrate, furthermore sulfonate anions such as besylate (benzenesulfonate), tosylate (p-toluenesulfonate), napsylate (naphthalene-2-sulfonate), mesylate (methanesulfonate), esylate (ethanesulfonate), and ethanedisulfonate. They can be formed by reacting compounds according to the invention that have a basic functionality with an acid of the corresponding anion.

Depending on the substitution pattern, the compounds according to the invention may have one or more centres of chirality, including axial chirality providing different stereoisomers. The invention provides both, pure enantiomers or pure diastereomers, of the compounds according to the invention, and their mixtures, including racemic mixtures.

Suitable compounds according to the invention also include all possible geometrical stereoisomers (cis/trans isomers or E/Z isomers) and mixtures thereof. E/Z- isomers may be present with respect to, e.g., an alkene, carbon-nitrogen double-bond or amide group.

Further, suitable compounds according to the invention also include conformational stereoisomers (conformers), which refers to isomers that interconvert by rotations about single bonds. Rotations about single bonds involve overcoming a rotational energy barrier to interconvert one conformer to another. If the energy barrier is high enough, rotation about the single bond can be hindered so that there is no free rotation and the compound exists as a rotational isomer (rotamer) for a relatively long time. If the energy barrier to rotation is high enough so that the time scale of interconversion is long enough to allow for isolation of individual rotamers, the rotamers are termed atropisomers. Thus, the term "atropisomers" as used herein refers to conformational stereoisomers (conformers) resulting from hindered rotation about a single bond, where the energy differences due to steric strain or other contributors create a barrier to rotation, which is high enough to allow for the isolation of the conformers. The term rotational isomers (rotamers) as used herein also includes atropisomers. Rotational isomers (rotamers), in particular atropisomers, do not require an asymmetric atom but an axis of chirality and thus represent a form of axial chirality. Determining the axial stereochemistry can be accomplished through the use of a Newman projection along the axis of hindered rotation. The four substituents are first assigned priority based on Cahn-Ingold-Prelog priority rules. Starting with the substituent of highest priority on the closest atom in the Newman projection and moving along the shortest path to the substituent of highest priority on the other atom, the absolute configuration is assigned P or Δ for clockwise and M or Λ for counterclockwise. Alternatively, all four groups can be ranked by Cahn-Ingold-Prelog priority rules, with overall priority given to the substituents on the "front" atom of the Newman projection, i.e., the substituents on the end of the single bond, which is closest in the Newman projection. The two configurations are termed *R*ₐ and *S*ₐ in analogy to the traditional R/S for a traditional tetrahedral stereocenter as exemplarily illustrated below in case of A having a higher priority than B, and C having a higher priority than D by Cahn-Ingold-Prelog priority rules.

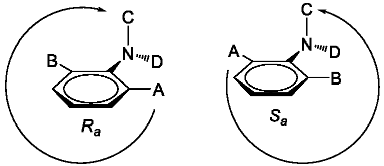

Further, rotational isomers or atropisomers resulting, e.g., from rotation about an amide bond may also be defined as cis/trans isomers or E/Z isomers. Thus, such cis/trans isomers and E/Z isomers are also covered by the term rotational isomer (rotamer) or atropisomer. The invention provides both, pure rotational isomers (rotamers) or atropisomers of the compounds according to the invention, and their mixtures, including racemic mixtures. For example, rotational isomers (rotamers) of the compounds of the present invention may be present with respect to a restricted rotation about the C-N amide bond and/or a restricted rotation about the C-N bond between the tertiary amide nitrogen and the A moiety.

However, if the barrier to rotation is high enough, the rotational isomers (rotamers) of the compounds according to the invention can be separated and isolated and can thus be termed atropisomers. In particular, depending on the substitution pattern, the barrier to rotation about the C-N bond between the tertiary amide nitrogen and the A moiety of the compounds according to the invention may be high enough to form atropisomers, which can be separated and isolated.The mixture may be racemic (1 to 1 mixture) or one of the rotational isomers (rotamers) in the mixture may be enriched over the other rotational isomer (rotamer). If the barrier to rotation is high enough to form atropisomers, the two atropisomers may be separated and isolated. Further, the compounds of the present invention may be present as a mixture of stereoisomers resulting from more than one restricted bond rotation. For example, if there are two restricted bond rotations, there may be four rotational isomers (rotamers), which can be grouped into two "diastereoisomer-like" components that are distinguishable by NMR analysis. Such a mixture is referred to as "diastereoisomer-like" mixture and the stereoisomers therein which are distinguishable by NMR analysis are referred to as "diastereoisomer-like" components. If one of the two barriers to rotation is high enough to form atropisomers, e.g. the barrier to rotation about the C-N bond between the tertiary amide nitrogen and the A moiety of the compounds according to the invention, the two atropisomers may be separated and isolated. Each of the separated and isolated atropisomers may then be present as a mixture of two rotational isomers (rotamers) resulting from the second restricted bond rotation, e.g. the rotation about the C-N amide bond. A "diastereoisomer-like" mixture with "diastereoisomer-like" distinguishable components may also result if the compounds of the present invention contain a combination of one or more restricted bond rotation(s) and one or more asymmetric atom(s) (i.e., chiral centres). For example, there may be one chiral centre resulting in two stereoisomers, and additionally at least one restricted bond rotation, e.g., the restricted rotation about the C-N amide bond, resulting in two additional stereoisomers, i.e. rotational isomers (rotamers). A compound of the present invention may thus be present as a mixture of four stereoisomers (or even 8 stereoisomers in case of two restricted bond rotations), which can be grouped into "diastereoisomer-like" components that are distinguishable by NMR analysis. If, e.g., the stereoisomers resulting from the chiral centre are separated and isolated, they may then be present as a mixture of the rotational isomers (rotamers) resulting from the additional restricted bond rotation(s), e.g. the rotation about the C-N amide bond. The skilled person is aware that the presence of further centres of chirality and/or further restricted bond rotations (chirality axes) increases the number of stereoisomers present in the mixture up to a maximum of 2ⁿ, with n being the number of chiral centres including chiral atoms and chiral axes (restricted bond rotations).

Atropisomers may be classified according to their stereochemical stabilities as mentioned in doi: 10.4155/fmc-2017-0152: Class 1 atropisomers possess barriers to rotation around the chiral axis of <84 kJ/mol(20 kcal/mol) and racemize on the minute or faster time scale at room temperature; class 2 atropisomers possess a barrier to rotation between 84 and 117 kJ/mol (20-28 kcal/mol) and racemize on the hour to month timescale at room temperature; and class 3 atropisomers possess a barrier to rotation>1 17 kJ/mol (28 kcal/mol) and racemize on the year or greater timescale at room temperature. Typically, only class 2 and class 3 atropisomers are separable. Without being bound to theory, class 2 or class 3 atropisomers of the compounds of the invention may be present, if the A moiety of the compounds of the present invention is substituted in ortho position on both sides of the carbon connected to the tertiary amide nitrogen of the remainder of the molecule. For class 2 atropisomers, it may be possible to distinguish the atropisomers in NMR or short time LC, but they may nevertheless be inseparable due to the fact that a single atropisomer would reequilibrate to a mixture within a few hours. ¹H NMR at 80 °C may be useful to identify stable atropisomers if their signals do not significantly coalesce at this temperature.

Tautomers may be formed, if a substituent is present at the compound of formula (I), which allows for the formation of tautomers such as keto-enol tautomers, imine-enamine tautomers, amide-imidic acid tautomers or the like.

The term "N-oxide" includes any compound of the present invention which has at least one tertiary nitrogen atom that is oxidized to an N-oxide moiety.

Any formula or structure given herein, including compounds of formula (I), is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, phosphorous, fluorine and chlorine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl and ¹²⁵I. For example, radioactive isotopes such as ³H, ¹³C and ¹⁴C provide isotopically labelled compounds useful in metabolic studies, reaction kinetic studies, detection or imaging techniques, such as positron emission tomography (PET) or single-photon emission computed tomography (SPECT) including drug or substrate tissue distribution assays or in radioactive treatment of patients. Further, the disclosure includes compounds of formula (I) in which from 1 to n hydrogens attached to a carbon atom is/are replaced by deuterium, in which n is the number of hydrogens in the molecule. Deuterium labeled or substituted therapeutic compounds of the disclosure may have improved DMPK (drug metabolism and pharmacokinetics) properties, relating to distribution, metabolism and excretion (ADME). Substitution with heavier isotopes such as deuterium may afford certain therapeutic advantages resulting from greater metabolic stability, for example increased in vivo half-life, reduced dosage requirements and/or an improvement in therapeutic index. See, for example, Poster, "Deuterium Isotope Effects in Studies of DrugMetabolism", Trends Pharmacol. Sei. 5(12):524-527 (1984). Such compounds are synthesized by means well known in the art, for example by employing starting materials in which one or more hydrogens have been replaced by deuterium. The concentration of such a heavier isotope, specifically deuterium, may be defined by an isotopic enrichment factor. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium.

The term "substituted", as used herein, means that a hydrogen atom bonded to a designated atom is replaced with a specified substituent, provided that the substitution results in a stable or chemically feasible compound. Unless otherwise indicated, a substituted atom may have one or more substituents and each substituent is independently selected.

The term "substitutable", when used in reference to a designated atom, means that attached to the atom is a hydrogen, which can be replaced with a suitable substituent.

When it is referred to certain atoms or moieties being substituted with "one or more" substituents, the term "one or more" is intended to cover at least one substituent, e.g. 1 to 10 substituents, preferably 1, 2, 3, 4, or 5 substituents, more preferably 1, 2, or 3 substituents, most preferably 1, or 2 substituents. When neither the term "unsubstituted" nor "substituted" is explicitly mentioned concerning a moiety, said moiety is to be considered as unsubstituted.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine, or bromine.

The term "alkyl" as used herein denotes in each case a straight-chain or branched alkyl group having usually from 1 to 6 carbon atoms, preferably 1 to 5 or 1 to 4 carbon atoms, more preferably 1 to 3 or 1 or 2 carbon atoms. Examples of an alkyl group are methyl, ethyl, n-propyl, isopropyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein denotes in each case a straight-chain or branched alkyl group having usually from 1 to 4 carbon atoms, preferably 1 to 3 or 1 or 2 carbon atoms, wherein the hydrogen atoms of this group are partially or totally replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like.

The term "alkoxy" as used herein denotes in each case a straight-chain or branched alkyl group which is bonded via an oxygen atom to the remainder of the molecule and has usually from 1 to 4 carbon atoms, preferably 1 to 2 carbon atoms, more preferably 1 carbon atom. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to an alkoxy group as defined herein having usually from 1 to 4 carbon atoms, preferably 1 to 2 carbon atoms, more preferably 1 carbon atom, which is bonded via an alkyl group as defined herein having usually from 1 to 4 carbon atoms, preferably 1 to 2 carbon atoms, more preferably 1 carbon atom, to the remainder of the molecule. Thus, it refers to an alkyl group, which is bonded via oxygen to a further alkyl group, which is then bonded to the remainder of the molecule. Examples of an alkoxyalkyl group are methoxymethyl, methoxyethyl, ethoxymethyl, ethoxyethyl, and the like.

The term "haloalkoxy" as used herein denotes in each case a straight-chain or branched alkoxy group having from 1 to 4 carbon atoms, preferably 1 to 2 carbon atoms, more preferably 1 carbon atom, wherein the hydrogen atoms of this group are partially or totally replaced with halogen atoms, in particular fluorine atoms. Preferred haloalkoxy moieties include C₁-haloalkoxy, in particular C₁-fluoroalkoxy, such as trifluoromethoxy and the like.

The term "hydroxyalkyl" as used herein denotes in each case a straight-chain or branched alkyl group having usually from 1 to 6 carbon atoms, preferably from 1 to 4 carbon atoms, more preferably 1 to 2 carbon atoms, and being further substituted with 1 to 5, preferably with 1 to 2 hydroxy groups, in particular with 1 hydroxy group, wherein a hydroxy group is a OH group. Preferably, the one hydroxy group is terminating the straight-chain or branched alkyl group so that the hydroxy group is bonded to an alkyl bridge, which is bonded to the remainder of the molecule. Examples of an hydroxyalkyl group are hydroxymethyl, hydroxyethyl, n-hydroxypropyl, 2-hydroxypropyl, n-hydroxybutyl, 2-hydroxybutyl, 2-hydroxy-2-methylpropyl, and n-hydroxypentyl. Hydroxymethyl, hydroxyethyl, hydroxypropyl, and hydroxybutyl, are preferred, in particular hydroxymethyl and hydroxyethyl.

The term "aminoalkyl" as used herein denotes in each case a straight-chain or branched alkyl group having usually from 1 to 6 carbon atoms, preferably from 1 to 4 carbon atoms, more preferably 1 to 2 carbon atoms, and being further substituted with 1 to 5, preferably with 1 to 2 amino groups, in particular with 1 amino group, wherein an amino group is a NH₂ group. Preferably, the one amino group is terminating the straight-chain or branched alkyl group so that the amino group is bonded to an alkyl bridge, which is bonded to the remainder of the molecule. Examples of an aminoalkyl group are aminomethyl, aminoethyl, n-aminopropyl, 2-aminopropyl, n-aminobutyl, 2-aminobutyl, 2-amino-2-methylpropyl, and n-aminopentyl. Aminomethyl, aminoethyl, aminopropyl, and aminobutyl, are preferred, in particular aminomethyl and aminoethyl.

The term "cycloalkyl" as used herein denotes in each case a monocyclic cycloaliphatic radical having usually from 3 to 10 or from 3 to 6 carbon atoms, such as cyclopropyl, cyclobutyl, cyclopentyl, cyclohexyl, cycloheptyl, cyclooctyl, cyclononyl and cyclodecyl or cyclopropyl, cyclobutyl, cyclopentyl and cyclohexyl. Cyclopropyl, cyclobutyl, cyclopentyl, and cyclohexyl are preferred.

The term "carbocyclic", "carbocyclyl", or "carbocycle" includes, unless otherwise indicated, in general a 3- to 10-membered monocyclic ring, preferably a 4- to 8-membered or a 3- to 6-membered or a 5- to 7-membered monocyclic ring, more preferably a 3-, 4-, 5- or 6-membered monocyclic ring, comprising 3 to 10, preferably 4 to 8 or 3 to 6 or 5 to 7, more preferably 3, 4, 5 or 6 carbon atoms. The carbocycle may be saturated, partially or fully unsaturated, or aromatic, wherein saturated means that only single bonds are present, and partially or fully unsaturated means that one or more double bonds may be present in suitable positions, while the Hückel rule for aromaticity is not fulfilled, whereas aromatic means that the Hückel (4n + 2) rule is fulfilled. Also "aryls" are covered by the term "carbocycles". The term "aryl" or "aromatic carbocycle" refers to aromatic carbocyclic rings based on carbon atoms as ring members, preferably 6-membered aromatic carbocyclic rings based on carbon atoms as ring members. A preferred example is phenyl. Unless otherwise indicated, the term "aryl" further covers "aromatic carbobicycles" as defined herein. The term "carbocyclic" or "carbocyclyl", unless otherwise indicated, may therefore cover inter alia cycloalkyl, cycloalkenyl, as well as phenyl. Preferably, the term "carbocyclic" or "carbocyclyl" covers phenyl and cycloalkyl, for example phenyl, cyclopropyl, cyclobutyl, cyclopentyl, and cyclohexyl.

The term "carbobicyclic" or "carbobicyclyl" includes in general 6 to 14-membered, preferably 7-to 12-membered or 8- to 10-membered, more preferably 9- or 10-membered bicyclic rings comprising 6 to 14, preferably 7 to 12 or 8 to 10, more preferably 9 or 10 carbon atoms. The carbobicycle may be saturated, partially or fully unsaturated, or aromatic, wherein saturated means that only single bonds are present, and partially or fully unsaturated means that one or more double bonds may be present in suitable positions, while the Hückel rule for aromaticity is not fulfilled, whereas aromatic means that the Hückel (4n + 2) rule is fulfilled. Preferably, the term "aromatic" in connection with the carbobicyclic ring means that both rings of the bicylic moiety are aromatic, so that, e.g., 8 π electrons are present in case of a 10-membered aromatic carbobicyclic ring.The term "carbobicylce" or "carbobicyclyl", unless otherwise indicated, may therefore cover inter alia bicycloalkyl, bicycloalkenyl, as well as bicyclic aromatic groups, for example bicyclohexane (decalin), bicycloheptane (such as norbornane), bicyclooctane (such as bicyclo[2.2.2]octane, bicyclo[3.2.1]octane or bicyclo[4.2.0]octane), bicyclononane (such as bicyclo[3.3.1]nonane or bicyclo[4.3.0]nonane ), bicyclodecane (such as bicyclo[4.4.0]decane), bicycloundecane (such as bicyclo[3.3.3]undecane), norbornene, naphthalene and the like. Preferably, the carbo bicycle is a fused carbobicycle, which is preferably aromatic, for example naphthalene.

The term "carbocyclylalkyl" as used herein, refers to carbocyclyl as defined herein, which is bonded to the remainder of the molecule via an alkyl group having usually from 1 to 2 carbon atoms, preferably 1 carbon atom. Preferably, the term "carbocyclylalkyl" refers to phenylalkyl or cycloalkylalkyl, which refers to the corresponding groups being bonded to the remainder of the molecule via an alkyl group. Preferred examples of carbocyclylalkyl include benzyl (i.e. phenylmethyl), phenylethyl, cyclopropylmethyl, cyclopropylethyl, cyclobutylmethyl, cyclobutylethyl, cyclopentylmethyl, cyclopentylethyl, cyclohexylmethyl, cyclohexylethyl.

The term "carbocyclyloxy" as used herein denotes in each case a carbocyclyl as defined herein, which is bonded via an oxygen atom to the remainder of the molecule. Examples of carbocyclyloxy include phenyloxy or cyclopropyloxy. The same applies to the terms "aryloxy" and "benzyloxy" referring to the corresponding groups, which are bonded to the remainder of the molecule via an oxygen atom.

The term "carbocyclyloxyalkyl" as used herein denotes in each case a carbocyclyloxy group, which is bonded to the remainder of the molecule via an alkyl group having usually from 1 to 2 carbon atoms, preferably 1 carbon atom. Thus, it refers to a carbocyclyl, which is bonded via an oxygen atom to an alkyl group having usually from 1 to 2 carbon atoms, preferably 1 carbon atom, which is then bonded to the remainder of the molecule. Examples of include phenyloxymethyl, phenyloxyethyl, cyclopropyloxymethyl, and cyclopropyloxyethyl.

The term "heterocyclic" or "heterocyclyl" includes, unless otherwise indicated, in general a 3-to 10-membered, preferably a 4- to 8-membered or 5- to 7-membered, more preferably 5- or 6-membered, in particular 6-membered monocyclic ring. The heterocycle may be saturated, partially or fully unsaturated, or aromatic, wherein saturated means that only single bonds are present, and partially or fully unsaturated means that one or more double bonds may be present in suitable positions, while the Hückel rule for aromaticity is not fulfilled, whereas aromatic means that the Hückel (4n + 2) rule is fulfilled. The heterocycle typically comprises one or more, e.g. 1, 2, 3, or 4, preferably 1, 2, or 3 heteroatoms selected from N, O and S as ring members, where S-atoms as ring members may be present as S, SO or SO₂. The remaining ring members are carbon atoms. In a preferred embodiment, the heterocycle is an aromatic heterocycle, preferably a 5- or 6-membered aromatic heterocycle comprising one or more, e.g. 1, 2, 3, or 4, preferably 1, 2, or 3 heteroatoms selected from N, O and S as ring members, where S-atoms as ring members may be present as S, SO or SO₂. Examples of aromatic heterocycles are provided below in connection with the definition of "hetaryl". "Hetaryls" or "heteroaryls" are covered by the term "heterocycles". The saturated or partially or fully unsaturated heterocycles usually comprise 1, 2, 3, 4 or 5, preferably 1, 2 or 3 heteroatoms selected from N, O and S as ring members, where S-atoms as ring members may be present as S, SO or SO₂. The skilled person is aware that S, SO or SO₂ is to be understood as follows:

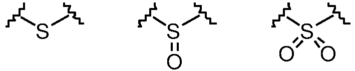

Further, a skilled person is aware that resonance structures of the oxidized forms may be possible. Saturated heterocycles include, unless otherwise indicated, in general 3- to 10-membered, preferably 4- to 8-membered or 5- to 7-membered, more preferably 5- or 6-membered monocyclic rings comprising 3 to 10, preferably 4 to 8 or 5 to 7, more preferably 5 or 6 atoms comprising at least one heteroatom, such as pyrrolidine, tetrahydrothiophene, tetrahydrofuran, piperidine, tetrahydropyran, dioxane, morpholine or piperazine.

The term "heterobicyclic" or "heterobicyclyl" includes, unless otherwise indicated, in general 6 to 14-membered, preferably 7- to 12-membered or 8- to 10-membered, more preferably 8- or 9-membered bicyclic rings. The heterobicycle may be saturated, partially or fully unsaturated, or aromatic, wherein saturated means that only single bonds are present, and partially or fully unsaturated means that one or more double bonds may be present in suitable positions, while the Hückel rule for aromaticity is not fulfilled, whereas aromatic means that the Hückel (4n + 2) rule is fulfilled. For being "aromatic", it is sufficient if one of the two rings of the bicyclic moieties is aromatic, while the other is non-aromatic. The heterobicycle typically comprises one or more, e.g. 1, 2, 3, or 4, preferably 1, 2, or 3 heteroatoms selected from N, O and S as ring members, where S-atoms as ring members may be present as S, SO or SO₂. The remaining ring members are carbon atoms. Examples of heterobicycles include benzofuranyl, benzothienyl, indolyl, indazolyl, benzimidazolyl, benzoxathiazolyl, benzoxadiazolyl, benzothiadiazolyl, benzoxazinyl, quinolinyl, isoquinolinyl, purinyl, 1,8-naphthyridyl, pteridyl, pyrido[3,2-d]pyrimidyl, pyridoimidazolyl, triethylenediamine or quinuclidine and the like.

The term "hetaryl" or "heteroaryl" or "aromatic heterocycle" or "aromatic heterocyclic ring" includes monocyclic 5- or 6-membered aromatic heterocycles comprising as ring members 1, 2, 3 or 4 heteroatoms selected from N, O and S, where S-atoms as ring members may be present as S, SO or SO₂. Examples of 5- or 6-membered aromatic heterocycles include pyridyl (also referred to as pyridinyl), i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl. Unless otherwise indicated, the term "hetaryl" further covers "aromatic heterobicycles" as defined above.

The term "heterocyclylalkyl" as used herein, refers to a heterocyclyl as defined herein, which is bonded to the remainder of the molecule via an alkyl group having usually from 1 to 2 carbon atoms, preferably 1 carbon atom. Examples of heterocyclylalkyl include pyridinylmethyl, pyrimidinylmethyl, pyrazolylmethyl, and piperidinylmethyl.

The term "heterocyclyloxy" as used herein denotes in each case a heterocyclyl as defined herein, which is bonded via an oxygen atom to the remainder of the molecule. Preferably, a carbon atom of the heterocyclyl is bonded to the oxygen atom. Examples heterocyclyloxy include pyridinyloxy, pyrimidinyloxy, pyrazolyloxy, and piperidinyloxy. The same applies to the terms "heteroaryloxy" referring to the corresponding group, which is bonded to the remainder of the molecule via an oxygen atom.

The term "heterocyclyloxyalkyl" as used herein denotes in each case a heterocyclyloxy group, which is bonded to the remainder of the molecule via an alkyl group having usually from 1 to 2 carbon atoms, preferably 1 carbon atom. Thus, it refers to a heterocyclyl, which is bonded via an oxygen atom to an alkyl group having usually from 1 to 2 carbon atoms, preferably 1 carbon atom, which is then bonded to the remainder of the molecule. Examples include pyridinyloxymethyl, pyrimidinyloxymethyl, pyrazolyloxymethyl, and piperidinyloxymethyl.

The term "cyclic" moiety can refer to any cyclic groups, which are present in the compounds of formula (I), and which are defined above, e.g., cycloalkyl, cycloalkenyl, carbocyclyl.

The term "bicyclic" moiety can refer to any bicyclic groups, which are present in the compounds of formula (I), and which are defined above.

As used in the specification and the claims, the singular forms of "a" and "an" also include the corresponding plurals unless the context clearly dictates otherwise. The same applies for plural forms used herein, which also include the singular forms unless the context clearly dictates otherwise.

The terms "about" and "approximately" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising of'. If hereinafter a group is defined to comprise at least a certain number of embodiments, this is also meant to encompass a group which preferably consists of these embodiments only.

The term "pharmaceutically acceptable excipient" as used herein refers to compounds commonly comprised in pharmaceutical compositions, which are known to the skilled person. Examples of suitable excipients are exemplary listed below. Typically, a pharmaceutically acceptable excipient can be defined as being pharmaceutically inactive.

The term "treatment" is to be understood as also including the option of "prophylaxis". Thus, whenever reference is made herein to a "treatment" or "treating", this is to be understood as "treatment and/or prophylaxis" or "treating and/or preventing".

### Description of pharmaceutical compositions according to the present invention

A pharmaceutical composition according to the present invention may be formulated for oral, inhalative, buccal, nasal, rectal, topical, transdermal or parenteral application. Preferred non-parenteral routes include mucosal (e.g., oral, vaginal, nasal, cervical, etc.) routes, of which the oral application may be preferred. Parenteral application may be preferred and includes intravenous, intraarterial, intratumoral, peri-tumoral, intradermal, intrathecal, intravesical, intramuscular, epidural or subcutaneous administration, but also intranasal and inhalative administration. Preferably administration is by subcutaneous, intra-tumoral or peri-tumoral routes, in particular in the treatment of cancer. Particularly preferred is intratumoral administration. In connection with the treatment of pulmonary diseases, such as asthma, chronic obstructive pulmonary disease, lung cancer and idiopathic pulmonary fibrosis, inhalative administration is preferred. Inhalative administration may be performed by using an inhaler for delivering the pharmaceutical composition into the body via the lungs.

Suitable inhalers include dry powder inhalers, metered-dose inhalers, and nebulizers. Dry powder inhalers provide the active ingredient in the form of a powder, which is then inhaled through the dry powder inhaler. Dry powder inhalers are advantageous because they are breath-actuated and do not require the use of any propellants. Nebulizers provide the active ingredient as an aerosol created from an aqueous formulation. Metered-dose inhalers release a fixed dose of medication in aerosol form, wherein a liquefied gas propellant, preferably a hydro-fluoroalkane (HFA), is used in the formulation of the active ingredient.

The compound according to formula (I) should be applied in pharmaceutically effective amounts, for example in the amounts as set out herein below.

A pharmaceutical composition of the present invention may also be designated as formulation or dosage form. A compound of formula (I) may also be designated in the following as (pharmaceutically) active agent or active compound.

Pharmaceutical compositions may be solid or liquid dosage forms or may have an intermediate, e.g. gel-like character depending inter alia on the route of administration.

In general, the inventive dosage forms can comprise various pharmaceutically acceptable excipients, which will be selected depending on which functionality is to be achieved for the dosage form. A "pharmaceutically acceptable excipient" in the meaning of the present invention can be any substance used for the preparation of pharmaceutical dosage forms, including coating materials, film-forming materials, fillers, disintegrating agents, release-modifying materials, carrier materials, diluents, binding agents, and other adjuvants. Typical pharmaceutically acceptable excipients include substances like sucrose, mannitol, sorbitol, starch and starch derivatives, lactose, and lubricating agents such as magnesium stearate, disintegrants and buffering agents. The term "carrier" denotes pharmaceutically acceptable organic or inorganic carrier substances with which the active ingredient is combined to facilitate the application. Suitable pharmaceutically acceptable carriers include, for instance, water, aqueous salt solutions, alcohols, oils, preferably vegetable oils, propylene glycol, polyoxyethelene sorbitans, polyethylene-polypropylene block co-polymers such as poloxamer 188 or poloxamer 407, polyethylene glycols such as polyethylene glycol 200, 300, 400, 600, etc., gelatin, lactose, amylose, magnesium stearate, surfactants, perfume oil, fatty acid monoglycerides, diglycerides and triglycerides, polyoxyethylated medium or long chain fatty acids such as ricinoleic acid, and polyoxyethylated fatty acid mono-, di, and triglycerides such as capric or caprilic acids, petroethral fatty acid esters, hydroxymethyl celluloses such as hydroxymethyl, hydroxyethyl, hydroxypropyl, hydroxypropyl acetate succinate, polyvinylpyrrolidone, crosspovidone and the like. Preferably, the compounds of the present invention are administered in a pharmaceutical composition comprising of lipids, interbilayer crosslinked multilamellar vesicles, biodegradeable poly(D,L-lactic-co-glycolic acid) [PLGA]-based or poly anhydride-based nanoparticles or microparticles, nanoporous particle-supported lipid bilayers and as a conjugate with an antibody.

The pharmaceutical compositions can be sterile and, if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compound. It is to be understood that the term "carrier" also covers an antibody that delivers the compound of formula (I).

If liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavoring agents.

For parenteral application, particularly suitable vehicles consist of solutions, preferably oily or aqueous solutions, as well as suspensions, emulsions, or implants. Pharmaceutical formulations for parenteral administration are particularly preferred and include aqueous solutions of the compounds of formula (I) in water-soluble form. Additionally, suspensions of the compounds of formula (I) may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran.

Particularly preferred dosage forms are injectable preparations of a compound of formula (I). Thus, sterile injectable aqueous or oleaginous suspensions can for example be formulated according to the known art using suitable dispersing agents, wetting agents and/or suspending agents. A sterile injectable preparation can also be a sterile injectable solution or suspension in a non-toxic parenterally acceptable diluent or solvent. Among the acceptable vehicles and solvents that can be used are water and isotonic sodium chloride solution. Sterile oils are also conventionally used as solvent or suspending medium. Preferred applications for injectable preparations comprising the compounds of the present invention are intravenous, intratumoral and peritumoral administration.

Suppositories for rectal administration of a compound of formula (I) can be prepared by e.g. mixing the compound with a suitable non-irritating excipient such as cocoa butter, synthetic triglycerides and polyethylene glycols which are solid at room temperature but liquid at rectal temperature such that they will melt in the rectum and release the compound according to formula (I) from said suppositories.

For administration by inhalation, the compounds according to the present invention may be conveniently delivered in the form of an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Oral dosage forms may be liquid or solid and include e.g. tablets, troches, pills, capsules, powders, effervescent formulations, dragees and granules. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. The oral dosage forms may be formulated to ensure an immediate release of the compound of formula (I) or a sustained release of the compound of formula (I).

A solid dosage form may comprise a film coating. For example, the inventive dosage form may be in the form of a so-called film tablet. A capsule of the invention may be a two-piece hard gelatin capsule, a two-piece hydroxypropylmethylcellulose capsule, a two-piece capsule made of vegetable or plant-based cellulose or a two-piece capsule made of polysaccharide.

The dosage form according to the invention may be formulated for topical application. Suitable pharmaceutical application forms for such an application may be a topical nasal spray, sublingual administration forms and controlled and/or sustained release skin patches. For buccal administration, the compositions may take the form of tablets or lozenges formulated in conventional manner.

The compositions may conveniently be presented in unit dosage forms and may be prepared by any of the methods well known in the art of pharmacy. The methods can include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product. Liquid dose units are vials or ampoules. Solid dose units are tablets, capsules and suppositories.

As regards human patients, the compound of formula (I) may be administered to a patient in an amount of about 0.001 mg to about 5000 mg per day, preferably of about 0.01 mg to about 1000 mg per day, more preferably of about 0.05 mg to about 250 mg per day, which is the effective amount. The phrase "effective amount" means an amount of compound that, when administered to a mammal in need of such treatment, is sufficient to treat or prevent a particular disease or condition.

Furthermore, the pharmaceutical composition may also contain the compound of formula (I) as a prodrug such as an ester or amide thereof. A prodrug is any compound which is converted under physiological conditions or by solvolysis to any of the compounds of the invention. A prodrug may be inactive prior to administration but may be converted to an active compound of the invention *in vivo.*

In a preferred embodiment relating to the pharmaceutical compositions of the present invention, said pharmaceutical composition comprises said compound as the only pharmaceutically active agent. Alternatively, said pharmaceutical composition comprises at least one further independent pharmaceutically active agent in addition to said compound, wherein said additional active agent is typically used for the intended indication(s) as outlined above. In particular, when it comes to cancer, said pharmaceutical composition comprises at least one further independent pharmaceutically active agent in addition to the compound of the present invention. Further details in this regard are provided below.

Indications, for which the compounds of the present invention may be used

The compounds according to the present invention are suitable for use in medicine. The compounds of the present invention are useful for (partially) inhibiting PRMT5, in particular MTA-bound PRMT5, which is enriched in MTAP deficient tumor cells.

Thus, the compounds according to the present invention are particularly suitable for use in the treatment of a disease associated with MTAP deficiency and/or MTA accumulation, in particular a proliferative disorder such as cancer or pre-cancerous syndromes associated with MTAP deficiency and/or MTA accumulation.

Thus, in one embodiment, the compound of the present invention or a pharmaceutical composition comprising the same is for use in the treatment of a disease selected from the group consisting of cancer or pre-cancerous syndromes. In another embodiment, the compound of the present invention or a pharmaceutical composition comprising the same is for use in the treatment of a disease selected from the group consisting of cancer or pre-cancerous syndromes associated with MTAP deficiency and/or MTA accumulation.

Preferably, said cancer is selected from the group consisting of Glioblastoma, Non-Small Cell Lung Cancer, B-Lymphoblastic Leukemia/Lymphoma, Pancreatic Cancer, Breast Cancer, Melanoma, Esophagogastric Cancer, Bladder Cancer, Glioma, Head and Neck Cancer, Hepatobiliary Cancer, Prostate Cancer, Pleural Mesothelioma, Sarcoma, Ovarian Epithelial Tumor, Soft Tissue Sarcoma, Bone Cancer, Colorectal Cancer, Bladder/Urinary Tract Cancer, Ovarian Cancer, T-Lymphoblastic Leukemia/Lymphoma, Nerve Sheath Tumor, Mature B-Cell Neoplasms, Renal Non-Clear Cell Carcinoma, Renal Cell Carcinoma, Endometrial Cancer, Mature B-cell lymphoma, High-grade glioma/astrocytoma, Renal Clear Cell Carcinoma, Prostate Adenocarcinoma, Adrenocortical Carcinoma, Salivary Gland Cancer, Invasive Breast Carcinoma, Cholangiocarcinoma, Gastrointestinal Stromal Tumor, Lung Cancer, Low-grade glioma/astrocytoma, Thymic Epithelial Tumor, Salivary Cancer, Intraductal Papillary Mucinous Neoplasm, Leukemia, Mesothelioma, Sex Cord Stromal Tumor, CNS Cancer, Embryonal Tumor, Skin Cancer, Cervical Cancer, Thyroid Cancer, Anal Cancer, Peripheral Nervous System.

In particular, said cancer is preferably selected from the group consisting of Glioblastoma Multiforme, B-Lymphoblastic Leukemia/Lymphoma, Pancreatic Adenocarcinoma, Breast Invasive Ductal Carcinoma, Lung Squamous Cell Carcinoma, Bladder Urothelial Carcinoma, Lung Adenocarcinoma, Cutaneous Melanoma, Head and Neck Squamous Cell Carcinoma, Melanoma, Prostate Adenocarcinoma, Esophageal Adenocarcinoma, Astrocytoma, Serous Ovarian Cancer, Stomach Adenocarcinoma, Esophageal Squamous Cell Carcinoma, Hepatocellular Carcinoma, Pancreatic Neuroendocrine Tumor, Glioblastoma, Pleural Mesothelioma, Epithelioid Type Diffuse Large B-Cell Lymphoma, Tubular Stomach Adenocarcinoma, Bladder/Urinary Tract Cancer, Invasive Breast Carcinoma, Colon Adenocarcinoma, Hepatocellular Adenoma, Malignant Peripheral Nerve Sheath Tumor, Papillary Renal Cell Carcinoma, Pleural Mesothelioma Biphasic Type, Bladder Squamous Cell Carcinoma, T-Lymphoblastic Leukemia/Lymphoma, Leiomyosarcoma, Undifferentiated Pleomorphic Sarcoma/Malignant Fibrous Histiocytoma/High-Grade Spindle Cell Sarcoma, Sarcoma, Osteosarcoma, Renal Clear Cell Carcinoma, Intestinal Type Stomach Adenocarcinoma, Adenoid Cystic Carcinoma, Oligodendroglioma, Breast Invasive Lobular Carcinoma, Cholangiocarcinoma, Pediatric High Grade Gliomas, Acral Melanoma, Oligoastrocytoma, Breast Invasive Carcinoma, Adrenocortical Carcinoma, Breast Mixed Ductal and Lobular Carcinoma, Gastrointestinal Stromal Tumor, Thymoma, Dedifferentiated Liposarcoma, Angiosarcoma, Diffuse Type Stomach Adenocarcinoma, Intracholecystic Papillary Neoplasm, Pediatric Low Grade Gliomas, Intrahepatic Cholangiocarcinoma, Ewing Sarcoma, Intraductal Papillary Mucinous Neoplasm, Myxofibrosarcoma, Chordoma, Papillary Stomach Adenocarcinoma, B-Lymphoblastic Leukemia/Lymphoma BCR-ABL1 Like, Acute Myeloid Leukemia, Metaplastic Breast Cancer, High-Grade Serous Ovarian Cancer, Low-Grade Serous Ovarian Cancer, Liposarcoma, Adenocarcinoma of the Gastroesophageal Junction, Granulosa Cell Tumor, Breast Invasive Cancer, Mucinous Stomach Adenocarcinoma, Medulloblastoma, Adamantinoma, Breast Invasive Mixed Mucinous Carcinoma, Cervical Squamous Cell Carcinoma, Synovial Sarcoma, Adenosquamous Carcinoma of the Pancreas, Rectal Adenocarcinoma, Mucinous Adenocarcinoma of the Colon and Rectum, Chromophobe Renal Cell Carcinoma, Peritoneal Mesothelioma, Uterine Carcinosarcoma/Uterine Malignant Mixed Mullerian Tumor, Anal Squamous Cell Carcinoma, Papillary Thyroid Cancer, Oral Cavity Squamous Cell Carcinoma, Pleural Mesothelioma Sarcomatoid Type, Anaplastic Ependymoma, Sweat Gland Carcinoma/Apocrine Eccrine Carcinoma, Poorly differentiated Non-Small Cell Lung Cancer, Sarcomatoid Carcinoma of the Lung, Neuroblastoma, Pleural Mesothelioma, Pilocytic Astrocytoma, Signet Ring Cell Carcinoma of the Stomach, Ganglioglioma, Collecting Duct Renal Cell Carcinoma, Small Cell Carcinoma of the Ovary, Non-Small Cell Lung Cancer, Pancreatoblastoma, Ependymomal Tumor, Adenosquamous Carcinoma of the Stomach, Carcinoma with Osseous Metaplasia, Fibroblastic Osteosarcoma, Adenocarcinoma, B-Lymphoblastic Leukemia/Lymphoma with t(9;22)(q34.1 ;q11.2);BCR-ABL1, Early T-Cell Precursor Lymphoblastic Leukemia.

More preferably, the cancer is selected from the group consisting of Glioblastoma, Non-Small Cell Lung Cancer, B-Lymphoblastic Leukemia/Lymphoma, Pancreatic Cancer, Breast Cancer, Melanoma, Esophagogastric Cancer, Bladder Cancer, Glioma, Head and Neck Cancer, Hepatobiliary Cancer, Prostate Cancer, Pleural Mesothelioma, Sarcoma, Ovarian Epithelial Tumor, Soft Tissue Sarcoma, Bone Cancer, Colorectal Cancer, Bladder/Urinary Tract Cancer, T-Lymphoblastic Leukemia/Lymphoma.

In particular, said cancer is more preferably selected from the group consisting of Glioblastoma Multiforme, B-Lymphoblastic Leukemia/Lymphoma, Pancreatic Adenocarcinoma, Breast Invasive Ductal Carcinoma, Lung Squamous Cell Carcinoma, Bladder Urothelial Carcinoma, Lung Adenocarcinoma, Cutaneous Melanoma, Head and Neck Squamous Cell Carcinoma, Melanoma, Prostate Adenocarcinoma, Esophageal Adenocarcinoma, Astrocytoma, Serous Ovarian Cancer, Stomach Adenocarcinoma, Esophageal Squamous Cell Carcinoma, Hepatocellular Carcinoma, Glioblastoma, Pleural Mesothelioma, Epithelioid Type Diffuse Large B-Cell Lymphoma, Tubular Stomach Adenocarcinoma, Bladder/Urinary Tract Cancer, Colon Adenocarcinoma, Hepatocellular Adenoma, Malignant Peripheral Nerve Sheath Tumor, Papillary Renal Cell Carcinoma, Pleural Mesothelioma Biphasic Type, T-Lymphoblastic Leukemia/Lymphoma, Undifferentiated Pleomorphic Sarcoma/Malignant Fibrous Histiocytoma/High-Grade Spindle Cell Sarcoma.

It is to be understood that in connection with the medical uses of the invention it can be preferred that the compounds according to the present invention are administered in combination with antibodies, radiotherapy, surgical therapy, immunotherapy, chemotherapy, toxin therapy, gene therapy, or any other therapy known to those of ordinary skill in the art for treatment of a particular disease. This is particularly relevant in connection with the treatment of cancer. Preferably, the compounds of the present invention may be coadministered with an anti-neoplastic agent and/or an anti-neoplastic agent may be comprised in the pharmaceutical composition according to the present invention. The cancer treated by the combination of (i) a compound according to the present invention and (ii) an anti-neoplastic agent may be selected from one of the cancers listed above. An anti-neoplastic agent has activity versus a tumor and examples can be found in Cancer Principles and Practice of Oncology by V.T. Devita and S. Hellman (editors), 6th edition (February 15, 2001), Lippincott Williams & Wilkins Publishers. Typical anti-neoplastic agents useful in the present invention include chemotherapeutic agents, topoisomerase II inhibitors, antimetabolites, topoisomerase I inhibitors, hormones and hormonal analogues, signal transduction pathway inhibitors, non-receptor tyrosine kinase inhibitors, angiogenesis inhibitors, proapoptotic agents, cell cycle signaling inhibitors, proteasome inhibitors, inhibitors of cancer metabolism, and immunotherapeutic agents (such as STING pathway modulating compounds, TLR agonists and checkpoint inhibitors). Examples for chemotherapeutic agents are anti-microtubule or anti-mitotic agents (such as paclitaxel), platinum coordination complexes (such as cisplatin), alkylating agents (such as cyclophosphamide) and antibiotic anti-neoplastics (such as doxorubicin).

Combination therapy may be achieved by use of a single pharmaceutical composition that includes both agents, or by administering two distinct compositions at the same time, wherein one composition includes a compound of the present invention, and the other includes the second agent(s).

The two therapies may be given in either order and may precede or follow the other treatment by intervals ranging from minutes to weeks. In embodiments where the other agents are applied separately, one would generally ensure that a significant period of time did not expire between the time of each delivery, such that the agents would still be able to exert an advantageously combined effect on the patient. In such instances, it is contemplated that one may administer both modalities within about 12-24 h of each other and, more preferably, within about 6-12 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations. In some embodiments, the compound of the present invention is administered prior to administration of the distinct cancer treatment. In other embodiments, the distinct cancer treatment is administered prior to administration of the compound of the present invention.

The present invention is further illustrated by the following examples.

### Examples

The following abbreviations are used herein:

| Abbreviation | Meaning |
|---|---|
| ))) | with sonication |
| Ac | Acetyl |
| ACN/MeCN/ CH₃CN | Acetonitrile |
| AcOH | Acetic acid |
| AIBN | Azobisisobutyronitrile |
| alamarBlue | Resazurin dye (7-hydroxy-3*H*-phenoxazin-3-one 10-oxide) |
| Aq. | Aqueous |
| Atm. | Atmosphere |
| BINAP | (±)-2,2'-Bis(diphenylphosphino)-1,1'-binaphthalene |
| Bn | Benzyl |
| (Bpin)2 | Bis(pinacolato)diboron |
| BTFFH | Fluoro-dipyrrolidinocarbenium hexafluorophosphate |
| (BzO)₂, BPO | Benzoyl peroxide |
| Boc | *tert*-Butoxycarbonyl |
| BocNH2 | *tert*-Butyl carbamate |
| BSA | Bovine serum albumin |
| calc. | calculated |
| cataCXium^{®} C | trans-Di(µ-acetato)bis[o-(di-o-tolyl-phosphino)benzyl]dipalladium(II) |
| CDI | 1,1'-Carbonyldiimidazole |
| conc. | Concentrate |
| CRISPR/Cas9 | Clustered Regularly Interspaced Short Palindromic Repeats / CRISPR-associated endonuclease 9 |
| DAD | Diode array detector |
| DAST | Diethylaminosulphur trifluoride |
| dba | Dibenzylideneacetone |
| DBU | 1,8-Diazabicyclo[5.4.0]undec-7-ene |
| DCE | 1,2-Dichloroethane |
| DCM | Dichloromethane |
| DIAD | Diisopropyl azodicarboxylate |
| DIBAL-D | Diisobutylaluminum deuteride |
| DIPEA | *N*,*N*-Diisopropylethylamine |
| Dioxane | 1,4-Dioxane |
| DMC | Dimethylcarbonate |
| DMA | *N*,*N*-Dimethylacetamide |
| DMAP | 4-Dimethylaminopyridine |
| DMBA | 1-(2,4-dimethoxyphenyl)methanamine |
| DME | 1,2-Dimethoxyethane |
| DMF | *N*,*N*-Dimethylformamide |
| dppf | 1,1-Bis(diphenylphosphino)ferrocene |
| DMSO | Dimethyl sulfoxide |
| DTT | Dithiothreitol |
| EC₅₀ | Half maximal effective concentration |
| EDC.HCl | 1-(3-Dimethylaminopropyl)-3-ethylcarbodiimide hydrochloride |
| EDTA | Ethylenediaminetetraacetic acid |
| ELISA | Enzyme-linked immunosorbent assay |
| ELSD | Evaporative light scattering detector |
| eq. | Equivalent |
| EtOAc, AcOEt | Ethyl acetate |
| Ex | Example |
| FA | Formic acid |
| FBS | Fetal bovine serum |
| FCC | Flash column chromatography |
| h | hour |
| HATU | 1-[Bis(dimethylamino)methylene]-1*H*-1,2,3-triazolo[4,5-b]pyridinium 3-oxid hexafluorophosphate |
| HCT116 | Human colorectal carcinoma cell line 116 |
| HOBt | 1-Hydroxybenzotriazole |
| HOAt | 1 -Hydroxy-7-azabenzotriazole |
| IC₅₀ | Half maximal inhibitory concentration |
| IPA | isopropyl alcohol |
| Int. | Intermediate |
| iPrOAc | isopropyl acetate |
| KO | Knock out |
| KOAc/AcOK | potassium acetate |
| LC, HPLC | High-Performance Liquid Chromatography |
| LC MS, LCMS, LC-MS or LC/MS | High-Performance Liquid Chromatography coupled to Mass Spectrometry |
| LiHMDS | Lithium bis(trimethylsilyl)amide |
| L-Pro-ONa | Sodium (2*S*)-pyrrolidine-2-carboxylate |
| M | Molarity |
| mCPBA | *meta*-Chloroperoxybenzoic acid |
| MEP50 | Methylosome protein 50 |
| 2-MeTHF | 2-Methyltetrahydrofuran |
| Me₄tBuXPhos | 2-Di-*tert*-butylphosphino-3,4,5,6-tetramethyl-2',4',6'-triisopropyl-1,1'-biphenyl |
| Min. | Minute |
| mm | Millimeter |
| MTA | 5'-Methylthioadenosine |
| MTAP | 5'-Methylthioadenosine Phosphorylase |
| MsCI | Methanesulfonyl chloride |
| NBS | *N*-bromosuccinimide |
| NCS | *N*-chlorosuccinimide |
| NMP | 1-Methyl-2-pyrrolidone |
| NMR | Nuclear magnetic resonance |
| No., # | Number |
| µW or MW | Microwave |
| PBS | Phosphate Buffered Saline |
| Pd/C | Palladium on carbon |
| Pd2dba3 | Tris(dibenzylideneacetone)dipalladium(0) |
| Pd(dppf)Cl₂ | [1,1'-Bis(diphenylphosphino)ferrocene]dichloropalladium(II) |
| Pd(dppf)Cl₂.DC M | [1,1'-Bis(diphenylphosphino)ferrocene]dichloropalladium(II) dichloromethane complex |
| Pd(PPh₃)₄ | Tetrakis(triphenylphosphine)palladium(0) |
| PhCH₃ | Toluene |
| P(*n*-Bu)₃ | Tributylphosphane |
| PMB | *p*-Methoxybenzyl |
| PTSA | p-Toluenesulfonic acid |
| Prep HPLC | Preparative HPLC |
| PRMT5 | Protein methyltransferase 5 |
| Py | Pyridine |
| RM | Reaction mixture |
| Rochelle salt | Potassium sodium tartrate tetrahydrate |
| rpm | Revolutions per minute |
| RPMI 1640 | Roswell Park Memorial Institute medium 1640 |
| RT | Room temperature |
| Rt | Retention time |
| s | Second |
| SAM | *S*-Adenosyl methionine |
| sat. | Saturated |
| SCX | Strong cation exchange |
| SDMA | Symmetrical dimethylarginine |
| SelectFluor^{™} | *N*-Chloromethyl-*N*-fluorotriethylenediammonium bis(tetrafluoroborate) |
| SEM | Trimethylsilylethoxymethyl |
| SM | Starting material |
| STAB | Sodium triacetoxyborohydride |
| T3P | Propylphosphonic anhydride |
| TBAB | Tetrabutylammonium bromide |
| TEA | Triethylamine |
| temp. | temperature |
| TES | Triethylsilane |
| Tf | Trifluoromethanesulfonate |
| Tf₂O | trifluoromethanesulfonyl trifluoromethanesulfonate |
| TFA | Trifluoroacetic acid |
| TFAA | Trifluoroacetic anhydride |
| THF | Tetrahydrofuran |
| TLC | Thin layer chromatography |
| TRIS | Tris(hydroxymethyl)aminomethane |
| Trt | Trityl (Triphenylmethyl) |
| Tween 20 | Polyoxyethylene (20) sorbitan monolaurate |
| UPLC | Ultra Performance Liquid Chromatography |
| UPLC-MS | Ultra Performance Liquid Chromatography coupled to Mass Spectrometry |
| WT | Wild type |
| Xantphos | 4,5-bis(Diphenylphosphino)-9,9-dimethylxanthene |
| XPhos | 2-Dicyclohexylphosphino-2',4',6'-triisopropylbiphenyl |
| XPhos Pd G3 | (2-Dicyclohexylphosphino-2',4',6'-triisopropyl-1,1'-biphenyl)[2-(2'-amino-1,1'-biphenyl)]palladium(II) methanesulfonate |

The compounds of the present invention can be prepared according to the procedures of the following Schemes and Examples, using appropriate materials and are further exemplified by the following specific examples. While the present invention is described herein in conjunction with the specific examples, many alternatives, modifications and variations thereof will be apparent to those of ordinary skill in the art. In some cases, the order of carrying out the steps of the reaction schemes may be varied to facilitate the reaction or to avoid unwanted reaction products. All such alternatives, modifications and variations are intended to fall within the spirit and scope of the present invention.

### Methods and analytical data:

Several methods for preparing the compounds of this invention are described in the following Schemes and Examples. Unless otherwise indicated, all variables are as previously defined. All anhydrous solvents were provided by commercial suppliers, e.g., Sigma- Aldrich^{®}, in appropriate containers, e.g., Sure/Seal^{™} bottles, and were used without further purification. Unless otherwise specified, all starting materials are obtained from commercial suppliers and used without further purifications. Unless otherwise specified, all temperatures are expressed in °C and all reactions are conducted at rt. Unless otherwise specified, compounds were purified by either flash column chromatography (FCC), preparative HPLC or preparative chiral HPLC. Unless otherwise specified, silica (50 µm average particle size) is the stationary phase used for flash column chromatography purifications.

### NMR

¹H NMR was recorded on a Bruker Ascend 400 MHz spectrometer. Chemical shifts (δ) are reported in ppm relative to the residual solvent signal (δ = 2.50 ppm for ¹H NMR in DMSO-*d₆*, δ = 3.31 ppm for ¹H NMR in CD₃OD, δ = 7.26 ppm for ¹H NMR in CDCl₃). ¹H NMR data are reported as follows: chemical shift (multiplicity, coupling constants and number of hydrogens). Multiplicity is abbreviated as follows: s (singlet), d (doublet), t (triplet), q (quartet), m (multiplet), dd (doublet of doublets), ddd (doublet of doublets of doublets), tt (triplet of triplets), td (triplet of doublets), dt (doublet of triplets), hept (heptuplet), dq (doublet of quartets), br s (broad singlet). NMR data were analyzed using MestReNova v10.0.2 and ulterior (Mestrelab Research S.L., Santiago de Compostela, Spain, www.mestrelab.com).

### UPLC long elution

Four types of eluent system were used:

| Buffer type | Eluant A | Eluant B |
|---|---|---|
| "FA" | MeCN | 0.1% FA in water |
| "TFA" | MeCN | 0.1% TFA in water |
| "NH₃" | MeCN | 0.1% NH₄OH in water |
| "neutral" | MeCN | water |

| | | |
|---|---|---|
| Column: Waters ACQUITY UPLC^{®} BEH C18 1.7 µm, 2.1 x 100 mm Detection: DAD (Diode Array Detector), CAD (Charged Aerosol Detector) Equipment: H Class Waters UPLC-MS | | |

### Methods:

3 default methods were available for every eluent system/buffer type (FA, TFA, NH₃ and neutral), flow rate 0.5 mUmin:

### Polar long method:

| t (min.) | Eluant A (%) | Eluant B (%) |
|---|---|---|
| 0 | 1 | 99 |
| 7 | 100 | 0 |
| 8 | 100 | 0 |
| 9 | 1 | 99 |
| 12 | 1 | 99 |

### Mid polar long method:

| t (min.) | Eluant A (%) | Eluant B (%) |
|---|---|---|
| 0 | 10 | 90 |
| 7 | 100 | 0 |
| 8 | 100 | 0 |
| 9 | 10 | 90 |
| 12 | 10 | 90 |

### Non polar long method:

| t (min.) | Eluant A (%) | Eluant B (%) |
|---|---|---|
| 0 | 20 | 80 |
| 7 | 100 | 0 |
| 8 | 100 | 0 |
| 9 | 20 | 80 |
| 12 | 20 | 80 |

### UPLC

### Eluent composition:

| Buffer type | Eluant A | Eluant B |
|---|---|---|
| "FA" | MeCN | 0.1% FA in water |

| | | |
|---|---|---|
| Column: Waters ACQUITY UPLC^{®} BEH C18 1.7um, 2.1 x 100 mm. Detection: DAD, MS single quadrupole with positive and negative ionization, ESCI or ESI ion sources | | |

### Methods:

3 default methods were available for UPLC, flow rate 0.5 mUmin:

### Polar:

| t (s) | Eluant A (%) | Eluant B (%) |
|---|---|---|
| 0 | 1 | 99 |
| 6 | 1 | 99 |
| 66 | 100 | 0 |
| 120 | 100 | 0 |
| 150 | 1 | 99 |

### Mid polar:

| t (s) | Eluant A (%) | Eluant B (%) |
|---|---|---|
| 0 | 20 | 80 |
| 6 | 20 | 80 |
| 66 | 100 | 0 |
| 120 | 100 | 0 |
| 150 | 20 | 80 |

### Non polar:

| t (s) | Eluant A (%) | Eluant B (%) |
|---|---|---|
| 0 | 50 | 50 |
| 6 | 100 | 0 |
| 66 | 100 | 0 |
| 120 | 50 | 50 |
| 150 | 50 | 50 |

Equipment:
- I Class Waters UPLC-MS with SQD2 and ESCI ion source.
- I Class Waters UPLC-MS with SQD2 and ESI ion source.

### Preparative HPLC purifications

The following equipment was used for Preparative HPLC purifications: Waters Autopurification system (Waters 2767 - Sample Manager, Waters 2545 - Binary Gradient Module, Waters SFO - System Fluidics Organizer, Waters Prep Degasser, Waters 515 - HPLC Pump, Waters UV Fraction Manager) with DAD (Waters 2998 - Photodiode Array Detector) and QDa (Waters Acquity QDa) detection, using a Phenomenex Gemini^{®} 5µm NX-C18 110 Å (00G-4454-P0-AX LC Column 250 x 21.2 mm, AX) column.

Three types of eluent system were used:

| Buffer type | Eluant A | Eluant B |
|---|---|---|
| "FA" | MeCN | 0.1% FA in water |
| "TFA" | MeCN | 0.1% TFA in water |
| "NH₃" | MeCN | 0.1% NH₄OH in water |

General gradient: flow rate 20 mUmin.

| t (min.) | Eluant A (%) | Eluant B (%) |
|---|---|---|
| 0 | 10 | 90 |
| 1 | 10 | 90 |
| 10 | 40 or 60 | 60 or 40 |
| 12 | 95 | 5 |
| 14 | 95 | 5 |
| 15 | 10 | 90 |

Chiral HPLC purifications: The following equipment was used for Chiral HPLC purifications: HPLC Shimadzu hardware: 2x LC 20AP pumps, SPD M20A DAD detector, CBM -20A, auto sampler SIL 10AP, FRC-10A fraction collector. The chiral columns were of 4 types:
Daicel CHIRALPAK^{®} AY-H column (amylose tris(5-chloro-2-methylphenylcarbamate) coated on 5 µm silica-gel, 250 mm x 20 mm)
Daicel CHIRALPAK^{®} AD-H column (amylose tris-(3,5-dimethylphenylcarbamate) coated on 5 µm silica-gel, 250 mm x 20 mm)
IF column Dr. Maisch (Amylose tris-(3-Chloro-4-Methylphenyl) Carbamate), (Particle Size: 5µm, 20mm x L 250mm)
Daicel CHIRALPAK IE HPLC Semi-Preparative Column, (Amylose tris(3,5-dichlorophenylcarbamate)), (particle size: 5 µm, 20 mm x L 250 mm)

### Method A

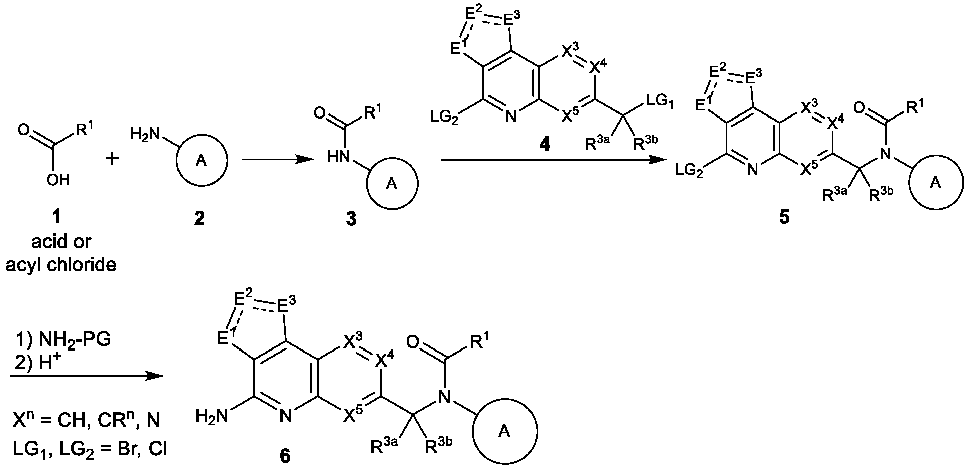

Compounds of Formula (I) may be prepared according to Method A. Compound **3** might be product of acid **1** or acid chloride of the acid **1** with amine **2** coupling reaction. Compound **3** when treated with **4** and a base, such as Cs₂CO₃, provide **5.** Compound **5** is subject of reaction with ammonia equivalent, for example DMBA or *tert*-butyl carbamate, followed by deprotection in acidic conditions to provide desired product **6** of Formula (I)

### Examples

### Intermediate A: 4-chloro-7-(chloromethyl)-1H,3H-furo[3,4-c]quinoline

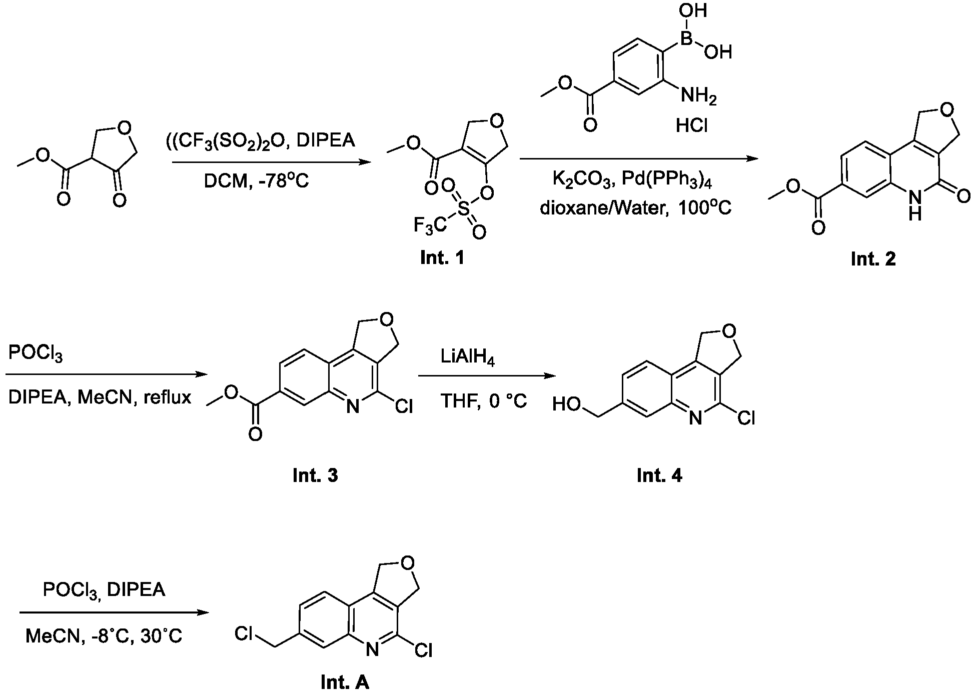

### Methyl 4-(trifluoromethanesulfonyloxy)-2,5-dihydrofuran-3-carboxylate (Int. 1):

To a stirred solution of methyl 4-oxooxolane-3-carboxylate (9.0 g, 62.445 mmol, 1.0 eq.) in DCM (300 mL), DIPEA (13.05 mL, 74.934 mmol, 1.2 eq.) was added and the RM was cooled to -78°C. Then triflic anhydride (21.14 g, 74.934 mmol, 1.2 eq.) was added dropwise. The reaction mixture was stirred at -78°C for 1 h. RM was diluted with water and aqueous layer was extracted with DCM (3x). Combined organic layers were washed with aq. NaHCOs and brine, dried over Na₂SO₄ and evaporated. The residue was purified by FCC (0 to 100% DCM gradient in heptane) to obtain methyl 4-(trifluoromethanesulfonyloxy)-2,5-dihydrofuran-3-carboxylate (**Int**. **1**, 15.9 g, 51.814 mmol, 83%, yellow oil).

¹H NMR (400 MHz, Chloroform-d) δ 4.98 - 4.89 (m, 2H), 4.85 - 4.76 (m, 2H), 3.85 (s, 3H).

### Methyl 4-oxo-1H,3H,4H,5H-furo[3,4-c]quinoline-7-carboxylate (Int. 2):

A mixture of methyl 4-(trifluoromethanesulfonyloxy)-2,5-dihydrofuran-3-carboxylate (**Int. 1**, 15.9 g, 51.806 mmol, 1.1 eq.), 2-amino-4-(methoxycarbonyl)phenylboronic acid hydrochloride (10.9 g, 47.096 mmol, 1.0 eq.), K₂CO₃ (15.287 g, 110.612 mmol, 4.0 eq.), water (12.5 mL) and 1,4-dioxane (125 mL) was sparged with argon, then Pd(PPh₃)₄ (1.088 g, 0.942 mmol, 0.02 eq.) was added and the mixture heated at 100°C for 2.5 h. The RM was cooled to RT, diluted with water and extracted with CHCl₃/*i*-PrOH (3:1) mixture (2x). Combined organic phases were washed with brine, dried over Na₂SO₄, filtered and evaporated. Crude product was triturated with Et₂O and a few drops of *i*-PrOH and acetone, then filtered and washed with Et₂O to obtain methyl 4-oxo-1H,3H,4H,5H-furo[3,4-c]quinoline-7-carboxylate (**Int. 2**, 9.26 g, 36.627 mmol, 78%, light brown solid, m/z [M+H]⁺: 246.1).

¹H NMR (400 MHz, DMSO- *d*₆) δ 12.06 (s, 1H), 8.03 (d, J = 1.6 Hz, 1H), 7.74 (dd, J = 8.2, 1.6 Hz, 1H), 7.60 (d, J = 8.2 Hz, 1H), 5.36 - 5.31 (m, 2H), 4.99 (t, J = 4.1 Hz, 2H), 3.90 (s, 3H).

### Methyl 4-chloro-1H,3H-furo[3,4-c]quinoline-7-carboxylate (Int. 3)

A 2500 mL flask was charged with acetonitrile (450 mL) and DIPEA (34 mL, 196.054 mmol, 1.0 eq.) under flow of N₂. The mixture was cooled down in an ice bath and POCl₃ (45.1 g, 294.082 mmol, 1.5 eq.) was added portionwise. It was followed by addition of solid methyl 4-oxo-1H,3H,4H,5H-furo[3,4-c]quinoline-7-carboxylate (**Int. 2**, 50.25 g, 196.054 mmol, 1.0 eq.). The addition funnel was washed with ACN (50 mL) and the cooling bath was replaced with a heating block. RM was heated to reflux and stirred for 3 h. RM was cooled to a °C and then quenched with 2 M NaOH (400 mL), while ensuring that the internal temperature remained below 10°C. Then, water was added (500 mL) and the mixture was stirred for 30 min at 5-10°C. Then, the solid was filtered off, washed with water (250 mL), ACN (100 mL), water (3x250 mL) and heptane (500 mL) and air-dried for 3 h. The solid was transferred to a crystallizer and dried in oven (0 mbar, 60°C) for 24 h to give methyl 4-chloro-1H,3H-furo[3,4-c]quinoline-7-carboxylate (**Int. 3**, 49.477 g, 185.764 mmol, 95%, light gray solid).

¹H NMR (400 MHz, CDCl₃) δ 8.80 (s, 1H), 8.19 (dd, J = 8.6, 1.7 Hz, 1H), 7.67 (d, J = 8.6 Hz, 1H), 5.57 (t, J = 3.2 Hz, 2H), 5.33 (t, J = 3.2 Hz, 2H), 4.01 (s, 3H).

### {4-Chloro-1H,3H-furo[3,4-c]quinolin-7-yl}methanol (Int. 4)

The 1.5 L flask equipped with mechanical stirrer, condenser, nitrogen inlet, thermocouple and rubber septum was charged with anhydrous THF (380 mL) followed by methyl 4-chloro-1H,3H-furo[3,4-c]quinoline-7-carboxylate (**Int. 3**, 35.477 g, 133.2 mmol, 1.0 eq.). Then mixture was cooled in ice/salt/water bath to -8°C and 2M LAH solution (53.28 mL, 106.565 mmol, 0.8 eq.) was slowly added via syringe below 0°C. When addition of LAH was completed reaction was stirred for 30 minutes in temperature below 0°C. Reaction mixture was cooled to -5°C and acetone (48.9 mL, 666.0 mmol, 5.0 eq.) was added via syringe keeping temperature below 0°C. After addition mixture was stirred for 15 minutes in 0°C, then mixture was quenched with 2M HCl (700 mL). Mixture was stirred for 10 minutes, then THF was removed on rotavap (-380 mL) and obtained solid was filtered off, washed with water, heptane and dried on filter overnight. First filtrate was extracted with DCM, combined organic layers were dried over MgSO₄, then drying agent was removed and second portion of the product was isolated via evaporation {4-chloro-1H,3H-furo[3,4-c]quinolin-7-yl}methanol (**Int**. **4,** 29.629 g, 123.686 mmol, 93%, light gray solid).

¹H NMR (400 MHz, DMSO- *d*₆) δ 7.94 (s, 1H), 7.81 (d, J = 8.4 Hz, 1H), 7.64 (dd, J = 8.4, 1.6 Hz, 1H), 5.56 - 5.48 (m, 3H), 5.21 (t, J = 3.1 Hz, 2H), 4.73 (d, J = 5.8 Hz, 2H).

### Intermediate A: 4-chloro-7-(chloromethyl)-1H,3H-furo[3,4-c]quinoline

The 100 mL flask equipped with condenser, magnetic stirrer, rubber septum and thermometer was charged with acetonitrile (20.0 mL) under flow of nitrogen. Then mixture was cooled in ice/salt/water bath to -8°C and DIPEA (1.486 mL, 8.532 mmol, 1.2 eq.) was added followed by slow addition of POCl₃ (1.308 g, 8.532 mmol, 1.2 eq.). The mixture was stirred for 10 minutes in -5°C. Then {4-chloro-1H,3H-furo[3,4-c]quinolin-7-yl}methanol (**Int**. **4,** 1.742 g, 7.11 mmol, 1.0 eq.) was added and glassware were washed with MeCN (5 mL). The mixture was stirred at 30°C for 60 minutes. RM was cooled to 0°C in ice bath and 25 mL of DCM was added to the reaction mixture followed by slow addition of 2M NaOH (25 mL). Mixture was stirred at RT for 30 minutes and transferred to separation funnel (~30mL of DCM and 30mL of water added), layers were separated and aqueous layer was washed with DCM (3 x 30 mL). Combined organic layers were washed with brine (30 mL) dried over Na₂SO₄, then drying agent was removed via filtration, solution of product was evaporated together with 72 mL of heptane. After evaporation of -170 mL of DCM precipitate occurs. Solid was filtered off, washed with small amount of heptane to obtain 4-chloro-7-(chloromethyl)-1H,3H-furo[3,4-c]quinoline (**Int**. **A,** 1.704 g, 6.37 mmol, 90%, beige solid, m/z [M+H]⁺: 255.1).

¹H NMR (400 MHz, CDCl₃) δ 8.08 (s, 1H), 7.64 (s, 2H), 5.55 (t, J = 3.2 Hz, 2H), 5.31 (t, J = 3.1 Hz, 2H), 4.77 (s, 2H).

### Example 1: N-({4-Amino-1H,3H-furo[3,4-c]quinolin-7-yl}methyl)-N-[1-methyl-3-(trifluoromethyl)-1H-pyrazol-4-yl]-6-(piperazin-1-yl)pyridine-3-carboxamide

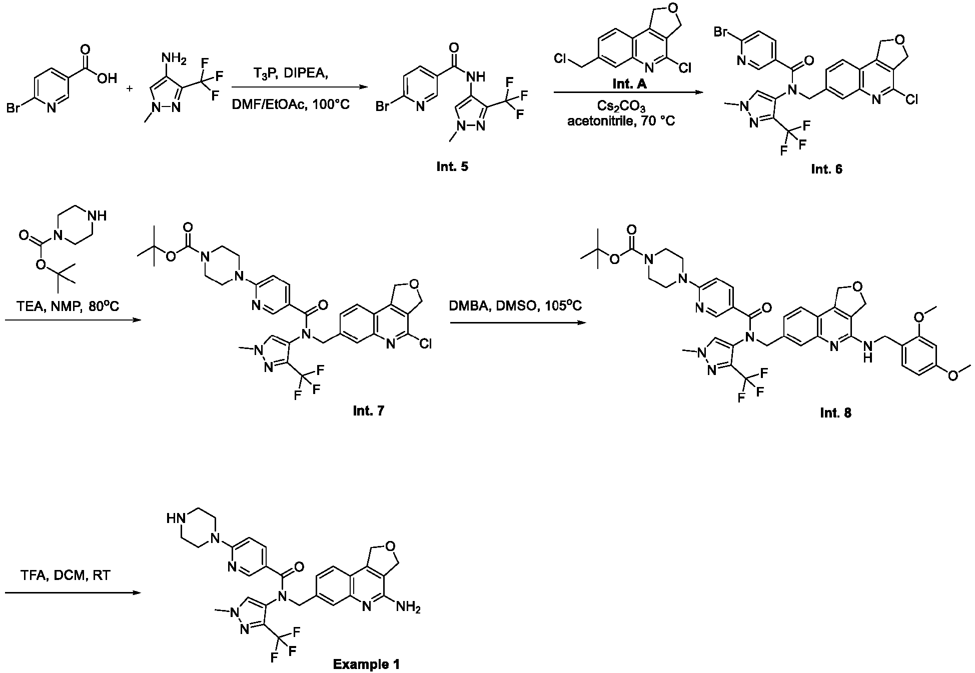

### 6-Bromo-N-[1-methyl-3-(trifluoromethyl)-1H-pyrazol-4-yl]pyridine-3-carboxamide (Int. 5)

1-Methyl-3-(trifluoromethyl)-1H-pyrazol-4-amine (0.45 g, 2.725 mmol, 0.611 eq.), 6-bromopyridine-3-carboxylic acid (0.901 g, 4.46 mmol, 1.0 eq.) and DMAP (0.25 g, 2.046 mmol, 0.459 eq.) were taken in DMF (10 ml), followed by an addition of DIPEA (1.56 mL, 8.956 mmol, 2.008 eq.). T3P as 50% solution in EtOAc (3.35 mL, 11.255 mmol, 2.523 eq.) was added slowly and the reaction mixture was stirred at 100°C for 1 h. Then the reaction mixture was diluted with EtOAc and neutralized with a saturated solution of NaHCOs. The organic phase was washed with a saturated solution of NaHCOs, brine, dried over Na₂SO₄, filtered and evaporated to obtain 6-bromo-N-[1-methyl-3-(trifluoromethyl)-1H-pyrazol-4-yl]pyridine-3-carboxamide (**Int. 5**, 1.5 g, 3.222 mmol, 72%, white solid, m/z [M+H]⁺: 351.0). This product was used in the next step without further purification.

¹H NMR (400 MHz, DMSO- *d*₆) δ 10.23 (s, 1H), 8.85 (dd, J = 2.5, 0.8 Hz, 1H), 8.18 (dd, J = 8.3, 2.6 Hz, 1H), 8.15 (d, J = 1.0 Hz, 1H), 7.85 (dd, J = 8.3, 0.7 Hz, 1H), 3.94 (s, 3H).

### 6-Bromo-N-({4-chloro-1H,3H-furo[3,4-c]quinolin-7-yl}methyl)-N-[1-methyl-3-(trifluoromethyl)-1H-pyrazol-4-yl]pyridine-3-carboxamide (Int. 6)

To 6-bromo-N-[1-methyl-3-(trifluoromethyl)-1H-pyrazol-4-yl]pyridine-3-carboxamide (**Int. 5**, 1.5 g, 3.222 mmol, 1.0 eq.) in MeCN (20.0 mL), Cs₂CO₃ (1.524 g, 4.677 mmol, 1.452 eq.) was added. The mixture was stirred at RT for 15 min. Then, 4-chloro-7-(chloromethyl)-1H,3H-furo[3,4-c]quinoline (**Int**. **A,** 0.924 g, 3.6 mmol, 1.117 eq.) was added and the reaction mixture was stirred at 70°C for 5 h. The reaction mixture was cooled down, diluted with EtOAc and water and shaken vigorously. The organic phase was separated, washed with brine, dried over Na₂SO₄, filtered, concentrated. The residue was purified by FCC (0% to 100% EtOAc gradient in Hexane) to obtain 6-bromo-N-({4-chloro-1H,3H-furo[3,4-c]quinolin-7-yl}methyl)-N-[1-methyl-3-(trifluoromethyl)-1H-pyrazol-4-yl]pyridine-3-carboxamide (**Int. 6**, 1.436 g, 2.483 mmol, 77%, beige solid, m/z [M+H]⁺: 568.0).

¹H NMR (400 MHz, DMSO- *d*₆) δ 8.38 (d, *J* = 2.5 Hz, 1H), 8.10 (s, 1H), 7.94 (s, 1H), 7.86 (d, *J* = 8.4 Hz, 1H), 7.78 - 7.61 (m, 3H), 5.55 (t, *J* = 3.1 Hz, 2H), 5.37 - 5.17 (m, 3H), 5.04 - 4.89 (m, 1H), 3.81 (s, 3H).

### tert-Butyl 4-{5-[({4-chloro-1H,3H-furo[3,4-c]quinolin-7-yl}methyl)[1-methyl-3-(trifluoromethyl)-1H-pyrazol-4-yl]carbamoyl]pyridin-2-yl}piperazine-1-carboxylate (Int. 7)

A solution of 6-bromo-N-({4-chloro-1H,3H-furo[3,4-c]quinolin-7-yl}methyl)-N-[1-methyl-3-(trifluoromethyl)-1H-pyrazol-4-yl]pyridine-3-carboxamide (**Int. 6**, 0.3 g, 0.519 mmol, 1.0 eq.), tert-butyl piperazine-1-carboxylate (0.106 g, 0.569 mmol, 1.097 eq.) and triethylamine (0.22 mL, 1.578 mmol, 3.042 eq.) in dry NMP (7.0 mL) was stirred at 80°C for 65 hours. Then, the reaction mixture was cooled down, diluted with EtOAc and then the organic solution was washed four times with brine, dried over Na₂SO₄, filtered and concentrated. The residue was purified by FCC (0% to 5% MeOH gradient in DCM) to obtain tert-butyl 4-{5-[({4-chloro-1H,3H-furo[3,4-c]quinolin-7-yl}methyl)[1-methyl-3-(trifluoromethyl)-1H-pyrazol-4-yl]carbamoyl]pyridin-2-yl}piperazine-1-carboxylate (**Int. 7**, 0.238 g, 0.333 mmol, 64%, yellow solid, m/z [M+H]⁺: 673.1).

¹H NMR (400 MHz, DMSO- *d*₆) δ 8.15 (d, J = 2.4 Hz, 1H), 8.05 (s, 1H), 7.90 (d, J = 1.6 Hz, 1H), 7.85 (d, J = 8.4 Hz, 1H), 7.67 (dd, J = 8.5, 1.7 Hz, 1H), 7.55 (dd, J = 9.0, 2.5 Hz, 1H), 6.73 (d, J = 9.0 Hz, 1H), 5.55 (t, J = 3.1 Hz, 2H), 5.37 - 5.11 (m, 3H), 5.03 - 4.75 (m, 1H), 3.81 (s, 3H), 3.57 - 3.50 (m, 4H), 3.43 - 3.36 (m, 4H), 1.42 (s, 9H).

### tert-Butyl 4-{5-[({4-chloro-1H,3H-furo[3,4-c]quinolin-7-yl}methyl)[1-methyl-3-(trifluoromethyl)-1H-pyrazol-4-yl]carbamoyl]pyridin-2-yl}piperazine-1-carboxylate (Int. 8)

A solution of tert-butyl 4-{5-[({4-chloro-1H,3H-furo[3,4-c]quinolin-7-yl}methyl)[1-methyl-3-(trifluoromethyl)-1H-pyrazol-4-yl]carbamoyl]pyridin-2-yl}piperazine-1-carboxylate (**Int. 7**, 0.234 g, 0.327 mmol, 1.0 eq.) and 1-(2,4-dimethoxyphenyl)methanamine (0.328 g, 1.962 mmol, 5.994 eq.) in DMSO (4.0 mL) was stirred at 100 °C for 40 h. Then, the reaction mixture was cooled down, diluted with EtOAc and then the organic solution was washed four times with brine, dried over Na₂SO₄, filtered and concentrated. The residue was purified by FCC (0% to 10% MeOH gradient in DCM) to obtain tert-butyl 4-(5-{[(4-{[(2,4-dimethoxyphenyl)methyl]amino}-1H,3H-furo[3,4-c]quinolin-7-yl)methyl][1-methyl-3-(trifluoromethyl)-1H-pyrazol-4-yl]carbamoyl}pyridin-2-yl)piperazine-1-carboxylate (**Int. 8**, 0.133 g, 0.157 mmol, 48%, brown solid, m/z [M+H]⁺: 803.8).

¹H NMR (400 MHz, DMSO- *d*₆) δ 8.14 - 8.09 (m, 1H), 7.88 (s, 1H), 7.56 - 7.50 (m, 1H), 7.48 - 7.40 (m, 2H), 7.17 (d, J = 8.4 Hz, 1H), 7.13 (dd, J = 8.2, 1.7 Hz, 1H), 7.01 (t, J = 5.7 Hz, 1H), 6.72 (d, J = 9.1 Hz, 1H), 6.56 (d, J = 2.4 Hz, 1H), 6.43 (dd, J = 8.4, 2.4 Hz, 1H), 5.39 - 5.19 (m, 3H), 5.08 - 5.03 (m, 2H), 4.74 - 4.45 (m, 3H), 3.82 (s, 3H), 3.78 (s, 3H), 3.73 (s, 3H), 3.56 - 3.49 (m, 4H), 3.41 - 3.35 (m, 4H), 1.41 (s, 9H).

### N-({4-Amino-1H,3H-furo[3,4-c]quinolin-7-yl}methyl)-N-[1-methyl-3-(trifluoromethyl)-1H-pyrazol-4-yl]-6-(piperazin-1-yl)pyridine-3-carboxamide (Example 1)

To a solution of tert-butyl 4-(5-{[(4-{[(2,4-dimethoxyphenyl)methyl]amino}-1H,3H-furo[3,4-c]quinolin-7-yl)methyl][1-methyl-3-(trifluoromethyl)-1H-pyrazol-4-yl]carbamoyl}pyridin-2-yl)piperazine-1-carboxylate (**Int. 8**, 0.13 g, 0.154 mmol, 1.0 eq.) in DCM (8.0 mL), TFA (2.0 mL, 26.118 mmol, 169.789 eq.) was added and the reaction mixture was stirred at RT overnight. Then, the reaction mixture was diluted with DCM (50 mL) and carefully basified with a saturated solution of NaHCOs. After 30 minutes of vigorous stirring the organic layer was separated, washed carefully with a saturated solution of NaHCOs, dried over anhydrous Na₂SO₄, filtered and concentrate. The residue was purified by FCC (0% to 5% MeOH gradient in DCM) to obtain after lyophilization N-({4-amino-1H,3H-furo[3,4-c]quinolin-7-yl}methyl)-N-[1-methyl-3-(trifluoromethyl)-1H-pyrazol-4-yl]-6-(piperazin-1-yl)pyridine-3-carboxamide (**Example 1**, 0.036 g, 0.065 mmol, 42%, white solid, UPLC long elution (Polar long method, buffer type "FA") purity: 99.2%).

¹H NMR (400 MHz, DMSO- *d*₆) δ 8.11 (d, J = 2.4 Hz, 1H), 7.88 (s, 1H), 7.52 - 7.43 (m, 2H), 7.40 (d, J = 1.7 Hz, 1H), 7.14 (dd, J = 8.2, 1.7 Hz, 1H), 6.68 (d, J = 9.1 Hz, 1H), 6.48 (s, 2H), 5.42 - 5.16 (m, 3H), 5.00 (t, J = 3.4 Hz, 2H), 4.72 - 4.51 (m, 1H), 3.79 (s, 3H), 3.47 - 3.41 (m, 4H), 2.79 - 2.68 (m, 4H).

¹⁹F NMR (377 MHz, DMSO- *d*₆) δ -60.20.

### Example 2: N-{4-Amino-1H,3H-furo[3,4-c]quinolin-7-yl}methyl)-6-{4,7-diazaspiro[2.5]octan-7-yl}-N-[1-methyl-3-(trifluoromethyl)-1H-pyrazol-4-yl]pyridine-3-carboxamide

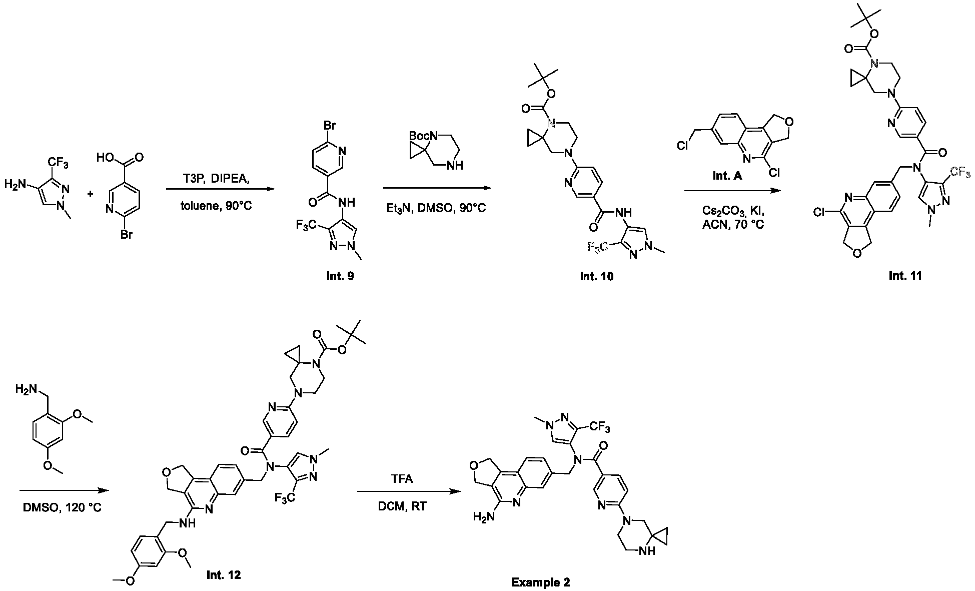

### 6-Bromo-N-[1-methyl-3-(trifluoromethyl)-1H-pyrazol-4-yl]pyridine-3-carboxamide (Int. 9)

DMAP (0.019 g, 0.151 mmol, 0.05 eq.), DIPEA (1.36 mL, 7.5705 mmol, 2.5 eq.), 1-methyl-3-(trifluoromethyl)-1H-pyrazol-4-amine (0.5 g, 3.028 mmol, 1.0 eq.) 6-bromopyridine-3-carboxylic acid (0.612 g, 3.028 mmol, 1.09 eq.) were taken in toluene (10 mL) and T3P as 50% in EtOAc (3.61mL, 6.06 mmol, 2 eq.) was added dropwise at 0°C. Then, the reaction mixture was stirred at 90°C for 2 h. The reaction mixture was diluted with a saturated solution of Na₂CO₃, extracted with EtOAc (2x) and the combined organic layers were washed with brine and dried over MgSO₄, filtered and evaporated. The crude product was purified by FCC (0% to 5% MeOH gradient in DCM) to obtain 6-bromo-N-[1-methyl-3-(trifluoromethyl)-1H-pyrazol-4-yl]pyridine-3-carboxamide (**Int. 9**, 987 mg, 2.83 mmol, 88%, white powder, m/z [M+H]⁺: 349.20).

¹H NMR (400 MHz, DMSO- *d*₆) δ 10.22 (s, 1H), 8.85 (dd, J = 2.5, 0.8 Hz, 1H), 8.18 (dd, J = 8.3, 2.5 Hz, 1H), 8.15 (s, 1H), 7.85 (dd, J = 8.3, 0.7 Hz, 1H), 3.94 (s, 3H).

¹⁹F NMR (377 MHz, DMSO- *d*₆) δ -59.51.

### tert-Butyl 7-(5-{[1-methyl-3-(trifluoromethyl)-1H-pyrazol-4-yl]carbamoyl}pyridin-2-yl)-4,7-diazaspiro[2.5]octane-4-carboxylate (Int. 10)

To a solution of 6-bromo-N-[1-methyl-3-(trifluoromethyl)-1H-pyrazol-4-yl]pyridine-3-carboxamide (**Int. 9**, 0.366 g, 1.01 mmol, 1.0 eq.) in DMSO (5 mL), tert-butyl 4,7-diazaspiro[2.5]octane-4-carboxylate (0.256 g, 1.21 mmol, 1.2 eq.) and TEA ( 0.42 mL, 3.02 mmol, 3 eq.) were added. The reaction mixture was stirred at 90°C for 24 h. The reaction mixture was diluted with EtOAc and poured into a saturated solution of NaHCOs. The organic layers were separated and the water layer was extracted with EtOAc. The combined organic layers were washed with brine and dried over MgSO₄, filtered and evaporated. The crude product was purified by FCC (0% to 5% MeOH gradient in DCM) to obtain tert-butyl 7-(5-{[1-methyl-3-(trifluoromethyl)-1H-pyrazol-4-yl]carbamoyl}pyridin-2-yl)-4,7-diazaspiro[2.5]octane-4-carboxylate (**Int. 10**, 330 mg, 0.687 mmol, 68%, m/z [M+H]⁺: 482.10).

¹H NMR (400 MHz, DMSO- *d*₆) δ 9.66 (s, 1H), 8.75 - 8.56 (m, 1H), 8.07 (d, J = 1.1 Hz, 1H), 8.00 (dd, J = 9.1, 2.5 Hz, 1H), 6.96 - 6.78 (m, 1H), 3.92 (s, 3H), 3.70 - 3.58 (m, 2H), 3.56 - 3.44 (m, 4H), 1.00 - 0.69 (m, 4H).

¹⁹F NMR (377 MHz, DMSO- *d*₆) δ -59.44.

### tert-Butyl 7-{5-[({4-chloro-1H,3H-furo[3,4-c]quinolin-7-yl}methyl)[1-methyl-3-(trifluoromethyl)-1H-pyrazol-4-yl]carbamoyl]pyridin-2-yl}-4,7-diazaspiro[2.5]octane-4-carboxylate (Int. 11)

The mixture of tert-butyl 7-(5-{[1-methyl-3-(trifluoromethyl)-1H-pyrazol-4-yl]carbamoyl}pyridin-2-yl)-4,7-diazaspiro[2.5]octane-4-carboxylate (**Int**. **10,** 0.330 g, 0.687 mmol), 4-chloro-7-(chloromethyl)-1H,3H-furo[3,4-c]quinoline (**Int**. **A,** 0.175 g, 0.68 mmol), KI (0.113 g, 0.68 mmol) and Cs₂CO₃ (0.266 g, 0.82 mmol) in MeCN (3.4 mL) was stirred at 70 °C for 4 h. The reaction mixture was diluted with EtOAc and poured into water. The organic layers were separated and the water layer was extracted with EtOAc several times. The combined organic layers were washed with brine and dried over anhydrous MgSO₄, filtered and evaporated. The crude product was purified by FCC (0% to 80% EtOAc gradient in Hexane) to obtain tert-butyl 7-{5-[({4-chloro-1H,3H-furo[3,4-c]quinolin-7-yl}methyl)[1-methyl-3-(trifluoromethyl)-1H-pyrazol-4-yl]carbamoyl]pyridin-2-yl}-4,7-diazaspiro[2.5]octane-4-carboxylate (**Int**. **11,** 292 mg, 0.418 mmol, 60%, m/z [M+H]⁺: 698.90).

¹H NMR (400 MHz, DMSO- *d*₆) δ 8.12 (d, J = 2.5 Hz, 1H), 8.03 (s, 1H), 7.88 (d, J = 1.6 Hz, 1H), 7.84 (d, J = 8.4 Hz, 1H), 7.65 (dd, J = 8.5, 1.7 Hz, 1H), 7.51 (dd, J = 9.0, 2.5 Hz, 1H), 6.69 (d, J = 9.1 Hz, 1H), 5.53 (t, J = 3.1 Hz, 2H), 5.41 - 5.14 (m, 3H), 5.02 - 4.71 (m, 1H), 3.80 (s, 3H), 3.57 - 3.43 (m, 4H), 3.39 (s, 2H), 1.40 (s, 9H), 0.99 - 0.68 (m, 4H).

¹⁹F NMR (377 MHz, DMSO- *d*₆) δ -60.20.

### tert-Butyl 7-(5-{[(4-{[(2,4-dimethoxyphenyl)methyl]amino}-1H,3H-furo[3,4-c]quinolin-7-yl)methyl][1-methyl-3-(trifluoromethyl)-1H-pyrazol-4-yl]carbamoyl}pyridin-2-yl)-4,7-diazaspiro[2.5]octane-4-carboxylate (Int. 12)

Tert-butyl 7-{5-[({4-chloro-1H,3H-furo[3,4-c]quinolin-7-yl}methyl)[1-methyl-3-(trifluoromethyl)-1H-pyrazol-4-yl]carbamoyl]pyridin-2-yl}-4,7-diazaspiro[2.5]octane-4-carboxylate (11, 0.288 g, 0.40 mmol, 1.0 eq.) and 1-(2,4-dimethoxyphenyl)methanamine (0.30 mL, 2.00 mmol, 5 eq.) were stirred in DMSO (4 mL) at 120°C for 10 h. The reaction mixture was diluted with EtOAc, poured into a saturated solution of NaHCOs and the organic layers were separated and the water layer was extracted with EtOAc twice. The combined organic layers were washed with brine and dried over anhydrous MgSO₄, filtered and evaporated. The crude product was purified by FCC (0 to 25% MeCN gradient in DCM) to obtain tert-butyl 7-(5-{[(4-{[(2,4-dimethoxyphenyl)methyl]amino}-1H,3H-furo[3,4-c]quinolin-7-yl)methyl][1-methyl-3-(trifluoromethyl)-1H-pyrazol-4-yl]carbamoyl}pyridin-2-yl)-4,7-diazaspiro[2.5]octane-4-carboxylate (**Int**. **12,** 366 mg, 0.441 mmol, 100%, m/z [M+H]⁺: 828.00).

¹H NMR (400 MHz, DMSO- *d*₆) δ 8.10 - 8.06 (m, 1H), 7.86 (s, 1H), 7.51 - 7.46 (m, 1H), 7.46 - 7.41 (m, 2H), 7.16 (d, J = 8.4 Hz, 1H), 7.12 (dd, J = 8.2, 1.7 Hz, 1H), 7.00 (t, J = 5.7 Hz, 1H), 6.73 - 6.64 (m, 1H), 6.55 (d, J = 2.4 Hz, 1H), 6.42 (dd, J = 8.4, 2.4 Hz, 1H), 5.45 - 5.15 (m, 3H), 5.04 (t, J = 3.5 Hz, 2H), 4.76 - 4.40 (m, 3H), 3.81 (s, 3H), 3.77 (s, 3H), 3.72 (s, 3H), 3.56 - 3.43 (m, 4H), 3.38 (s, 2H), 1.40 (s, 9H), 0.96 - 0.67 (m, 4H).

¹⁹F NMR (377 MHz, DMSO- *d*₆) δ -60.18.

### N-{4-amino-1H,3H-furo[3,4-c]quinolin-7-yl}methyl)-6-{4,7-diazaspiro[2.5]octan-7-yl}-N-[1-methyl-3-(trifluoromethyl)-1H-pyrazol-4-yl]pyridine-3-carboxamide (Example 2)

To a solution of tert-butyl 7-(5-{[(4-{[(2,4-dimethoxyphenyl)methyl]amino}-1H,3H-furo[3,4-c]quinolin-7-yl)methyl][1-methyl-3-(trifluoromethyl)-1H-pyrazol-4-yl]carbamoyl}pyridin-2-yl)-4,7-diazaspiro[2.5]octane-4-carboxylate (**Int. 12**, 0.125 g, 0.122 mmol, 1.0 eq.) in DCM (1.4 mL), TFA (0.47 mL, 6.11 mmol, 50 eq.) was added dropwise and the reaction mixture was stirred at RT for 6 h. Then, the reaction mixture was diluted with DCM and poured into ice water. A solution of NaOH 1M was added until to get a basic pH and the layers were separated. The water layer was extracted with DCM (2x). The organic layers were combined, washed with a saturated solution of Na₂CO₃, brine and dried over MgSO₄, filtered and evaporated. The crude product was purified by preparative HPLC (basic conditions) and lyophilized to obtain (**Example 2**, 0.014 g, 0.024 mmol, 20%, white solid, UPLC long elution (polar long method, buffer type "FA") purity 99.82%, m/z [M+H]⁺: 579.80).

¹H NMR (400 MHz, DMSO-d₆) δ 8.07 (d, J = 2.4 Hz, 1H), 7.87 (d, J = 1.1 Hz, 1H), 7.50 - 7.41 (m, 2H), 7.39 (d, J = 1.6 Hz, 1H), 7.12 (dd, J = 8.2, 1.7 Hz, 1H), 6.65 (d, J = 9.1 Hz, 1H), 6.47 (s, 2H), 5.49 - 5.08 (m, 3H), 4.99 (t, J = 3.4 Hz, 2H), 4.79 - 4.39 (m, 1H), 3.78 (s, 3H), 3.51 - 3.41 (m, 2H), 3.34 (s, 2H), 2.85 - 2.70 (m, 2H), 0.57 - 0.29 (m, 4H).

¹⁹F NMR (377 MHz, DMSO- *d*₆) δ -60.20.

### Example 3: N-({4-Amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide

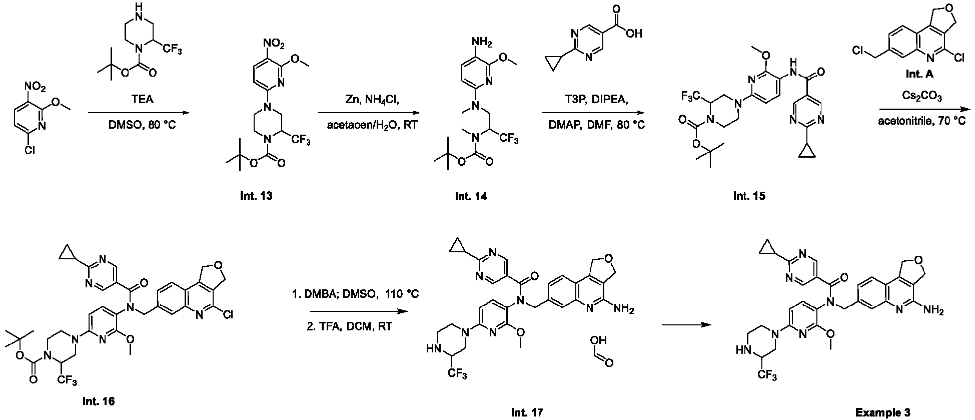

tert-Butyl 4-(6-methoxy-5-nitropyridin-2-yl)-2-(trifluoromethyl)piperazine-1-carboxylate (**Int. 13**)

To a solution of 6-chloro-2-methoxy-3-nitropyridine (0.1 g, 0.53 mmol, 1.0 eq.) and tert-butyl 2-(trifluoromethyl)piperazine-1-carboxylate (0.12 g, 0.47 mmol, 0.89 eq.) in DMSO (6 mL), TEA (0.11 mL, 0.789 mmol, 1.49 eq.) was added dropwise. The reaction mixture was stirred at RT for 15 min and then at 80°C for 2 h. Then, the reaction mixture was diluted with ethyl acetate and a saturated solution of NaHCOs. The combined organic layers were washed with brine, then dried over anhydrous Na₂SO₄, filtered, and evaporated to obtain tert-butyl 4-(6-methoxy-5-nitro-pyridin-2-yl)-2-(trifluoromethyl)piperazine-1-carboxylate (**Int**. **13,** 0.23 g, 0.56 mmol, 100%).

¹H NMR (400 MHz, DMSO-d₆) δ 8.26 (d, J = 9.2 Hz, 1H), 6.56 (d, J = 9.2 Hz, 1H), 4.90 (s, 2H), 4.33 (s, 1H), 4.05 - 3.96 (m, 1H), 3.95 (s, 3H), 3.55 (d, J = 14.7 Hz, 1H), 3.28 - 2.97 (m, 2H), 1.44 (s, 9H).

### tert-Butyl 4-(5-amino-6-methoxypyridin-2-yl)-2-(trifluoromethyl)piperazine-1-carboxylate (Int. 14)

To a solution of tert-butyl 4-(6-methoxy-5-nitropyridin-2-yl)-2-(trifluoromethyl)piperazine-1 - carboxylate (**Int**. **13,** 0.23 g, 0.56 mmol, 1.0 eq.) in acetone (6.0 mL), zinc (0.35 g, 5.35 mmol, 9.65 eq.) was added. Then, a saturated solution of NH₄Cl (3.0 mL) was added dropwise at 0°C and the RM was stirred at RT for 1 h. The reaction mixture was diluted with DCM and filtrated through Celite^{®}. The filtrate was diluted with water and a saturated solution of NaHCOs and extracted with DCM (2x). The organic layers were combined, washed with brine, dried over Na₂SO₄, filtered and evaporated to obtain tert-butyl 4-(5-amino-6-methoxypyridin-2-yl)-2-(trifluoromethyl)piperazine-1-carboxylate (**Int**. **14,** 0.22 g, 0.58 mmol, 100%, yellow oil).

¹H NMR (400 MHz, DMSO-d₆) δ 6.87 (d, J = 8.1 Hz, 1H), 6.17 (d, J = 8.1 Hz, 1H), 4.78 (s, 1H), 4.33 (d, J = 13.9 Hz, 1H), 4.23 (s, 2H), 3.89 (s, 1H), 3.81 (s, 3H), 3.00 - 2.90 (m, 1H), 2.63 (td, J = 13.0, 4.4 Hz, 1H), 1.43 (s, 9H).

### tert-Butyl 4-[5-(2-cyclopropylpyrimidine-5-amido)-6-methoxypyridin-2-yl]-2-(trifluoromethyl)piperazine-1-carboxylate (Int. 15)

To a solution of 4-(5-amino-6-methoxypyridin-2-yl)-2-(trifluoromethyl)piperazine-1-carboxylate (**Int**. **14,** 0.22 g, 0.58 mmol, 1.0 eq.) in DMF (4.0 mL), 2-cyclopropylpyrimidine-5-carboxylic acid (0.095 g, 0.58 mmol, 1.0 eq.) was added, followed by DMAP (0.037 g, 0.303 mmol, 0.52 eq.) and DIPEA (0.25 mL, 1.43 mmol, 2.48 eq.). Then, T3P as 50 wt% solution in EtOAc (0.35 mL, 1.18 mmol, 2.03 eq.) was added. The reaction mixture was stirred under reflux for 1.5 h The reaction mixture was cooled down to RT and a saturated solution of NaHCOs was added in portion to pH 8-9. Then, the organic layer was separated and water layer was extracted with EtOAc. The combined organic layers were washed with brine, dried over Na₂SO₄, filtered and evaporated. The crude product was purified by FCC (0% to 2% MeOH gradient in DCM) to obtain tert-butyl 4-[5-(2-cyclopropylpyrimidine-5-amido)-6-methoxypyridin-2-yl]-2-(trifluoromethyl)piperazine-1-carboxylate (**Int**. **15,** 0.28 g, 0.41 mmol, 71%, purple oil, m/z [M+H]⁺: 521).

¹H NMR (400 MHz, DMSO-d₆) δ 9.81 (s, 1H), 9.06 (s, 2H), 7.67 (d, J = 8.5 Hz, 1H), 6.39 (d, J = 8.5 Hz, 1H), 4.80 (s, 1H), 4.68 (d, J = 14.5 Hz, 1H), 4.13 (s, 1H), 3.98 (s, 1H), 3.86 (s, 1H), 3.83 (s, 3H), 3.32 - 3.01 (m, 1H), 3.03 - 2.92 (m, 1H), 2.29 (ddd, J = 12.3, 8.0, 4.7 Hz, 1H), 1.43 (d, J = 5.2 Hz, 9H), 1.18 - 1.02 (m, 4H).

### tert-Butyl 4-{5-[N-({4-chloro-1H,3H-furo[3,4-c]quinolin-7-yl}methyl)2-cyclopropylpyrimidine-5-amido]-6-methoxypyridin-2-yl}-2-(trifluoromethyl)piperazine-1-carboxylate (Int. 16)

A suspension of tert-butyl 4-[5-(2-cyclopropylpyrimidine-5-amido)-6-methoxypyridin-2-yl]-2-(trifluoromethyl)piperazine-1-carboxylate (**Int. 15**, 0.23 g, 0.34 mmol, 1.0 eq.), 4-chloro-7-(chloromethyl)-1H,3H-furo[3,4-c]quinoline (**Int. A**, 0.1 g, 0.39 mmol, 1.15 eq.) and Cs₂CO₃ (0.17 g, 0.52 mmol, 1.54 eq.) in dry MeCN (2.0 mL) was stirred at 70°C for 2 h. The reaction mixture was cooled down, diluted with water and extracted with DCM. The combined organic layers were washed with brine, dried over Na₂SO₄, filtered, and evaporated. The residue was purified by FCC (0% to 50% EtOAc gradient in hexane) to obtain tert-butyl 4-{5-[N-({4-chloro-1H,3H-furo[3,4-c]quinolin-7-yl}methyl)2-cyclopropylpyrimidine-5-amido]-6-methoxypyridin-2-yl}-2-(trifluoromethyl)piperazine-1-carboxylate (**Int. 16**, 0.158 g, 0.19 mmol, 56%, brown solid).

### N-({4-Amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide; formic acid (Int. 17)

### Step 1:

A solution of tert-butyl 4-{5-[N-({4-chloro-1H,3H-furo[3,4-c]quinolin-7-yl}methyl)2-cyclopropylpyrimidine-5-amido]-6-methoxypyridin-2-yl}-2-(trifluoromethyl)piperazine-1-carboxylate (**Int. 16**, 0.158 g, 0.19 mmol, 1.0 eq.) and 1-(2,4-dimethoxyphenyl)methanamine (0.3 g, 1.79 mmol, 9.44 eq.) in DMSO (1.0 mL) was stirred at 120 °C for 16 h. The reaction mixture was diluted with EtOAc and washed three times with water followed by brine, dried over anhydrous Na₂SO₄, filtered and concentrated. The crude product was purified by FCC (2% to 10% MeOH gradient in DCM) to give tert-butyl 4-(5-{N-[(4-{[(2,4-dimethoxyphenyl)methyl]amino}-1H,3H-furo[3,4-c]quinolin-7-yl)methyl]2-cyclopropylpyrimidine-5-amido}-6-methoxypyridin-2-yl)-2-(trifluoromethyl)piperazine-1-carboxylate (0.2 g, 0.184 mmol, 97%, brown oil, m/z [M+H]⁺: 871).

### Step 2:

To a solution of tert-butyl 4-(5-{N-[(4-{[(2,4-dimethoxyphenyl)methyl]amino}-1H,3H-furo[3,4-c]quinolin-7-yl)methyl]2-cyclopropylpyrimidine-5-amido}-6-methoxypyridin-2-yl)-2-(trifluoromethyl)piperazine-1-carboxylate (0.2 g, 0.184 mmol, 1.0 eq.) in DCM (2.0 mL), TFA (0.30 mL, 3.90 mmol, 21.27 eq.) was added. The reaction mixture was stirred at RT for 16 h. Then, the reaction mixture was diluted with DCM and then partitioned between mixture of saturated solution of NaHCOs and chloroform/isopropanol 3:1 mixture. The aqueous phase was extracted with chloroform/isopropanol 3:1 and the combined organic layers were washed with a saturated solution of Na₂CO₃, brine, dried over Na₂SO₄, filtered and concentrated. The crude product was purified by FCC (0% to 5% MeOH gradient in DCM) to obtain N-({4-amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide; formic acid (**Int. 17**, 47 mg, 37%, white powder, m/z [M+H]⁺: 621).

¹H NMR (400 MHz, DMSO-d₆) δ 8.50 (d, J = 0.8 Hz, 2H), 7.46 - 7.29 (m, 2H), 7.21 - 7.08 (m, 2H), 6.45 (s, 2H), 6.20 (d, J = 8.5 Hz, 1H), 5.34 - 5.21 (m, 3H), 4.98 (t, J = 3.3 Hz, 2H), 4.67 (dd, J = 14.7, 8.5 Hz, 1H), 4.20 - 4.03 (m, 1H), 3.80 - 3.70 (m, 1H), 3.63 (d, J = 5.7 Hz, 3H), 2.98 - 2.81 (m, 4H), 2.65 - 2.57 (m, 1H), 2.17 - 2.06 (m, 1H), 1.06 - 1.00 (m, 2H), 0.99 - 0.88 (m, 2H).

¹⁹F NMR (377 MHz, DMSO-d₆) δ -74.07 (t, J = 6.3 Hz).

### N-({4-Amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide (Example 3)

A solution of N-({4-amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide; formic acid **(Int. 17)** in DCM was washed 2 times with a saturated solution of NaHCOs, dried over Na₂SO₄, filtered and concentrated to obtain N-({4-amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide

### (Example 3, 24 mg, white powder, UPLC long elution (polar long method, buffer type "FA") purity 98.28%, m/z [M+H]⁺: 621.00).

¹H NMR (400 MHz, DMSO-d₆) δ 1H NMR (400 MHz, DMSO) δ 8.50 (d, J = 0.8 Hz, 2H), 7.57 - 7.34 (m, 2H), 7.27 - 7.11 (m, 2H), 6.45 (s, 2H), 6.20 (d, 1H), 5.35 - 5.20 (m, 3H), 4.98 (t, J = 3.4 Hz, 2H), 4.67 (dd, J = 14.6, 8.6 Hz, 1H), 4.12 (t, J = 12.7 Hz, 1H), 3.87 - 3.67 (m, 1H), 3.63 (d, J = 5.8 Hz, 3H), 3.01 - 2.76 (m, 4H), 2.66 - 2.59 (m, 1H), 2.19 - 2.10 (m, 1H), 1.13 - 1.00 (m, 2H), 0.98 - 0.85 (m, 2H), silicone grease - 0,06 ppm (2H).

¹⁹F NMR (377 MHz, DMSO-*d*₆) δ -74.07.

### Example 4: N-({4-Amino-1H,3H-furo[3,4-c]quinolin-7-yl}methyl)-2-cyclopropyl-N-(3,4-dihydro-2H-1,4-benzoxazin-8-yl)pyrimidine-5-carboxamide

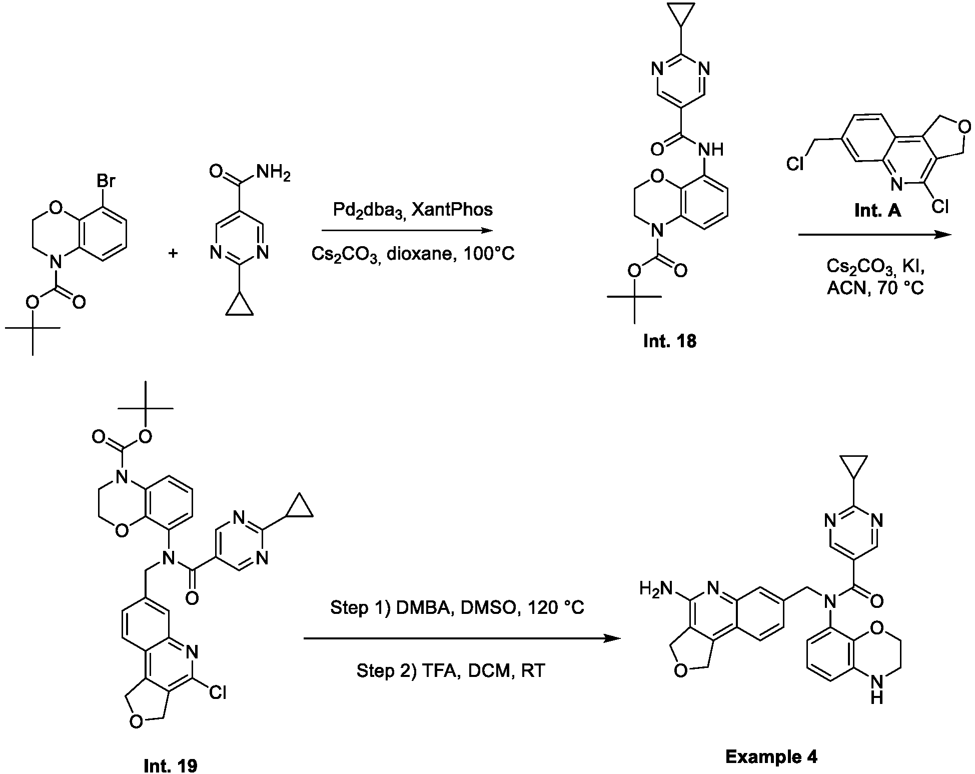

### tert-Butyl- 8-(2-cyclopropylpyrimidine-5-amido)-3,4-dihydro-2H-1,4-benzoxazine-4-carboxylate (Int. 18)

A solution of 2-cyclopropylpyrimidine-5-carboxamide (0.2 g, 1.19 mmol, 1.0 eq.), tert-butyl 8-bromo-3,4-dihydro-2H-1,4-benzoxazine-4-carboxylate (0.373 g, 1.19 mmol, 1.0 eq.) and Cs₂CO₃ (0.97 g, 2.97 mmol, 2.51 eq.) in dry dioxane (15.0 mL) was flushed with argon for 10min. Then, Pd₂dba₃ (0.22 g, 0.24 mmol, 0.20 eq.) and XantPhos (0.21 g, 0.36 mmol, 0.31 eq.) were added and the reaction mixture was stirred at 100°C for 4 h. Then, the RM was diluted with water/ saturated solution of NaHCOs and extracted with EtOAc. The combined organic layers were washed with brine and dried over Na₂SO₄, filtered and evaporated. The crude product was purified by FCC (0% to 40% EtOAc gradient in hexane) to obtain tert-butyl 8-(2-cyclopropylpyrimidine-5-amido)-3,4-dihydro-2H-1,4-benzoxazine-4-carboxylate (**Int. 18**, 294 mg, 59%).

¹H NMR (400 MHz, DMSO-*d*₆) δ 9.87 (s, 1H), 9.06 (s, 2H), 7.62 (d, J = 8.4 Hz, 1H), 7.37 (dd, J = 7.9, 1.4 Hz, 1H), 7.03 - 6.77 (m, 1H), 4.27 (dd, J = 5.2, 3.8 Hz, 2H), 3.80 (dd, J = 5.2, 3.8 Hz, 2H), 2.30 (tt, J = 8.0, 4.7 Hz, 1H), 1.49 (s, 9H), 1.20 - 1.02 (m, 4H).

### tert-Butyl- 8-[N-({4-chloro-1H,3H-furo[3,4-c]quinolin-7-yl}methyl)2-cyclopropylpyrimidine-5-amido]-3,4-dihydro-2H-1,4-benzoxazine-4-carboxylate (Int. 19)

The mixture of tert-butyl 8-(2-cyclopropylpyrimidine-5-amido)-3,4-dihydro-2H-1,4-benzoxazine-4-carboxylate (**Int. 18**, 0.272 g, 0.645 mmol), 4-chloro-7-(chloromethyl)-1H,3H-furo[3,4-c]quinoline (**Int**. **A,** 0.166 g, 0.645 mmol), KI (0.107 g, 0.645 mmol) and Cs₂CO₃ (0.252 g, 0.774 mmol) in MeCN (3.5 mL) was stirred at 70 °C for 20 h. The reaction mixture was diluted with EtOAc and water. The layers were separated and the water layer was extracted with EtOAc several times. The combined organic layers were washed with brine and dried over anhydrous MgSO₄, filtered and evaporated. The crude product was purified by FCC (0% to 80% EtOAc gradient in Hexane) to obtain tert-butyl 8-[N-({4-chloro-1H,3H-furo[3,4-c]quinolin-7-yl}methyl)2-cyclopropylpyrimidine-5-amido]-3,4-dihydro-2H-1,4-benzoxazine-4-carboxylate (**Int. 19**, 248 mg, 58%, solid, m/z [M+H]⁺ : 614.8).

¹H NMR (400 MHz, DMSO-*d*₆) δ 8.52 (s, 2H), 7.92 (d, J = 1.6 Hz, 1H), 7.80 (d, J = 8.4 Hz, 1H), 7.69 (dd, J = 8.5, 1.7 Hz, 1H), 7.67 - 7.59 (m, 1H), 6.89 (dd, J = 7.9, 1.5 Hz, 1H), 6.80 - 6.67 (m, 1H), 5.58 - 5.47 (m, 2H), 5.35 (d, J = 15.1 Hz, 1H), 5.25 - 5.15 (m, 2H), 5.01 (d, J = 15.1 Hz, 1H), 4.04 (t, J = 4.6 Hz, 2H), 3.73 (dt, J = 13.8, 4.1 Hz, 1H), 3.26 (dt, J = 13.9, 4.7 Hz, 1H), 2.11 (tt, J = 8.4, 4.6 Hz, 1H), 1.42 (s, 9H), 1.08 - 0.87 (m, 4H).

### N-({4-Amino-1H,3H-furo[3,4-c]quinolin-7-yl}methyl)-2-cyclopropyl-N-(3,4-dihydro-2H-1,4-benzoxazin-8-yl)pyrimidine-5-carboxamide (Example 4).

### Step 1:

tert-Butyl- 8-[N-({4-chloro-1H,3H-furo[3,4-c]quinolin-7-yl}methyl)2-cyclopropylpyrimidine-5-amido]-3,4-dihydro-2H-1,4-benzoxazine-4-carboxylate (**Int. 19**, 0.244 g, 0.369 mmol, 1.0 eq.) and 1-(2,4-dimethoxyphenyl)methanamine (0.28 mL, 1.85 mmol, 5 eq.) in DMSO (3.0 mL) was stirred at 120°C for 10 h. The reaction mixture was diluted with EtOAc and a saturated solution of NaHCOs. The water layer was extracted with EtOAc twice. The combined organic layers were washed with brine and dried over anhydrous MgSO₄, filtered and evaporated. The crude product was purified by FCC (0 to 5% MeOH gradient in DCM) to give of tert-butyl 8-{N-[(4-{[(2,4-dimethoxyphenyl)methyl]amino}-1H,3H-furo[3,4-c]quinolin-7-yl)methyl]2-cyclopropylpyrimidine-5-amido}-3,4-dihydro-2H-1,4-benzoxazine-4-carboxylate (295 mg, 67%, m/z [M+H]⁺ : 746.3).

¹H NMR (400 MHz, DMSO-*d*₆) δ 8.49 (s, 2H), 7.59 (d, J = 8.6 Hz, 1H), 7.46 (d, J = 1.6 Hz, 1H), 7.40 (d, J = 8.1 Hz, 1H), 7.16 (dd, J = 8.2, 1.6 Hz, 1H), 7.12 (d, J = 8.4 Hz, 1H), 7.00 (t, J = 5.7 Hz, 1H), 6.82 (dd, J = 7.9, 1.5 Hz, 1H), 6.76 - 6.66 (m, 1H), 6.55 (d, J = 2.4 Hz, 1H), 6.41 (dd, J = 8.4, 2.4 Hz, 1H), 5.29 (d, J = 3.2 Hz, 2H), 5.24 (d, J = 14.9 Hz, 1H), 5.04 (d, J = 3.2 Hz, 2H), 4.87 (d, J = 14.9 Hz, 1H), 4.66 - 4.45 (m, 2H), 4.08 - 3.93 (m, 2H), 3.82 (s, 3H), 3.71 (s, 3H), 2.10 (tt, J = 8.3, 4.6 Hz, 1H), 1.09 - 0.83 (m, 4H).

### Step 2:

To a solution of tert-butyl- 8-{N-[(4-{[(2,4-dimethoxyphenyl)methyl]amino}-1H,3H-furo[3,4-c] quinolin-7-yl)methyl-2-cyclopropylpyrimidine-5-amido}-3,4-dihydro -2H-1,4-benzoxazine-4- carboxylate (0.295 g, 0.30 mmol, 1.0 eq.) in DCM (3.5 mL), TFA (1.17 mL, 15.2 mmol, 50 eq.) was added dropwise. The reaction mixture was stirred at RT for 6 h. Then, the reaction mixture was diluted with DCM and poured into icy water. A solution of NaOH 1M was added to the mixture. The layers were separated. The water layer was extracted with DCM (2x). The organic layers were combined, washed with a saturated solution of Na₂CO₃, brine and dried over anhydrous MgSO₄, filtered and evaporated. The crude product was purified by FCC (0% to 20% MeOH gradient in DCM) followed by preparative HPLC purification (basic conditions) and lyophilized to give N-({4-amino-1H,3H-furo[3,4-c]quinolin-7-yl}methyl)-2-cyclopropyl-N- 3,4-dihydro-2H-1,4-benzoxazin -8-yl)pyrimidine-5-carboxamide (**Example 4**, 11.43 mg, 8%,off-white solid, UPLC long elution (polar long method, buffer type "FA") purity 99.12%, m/z [M+H]⁺ : 495.7).

¹H NMR (400 MHz, DMSO-*d*₆) δ 8.48 (s, 2H), 7.46 - 7.35 (m, 2H), 7.16 (dd, J = 8.2, 1.6 Hz, 1H), 6.59 - 6.47 (m, 1H), 6.44 (s, 2H), 6.36 (dd, J = 8.0, 1.5 Hz, 1H), 6.30 (dd, J = 7.8, 1.5 Hz, 1H), 5.90 - 5.80 (m, 1H), 5.28 (t, J = 3.4 Hz, 2H), 5.21 (d, J = 14.8 Hz, 1H), 4.98 (t, J = 3.4 Hz, 2H), 4.83 (d, J = 14.8 Hz, 1H), 3.96 - 3.86 (m, 1H), 3.69 - 3.58 (m, 1H), 3.18 - 3.06 (m, 1H), 3.03 - 2.92 (m, 1H), 2.18 -2.06 (m, 1H), 1.11 - 0.84 (m, 4H).

### Example 5: N-({4-Amino-1H,3H-furo[3,4-c]quinolin-7-yl}methyl)-N-(3-methoxy-1-methyl-1H-pyrazol-4-yl)-6-[3-(trifluoromethyl)piperazin-1-yl]pyridine-3-carboxamide

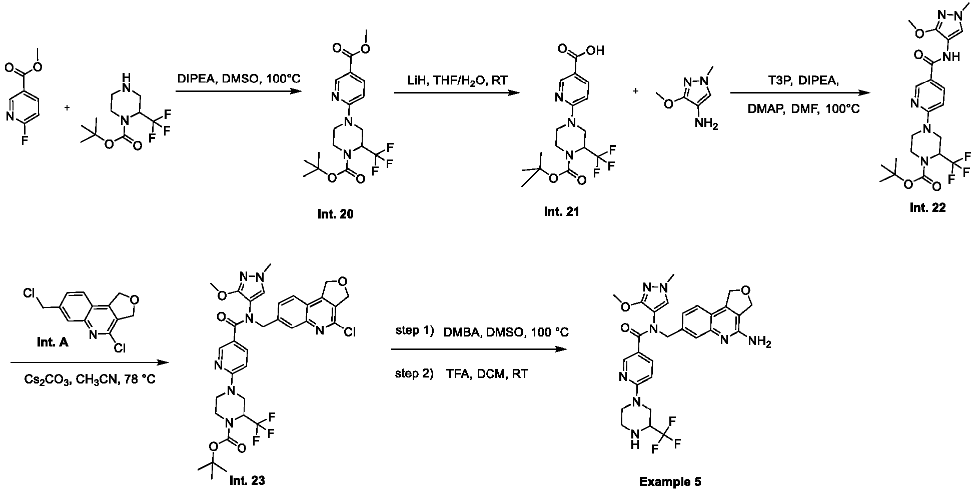

### tert-Butyl-4-[5-(methoxycarbonyl)pyridin-2-yl]-2-(trifluoromethyl)piperazine-1-carboxylate (Int. 20)

Solution of methyl 6-fluoropyridine-3-carboxylate (0.255 g, 1.644 mmol, 1.0 eq.), tert-butyl 2-(trifluoromethyl)piperazine-1-carboxylate (0.453 g, 1.782 mmol, 1.084 eq.) and DIPEA (1.4 ml, 8.037 mmol, 4.889 eq.) in DMSO (8.0 mL) was stirred at 100°C overnight. The reaction mixture was diluted with a saturated solution of Na₂CO₃ and the crude product was extracted with EtOAc (2x). The combined organic layers were washed with brine and dried over Na₂SO₄, filtered and evaporated. The crude product was purified by FCC (0% to 10% EtOAc gradient in heptane) to obtain tert-butyl-4-[5-(methoxycarbonyl)pyridin-2-yl]-2-(trifluoromethyl)piperazine-1-carboxylate (**Int. 20**, 0.47 g, 1.098 mmol, 67%, white solid, m/z [M+H]⁺: 390.4).

¹H NMR (400 MHz, DMSO-*d*₆) δ 8.66 (d, J = 0.7 Hz, 1H), 7.99 (dd, J = 9.1, 2.4 Hz, 1H), 6.91 (d, J = 9.1 Hz, 1H), 4.97 - 4.70 (m, 2H), 4.43 - 4.21 (m, 1H), 4.15 - 3.93 (m, 1H), 3.80 (s, 3H), 3.47 (dd, J = 15.1, 4.6 Hz, 1H), 3.23 - 3.05 (m, 2H), 1.44 (s, 9H).

### 6-{4-[(tert-Butoxy)carbonyl]-3-(trifluoromethyl)piperazin-1-yl}pyridine-3-carboxylic acid (Int. 21)

A solution of tert-butyl 4-[5-(methoxycarbonyl)pyridin-2-yl]-2 (trifluoromethyl)piperazine-1-carboxylate (**Int. 20**, 0.45 g, 1.052 mmol, 1.0 eq.), LiOH (0.09 g, 2.145 mmol, 2.039 eq.) in water (2.7 mL) and THF (5.3 mL) was stirred at RT over 48 h. The reaction mixture was concentrated, diluted with water and extraction with EtOAc (2x). The combined organic layers was washed with brine and dried over Na₂SO₄, filtered and evaporated to obtain 6-{4-[(tert-butoxy)carbonyl]-3-(trifluoromethyl)piperazin-1-yl}pyridine-3-carboxylic acid (**Int. 21**, 0.365 g, 0.904 mmol, 86%, white solid, m/z [M+H]⁺: 376.3 ).

¹H NMR (400 MHz, DMSO-*d*₆) δ 12.59 (s, 1H), 8.64 (d, 1H), 7.97 (dd, J = 9.0, 2.4 Hz, 1H), 6.89 (d, J = 9.1 Hz, 1H), 5.08 - 4.73 (m, 2H), 4.47 - 4.16 (m, 1H), 4.12 - 3.78 (m, 1H), 3.45 (dd, J = 14.2 Hz, 1H), 3.19 - 2.98 (m, 2H), 1.44 (s, 9H).

### tert-Butyl 4-{5-[(3-methoxy-1-methyl-1H-pyrazol-4-yl)carbamoyl]pyridin-2-yl}-2-(trifluoromethyl)piperazine-1-carboxylate (Int. 22)

To a solution of DMAP (0.051 g, 0.417 mmol, 0.512 eq.), DIPEA (0.355 ml, 2.038 mmol, 2.5 eq.), 3-methoxy-1-methyl-1H-pyrazol-4-amine hydrochloride (0.141 g, 0.862 mmol, 1.057 eq.) and 6-{4-[(tert-butoxy)carbonyl]-3-(trifluoromethyl)piperazin-1-yl}pyridine-3-carboxylic acid (**Int**. **21,** 0.329 g, 0.815 mmol, 1.0 eq.) in DMF (3.0 mL), T3P as 50% solution in EtOAc (0.5 mL, 1.68 mmol, 2.061 eq.) was added dropwise at 0°C and the reaction mixture was heated at 100°C for 2 h. Then, the reaction mixture was diluted with a saturated solution of Na₂CO₃, extracted with EtOAc (2x) and the combined organic layers were washed with brine and dried over Na₂SO₄, filtered and evaporated. The crude product was purified by FCC (0% to 5% MeOH gradient in DCM) to give tert-butyl 4-{5-[(3-methoxy-1-methyl-1H-pyrazol-4-yl)carbamoyl]pyridin-2-yl}-2-(trifluoromethyl)piperazine-1-carboxylate (**Int. 22**, 0.38 g, 0.604 mmol, 74%, brown oil, m/z [M+H]⁺: 485.6).

¹H NMR (400 MHz, DMSO-*d*₆) δ 9.47 (s, 1H), 8.68 (d, J = 2.4 Hz, 1H), 8.07 (dd, J = 9.0, 2.5 Hz, 1H), 7.77 (s, 1H), 6.89 (d, J = 9.1 Hz, 1H), 4.95 - 4.76 (m, 2H), 4.39 - 4.20 (m, 1H), 4.07 - 3.93 (m, 1H), 3.82 (s, 3H), 3.68 (s, 3H), 3.50 - 3.38 (m, 1H), 3.16 - 3.03 (m, 2H), 1.45 (s, 9H).

### tert-Butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-2-(trifluoromethyl)piperazine-1-carboxylate (Int. 23)

To a solution of tert-butyl 4-{5-[(3-methoxy-1-methyl-1H-pyrazol-4-yl)carbamoyl]pyridin-2-yl}-2-(trifluoromethyl)piperazine-1-carboxylate (**Int. 22**, 0.349 g, 0.555 mmol, 1.0 eq.) and 4-chloro-7-(chloromethyl)-1H,3H-furo[3,4-c]quinoline (**Int**. **A,** 0.187 g, 0.729 mmol, 1.313 eq.) in MeCN (3.0 mL), Cs₂CO₃ (0.365 g, 1.12 mmol, 2.02 eq.) was added and the reaction mixture was stirred at 78°C for 2 h. Then, the reaction mixture was diluted a saturated solution of Na₂CO₃, extracted with EtOAc (2x) and the combined organic layers were washed with brine and dried over Na₂SO₄, filtered and evaporated. The crude product was purified by FCC (0% to 2% MeOH gradient in DCM) to obtain tert-butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-2-(trifluoromethyl)piperazine-1-carboxylate (**Int. 23**, 0.34 g, 0.305 mmol, 55%, orange oil, m/z [M+H]⁺: 702.8).

¹H NMR (400 MHz, DMSO-*d*₆) δ 8.19 (d, J = 2.4 Hz, 1H), 7.89 (d, J = 1.6 Hz, 1H), 7.83 (d, J = 8.4 Hz, 1H), 7.65 (dd, J = 8.5, 2.4 Hz, 1H), 7.57 (dd, J = 9.0, 2.5 Hz, 1H), 7.44 (s, 1H), 6.74 (d, J = 9.0 Hz, 1H), 5.54 (t, J = 3.1 Hz, 2H), 5.23 (t, J = 3.1 Hz, 2H), 5.02 (s, 2H), 4.79 - 4.63 (m, 2H), 4.23 - 4.17 (m, 1H), 4.03 - 3.92 (m, 1H), 3.60 (s, 3H), 3.51 (s, 3H), 3.03 - 2.96 (m, 3H), 1.44 (s, 9H).

### N-({4-amino-1H,3H-furo[3,4-c]quinolin-7-yl}methyl)-N-(3-methoxy-1-methyl-1H-pyrazol-4-yl)-6-[3-(trifluoromethyl)piperazin-1-yl]pyridine-3-carboxamide (Example 5)

### Step 1:

To a solution of tert-butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-2(trifluoromethyl)piperazine-1-carboxylate (**Int. 23**, 0.315 g, 0.283 mmol, 1.0 eq.) in DMSO (1.5 mL) 1-(2,4-dimethoxyphenyl)methanamine (0.5 g, 2.99 mmol, 10.58 eq.) was added and the reaction was stirred at 100°C overnight. Then, the reaction mixture was cooled down to RT, diluted with water, extracted with EtOAc (2x) and the combined organic layers were washed with brine and dried over Na₂SO₄, filtered and evaporated. The crude product was purified by FCC (0% to 5% MeOH gradient in DCM)

### Step 2:

To a solution of the product dissolved in DCM (1.5 mL),TFA (1.1 mL, 14.374 mmol, 50.856 eq.) was added at 0°C and the reaction was stirred at RT overnight. Then, the reaction mixture was dilated in DCM and quenched with a saturated solution of Na₂CO₃ aq. The water layer was washed with DCM and then organic phases were combined, dried over Na₂SO₄, filtered and evaporated. The crude product was purified by preparative HPLC and lypophilzed with EtOH and water to obtain N-({4-amino-1H,3H-furo[3,4-c]quinolin-7-yl}methyl)-N-(3-methoxy-1-methyl-1H-pyrazol-4-yl)-6-[3-(trifluoromethyl)piperazin-1-yl]pyridine-3-carboxamide (**Example 5**, 0.042 g, 0.072 mmol, 25%, UPLC long elution (polar long method, buffer type "FA") purity 99.99%, white solid, m/z [M+H]⁺: 583.5).

¹H NMR (400 MHz, DMSO-*d*₆) δ 8.17 (d, J = 2.4 Hz, 1H), 7.54 (dd, J = 9.0, 2.4 Hz, 1H), 7.43 (d, J = 8.3 Hz, 1H), 7.41 (d, 1H), 7.30 (s, 1H), 7.13 (dd, J = 8.2, 1.7 Hz, 1H), 6.76 (d, J = 9.0 Hz, 1H), 6.45 (s, 2H), 5.30 (t, J = 3.4 Hz, 2H), 4.99 (t, J = 3.4 Hz, 2H), 4.89 (s, 2H), 4.34 (dd, 1H), 3.95 - 3.85 (m, 1H), 3.63 (s, 3H), 3.50 (s, 3H), 3.42 - 3.38 (m, 1H), 3.00 - 2.94 (m, 4H), 2.76 - 2.63 (m, 1H).

¹⁹F NMR (377 MHz, DMSO-*d*₆) δ -74.02 (d, J = 7.7 Hz).

### Example 6: N-({4-Amino-1H,3H-furo[3,4-c]quinolin-7-yl}methyl)-6-(6,6-difluoro-1,4-diazepan-1-yl)-N-(3-methoxy-1-methyl-1H-pyrazol-4-yl)pyridine-3-carboxamide

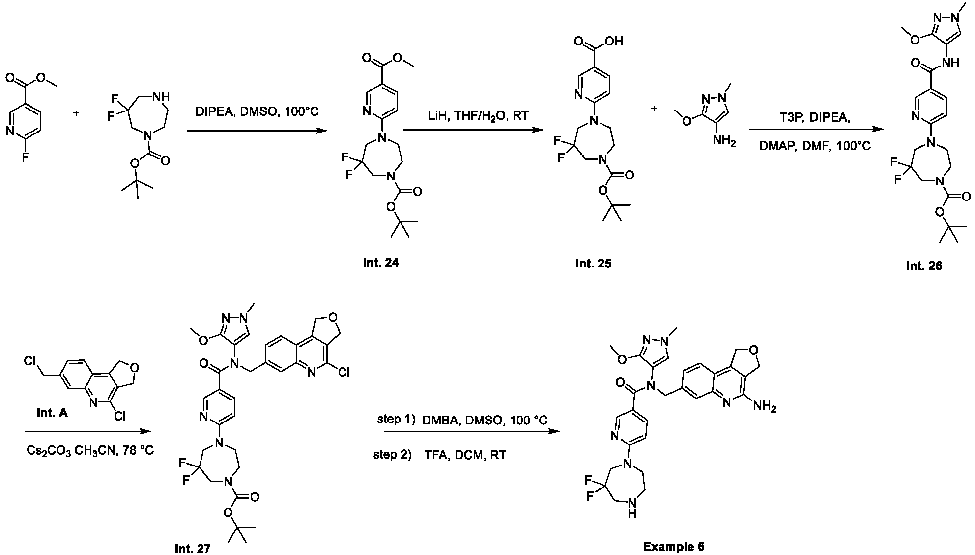

### tert-Butyl 6,6-difluoro-4-[5-(methoxycarbonyl)pyridin-2-yl]-1,4-diazepane-1-carboxylate (Int. 24)

Solution of methyl 6-fluoropyridine-3-carboxylate (0.152 g, 0.98 mmol, 1.0 eq.), tert-butyl 6,6-difluoro-1,4-diazepane-1-carboxylate (0.344 g, 1.456 mmol, 1.486 eq.) and DIPEA (0.9 ml, 5.167 mmol, 5.273 eq.) in DMSO (5.0 mL) was stirred at 125°C overnight. Then, the reaction mixture was quenched with a saturated solution of Na₂CO₃, extracted with EtOAc (2x) and the combined organic layers were washed with brine and dried over Na₂SO₄, filtered and evaporated. The residue was purified by FCC (0% to 20% EtOAc gradient in heptane) to give tert-butyl 6,6-difluoro-4-[5-(methoxycarbonyl)pyridin-2-yl]-1,4-diazepane-1-carboxylate (**Int**. **24,** 0.18 g, 0.417 mmol, 43%, white solid, m/z [M+H]⁺: 372.4).

¹H NMR (400 MHz, DMSO-*d*₆) δ 8.66 (d, J = 2.3 Hz, 1H), 8.00 (dd, J = 9.1, 2.4 Hz, 1H), 6.94 (d, J = 9.1 Hz, 1H), 4.27 (t, J = 12.2 Hz, 2H), 3.97 - 3.85 (m, 2H), 3.80 (s, 3H), 3.78 - 3.73 (m, 2H), 3.70 - 3.55 (m, 2H), 1.36 (d, J = 8.0 Hz, 9H).

¹⁹F NMR (377 MHz, DMSO-*d*₆) δ -99.75, -101.13.

### 6-{4-[(tert-Butoxy)carbonyl]-6,6-difluoro-1,4-diazepan-1-yl}pyridine-3-carboxylic acid (Int. 25)

To a solution of tert-butyl 6,6-difluoro-4-[5-(methoxycarbonyl)pyridin-2-yl]-1,4-diazepane-1-carboxylate (**Int**. **24,** 0.16 g, 0.371 mmol, 1.0 eq.), LiOH (0.033 g, 0.786 mmol, 2.123 eq.) was added water (1 mL) and THF (2 mL) and the reaction mixture was stirred at 60°C for 2.5h. The reaction mixture was concentrated, diluted with water and extracted with EtOAc (2x). The combined organic layers were washed with brine and dried over Na₂SO₄, filtered and evaporated to obtain 6-{4-[(tert-butoxy)carbonyl]-6,6-difluoro-1,4-diazepan-1-yl}pyridine-3-carboxylic acid (**Int**. **25,** 0.112 g, 0.216 mmol, 58%, white solid, m/z [M+H]⁺: 358.4).

¹H NMR (400 MHz, DMSO-*d*₆) δ 12.58 (s, 1H), 8.64 (d, J = 2.3 Hz, 1H), 7.97 (dd, J = 9.0, 2.4 Hz, 1H), 6.91 (d, J = 9.0 Hz, 1H), 4.26 (t, J = 12.2 Hz, 2H), 3.96 - 3.85 (m, 2H), 3.85 - 3.72 (m, 2H), 3.72 - 3.54 (m, 2H), 1.36 (d, J = 8.9 Hz, 9H).

¹⁹F NMR (377 MHz, DMSO-*d*₆) δ -99.59, -100.98.

### tert-Butyl 6,6-difluoro-4-{5-[(3-methoxy-1-methyl-1H-pyrazol-4-yl)carbamoyl]pyridin-2-yl}-1,4-diazepane-1-carboxylate (Int. 26)

DMAP (0.013 g, 0.106 mmol, 0.551 eq.), DIPEA (0.1 mL, 0.574 mmol, 2.973 eq.), 6-{4-[(tert-butoxy)carbonyl]-6,6-difluoro-1,4-diazepan-1-yl}pyridine-3-carboxylic acid (**Int**. **25,** 0.1 g, 0.193 mmol, 1.0 eq.) and 3-methoxy-1-methyl-1H-pyrazol-4-amine hydrochloride (0.035 g, 0.214 mmol, 1.108 eq.) were taken in DMF (1.0 mL) and T3P as 50% solution in EtOH (0.15 mL, 0.504 mmol, 2.61 eq.) was added dropwise at 0°C. Then, the reaction mixture was stirred at 100°C for 1 h. The reaction mixture was quenched with a saturated solution of Na₂CO₃, extracted with EtOAc (2x) and the combined organic layers were washed with brine and dried over Na₂SO₄, filtered and evaporated. The residue was purified by FCC (0% to 10% MeOH gradient in DCM) to give tert-butyl 6,6-difluoro-4-{5-[(3-methoxy-1-methyl-1H-pyrazol-4-yl)carbamoyl]pyridin-2-yl}-1,4-diazepane-1-carboxylate (**Int**. **26,** 0.065 g, 0.114 mmol, 59%, orange oil, m/z [M+H]⁺: 467.6). ¹H NMR (400 MHz, DMSO-*d*₆) δ 9.47 (s, 1H), 8.68 (d, J = 2.4 Hz, 1H), 8.07 (dd, J = 9.0, 2.5 Hz, 1H), 7.77 (s, 1H), 6.91 (d, J = 9.0 Hz, 1H), 4.26 (t, J = 12.3 Hz, 2H), 3.98 - 3.87 (m, 2H), 3.82 (s, 3H), 3.79 - 3.74 (m, 2H), 3.68 (s, 3H), 3.64 - 3.54 (m, 2H), 1.37 (d, J = 4.2 Hz, 9H).

### tert-Butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-6,6-difluoro-1,4-diazepane-1-carboxylate (Int. 27)

To a solution of tert-butyl 6,6-difluoro-4-{5-[(3-methoxy-1-methyl-1H-pyrazol-4-yl)carbamoyl]pyridin-2-yl}-1,4-diazepane-1-carboxylate (**Int. 26**, 0.06 g, 0.105 mmol, 1.0 eq.) and 4-chloro-7-(chloromethyl)-1H,3H-furo[3,4-c]quinoline (**Int. A**, 0.037 g, 0.144 mmol, 1.367 eq.) in MeCN (1.0 mL), Cs₂CO₃ (0.0723 g, 0.222 mmol, 2.104 eq.) was added. The reaction mixture was stirred at 78°C for 6 h. The reaction mixture was quenched with a saturated solution of Na₂CO₃, extracted with EtOAc (2x) and the combined organic layers were washed with brine and dried over Na₂SO₄, filtered and evaporated. The residue was purified by FCC (0% to 5% MeOH gradient in DCM) to give tert-butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-6,6-difluoro-1,4-diazepane-1-carboxylate (**Int. 27**, 0.083 g, 0.101 mmol, 96%, yellow solid, m/z [M+H]⁺: 685.0).

¹H NMR (400 MHz, DMSO-*d*₆) δ 8.19 (s, 1H), 7.89 (s, 1H), 7.83 (dd, J = 8.2, 1.8 Hz, 1H), 7.65 (d, J = 8.4 Hz, 1H), 7.58 (d, J = 9.0 Hz, 1H), 7.44 (s, 1H), 6.76 (d, J = 9.0 Hz, 1H), 5.56 - 5.50 (m, 2H), 5.23 (s, 2H), 5.02 (s, 2H), 4.17 (t, J = 12.2 Hz, 2H), 3.88 - 3.69 (m, 4H), 3.59 (s, 3H), 3.59 - 3.54 (m, 2H), 3.53 - 3.47 (m, 3H), 1.36 (s, 9H).

### N-({4-amino-1H,3H-furo[3,4-c]quinolin-7-yl}methyl)-6-(6,6-difluoro-1,4-diazepan-1-yl)-N-(3-methoxy-1-methyl-1H-pyrazol-4-yl)pyridine-3-carboxamide (Example 6)

### Step 1:

To a solution of tert-butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-6,6-difluoro-1,4-diazepane-1-carboxylate

(**Int. 27**, 0.08 g, 0.097 mmol, 1.0 eq.) in DMSO (1.0 mL) 1-(2,4-dimethoxyphenyl)methanamine (0.2 g, 1.196 mmol, 12.324 eq.) was added and the reaction was stirred at 100°C overnight. Then, the reaction mixture was cooled down to RT, diluted with water, extracted with EtOAc (2x) and the combined organic layers were washed with brine and dried over Na₂SO₄, filtered and evaporated.

### Step 2:

To a solution of the product in DCM (0.5 mL), TFA (0.4 mL, 5.227 mmol, 53.856 eq.) was added at 0°C and the reaction was stirred at RT overnight. Then, the reaction mixture was dissolved DCM and quenched with a saturated solution of Na₂CO₃. The crude product was extracted with DCM and then organic phases were combined, dried over Na₂SO₄, filtered and evaporated. The residue was purified by preparative HPLC and lypophilzed with EtOH and water to give N-({4-amino-1H,3H-furo[3,4-c]quinolin-7-yl}methyl)-6-(6,6-difluoro-1,4-diazepan-1-yl)-N-(3-methoxy-1-methyl-1H-pyrazol-4-yl)pyridine-3-carboxamide (**Example 6,** 0.014 g, 0.023 mmol, 24%, light yellow solid, UPLC long elution (polar long method, buffer type "FA") purity 98.55%, m/z [M+H]⁺: 565.6).

¹H NMR (400 MHz, DMSO-*d*₆) δ 8.14 (d, J = 2.4 Hz, 1H), 7.53 (dd, J = 9.0, 2.4 Hz, 1H), 7.46 - 7.39 (m, 2H), 7.31 (s, 1H), 7.13 (dd, J = 8.2, 1.7 Hz, 1H), 6.69 (d, J = 9.1 Hz, 1H), 6.45 (s, 2H), 5.30 (t, J = 3.3 Hz, 2H), 5.00 (t, J = 3.4 Hz, 2H), 4.89 (s, 2H), 4.16 (t, J = 12.6 Hz, 2H), 3.84 - 3.76 (m, 1H), 3.71 (t, J = 5.2 Hz, 2H), 3.62 (s, 3H), 3.50 (s, 3H), 2.97 - 2.83 (m, 4H).

¹⁹F NMR (377 MHz, DMSO-*d*₆) δ -96.91 (q, J = 12.9 Hz).

### Example 7: N-({4-Amino-1H,3H-furo[3,4-c]quinolin-7-yl}methyl)-N-(3-chloro-1-methyl-1H-pyrazol-4-yl)-6-[3-(trifluoromethyl)piperazin-1-yl]pyridine-3-carboxamide

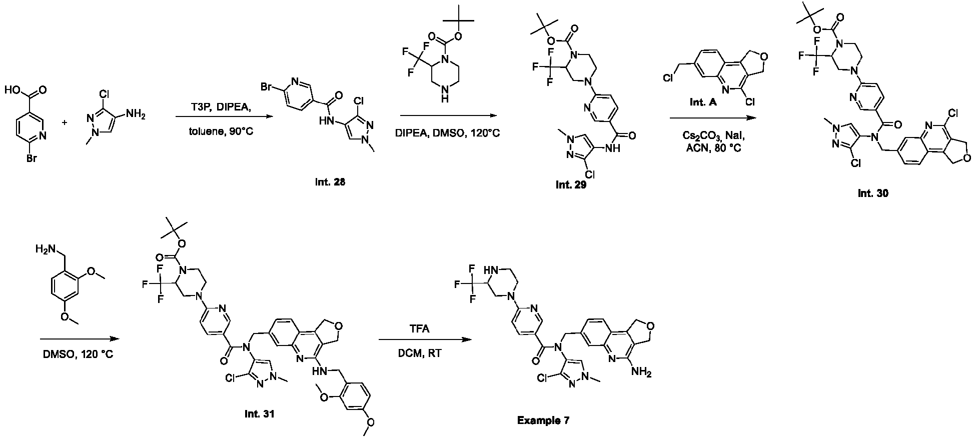

### 6-Bromo-N-(3-chloro-1-methyl-1H-pyrazol-4-yl)pyridine-3-carboxamide (Int. 28)

DMAP (0.023 g, 0.19 mmol, 0.05 eq.), DIPEA (1.71 ml, 9.50 mmol, 2.5 eq.), 3-chloro-1-methyl-1H-pyrazol-4-amine (0.5 g, 3.80 mmol, 1.0 eq.) 6-bromopyridine-3-carboxylic acid (0.768 g, 3.80 mmol, 1.0 eq.) were taken in toluene (11 mL) and T3P as 50% in EtOAc (4.52 mL, 7.60 mmol, 2 eq.) was added dropwise at 0°C. Then, the reaction mixture was stirred at 90°C for 2 h. The reaction mixture was diluted with a saturated solution of Na₂CO₃, extracted with EtOAc (2x) and the combined organic layers were washed with brine and dried over MgSO4, filtered and evaporated. The crude product was purified by FCC (0% to 100% EtOAc gradient in hexane) to obtain 6-bromo-N-(3-chloro-1-methyl-1H-pyrazol-4-yl)pyridine-3-carboxamide (**Int. 28**, 992 mg, 3.14 mmol, 82%, m/z [M+H]⁺: 315.20).

¹H NMR (400 MHz, DMSO-*d*₆) δ 10.19 (s, 1H), 8.88 (dd, J = 2.6, 0.8 Hz, 1H), 8.20 (dd, J = 8.3, 2.5 Hz, 1H), 8.08 (s, 1H), 7.83 (dd, J = 8.4, 0.8 Hz, 1H), 3.82 (s, 3H).

### tert-Butyl 4-{5-[(3-chloro-1-methyl-1H-pyrazol-4-yl)carbamoyl]pyridin-2-yl}-2-(trifluoromethyl)piperazine-1-carboxylate (Int. 29)

To a solution of 6-bromo-N-(3-chloro-1-methyl-1H-pyrazol-4-yl)pyridine-3-carboxamide (**Int. 28,** 0.223 g, 0.70 mmol, 1.0 eq.) in DMSO (3 mL), tert-butyl 2-(trifluoromethyl)piperazine-1-carboxylate (0.179 g, 0.70 mmol, 1.343 eq.) and DIPEA (0.3 mL, 1.75 mmol, 2.5 eq.) were added. The reaction mixture was stirred at 120°C for 24 h. The reaction mixture was diluted with EtOAc and poured into a saturated solution of NaHCOs. The organic layers were separated and the water layer was extracted with EtOAc. The combined organic layers were washed with brine and dried over MgSO₄, filtered and evaporated. The crude product was purified by FCC (0% to 60% EtOAc gradient in hexane) to obtain of tert-butyl 4-{5-[(3-chloro-1-methyl-1H-pyrazol-4-yl)carbamoyl]pyridin-2-yl}-2-(trifluoromethyl)piperazine-1-carboxylate (**Int. 29**, 200 mg, 0.409 mmol, 57%, m/z [M+H]⁺: 490.00).

¹H NMR (400 MHz, DMSO-*d*₆) δ 9.65 (s, 1H), 8.70 (d, *J*= 2.4 Hz, 1H), 8.07 (dd, *J* = 9.0, 2.5 Hz, 1H), 8.00 (s, 1H), 6.93 (d, *J* = 9.1 Hz, 1H), 4.83 (d, *J* = 14.7 Hz, 2H), 4.48 (s, 1H), 4.29 (s, 1H), 4.00 (s, 1H), 3.82 (s, 3H), 3.44 (d, *J=* 13.7 Hz, 1H), 3.16 (d, *J=* 13.7 Hz, 1H), 3.10 (s, 1H), 2.88 (d, *J=* 12.1 Hz, 1H), 1.43 (d, *J=* 14.4 Hz, 9H).

¹⁹F NMR (377 MHz, DMSO-*d*₆) δ -68.14, -70.03 (d, *J=* 8.6 Hz).

### tert-butyl 4-{5-[(3-chloro-1-methyl-1H-pyrazol-4-yl)({4-chloro-1H,3H-furo[3,4-c]quinolin-7-yl}methyl)carbamoyl]pyridin-2-yl}-2-(trifluoromethyl)piperazine-1-carboxylate (Int. 30)

A mixture of tert-butyl 4-{5-[(3-chloro-1-methyl-1H-pyrazol-4-yl)carbamoyl]pyridin-2-yl}-2-(trifluoromethyl)piperazine-1-carboxylate (**Int**. **29,** 0.19 g, 0.381 mmol), 4-chloro-7-(chloromethyl)-1H,3H-furo[3,4-c]quinoline (**Int. A,** 0.098 g, 0.380 mmol), NaI (0.057 g, 0.38 mmol) and Cs₂CO₃ (0.149 g, 0.457 mmol) in MeCN (2 mL) was stirred at 80 °C overnight. The reaction mixture was diluted with EtOAc and poured into water. The organic layers were separated and the water layer was extracted with EtOAc several times. The combined organic layers were washed with brine and dried over anhydrous MgSO₄, filtered and evaporated. The crude product was purified by FCC (0% to 60% EtOAc gradient in hexane) to obtain tert-butyl 4-{5-[(3-chloro-1-methyl-1H-pyrazol-4-yl)({4-chloro-1H,3H-furo[3,4-c]quinolin-7-yl}methyl)carbamoyl]pyridin-2-yl}-2-(trifluoromethyl)piperazine-1-carboxylate (**Int. 30,** 127 mg, 0.180 mmol, 42%, m/z [M+H]⁺: 706.70).

¹H NMR (400 MHz, DMSO-*d*₆) δ 8.18 (d, J = 2.4 Hz, 1H), 7.96 - 7.85 (m, 2H), 7.83 (d, J = 8.4 Hz, 1H), 7.66 (dd, J = 8.5, 1.7 Hz, 1H), 7.58 (dd, J = 9.0, 2.5 Hz, 1H), 6.74 (d, J = 9.0 Hz, 1H), 5.53 (t, J = 3.1 Hz, 2H), 5.22 (t, J = 3.0 Hz, 2H), 5.07 (s, 2H), 4.93 - 4.55 (m, 2H), 4.25 - 4.03 (m, 1H), 4.07 - 3.77 (m, 1H), 3.68 (s, 3H), 3.46 - 3.22 (m, 1H), 3.25 - 2.87 (m, 2H), 1.42 (s, 9H).

¹⁹F NMR (377 MHz, DMSO-*d*₆) δ -70.08 (d, J = 8.8 Hz).

### tert-Butyl 4-{5-[(3-chloro-1-methyl-1H-pyrazol-4-yl)[(4-{[(2,4-dimethoxyphenyl)methyl]amino}-1H,3H-furo[3,4-c]quinolin-7-yl)methyl]carbamoyl]pyridin-2-yl}-2-(trifluoromethyl)piperazine-1-carboxylate (Int. 31)

A solution of tert-butyl 4-{5-[(3-chloro-1-methyl-1H-pyrazol-4-yl)({4-chloro-1H,3H-furo[3,4-c]quinolin-7-yl}methyl)carbamoyl]pyridin-2-yl}-2-(trifluoromethyl)piperazine-1-carboxylate (**Int. 30,** 0.120 g, 0.151 mmol, 1.0 eq.) and 1-(2,4-dimethoxyphenyl)methanamine (0.11 mL, 0.76 mmol, 5 eq.) in DMSO (2 mL) was stirred at 120°C for 8 h. The reaction mixture was diluted with DCM and poured into a saturated solution of NaHCOs. The layers were separated and the water layer was extracted with DCM several times. The combined organic layers were washed with brine and dried over anhydrous MgSO₄, filtered and evaporated. The product was isolated by FCC (0% to 5% MeOH gradient in DCM) to obtain tert-butyl 4-{5-[(3-chloro-1-methyl-1H-pyrazol-4-yl)[(4-{[(2,4-dimethoxyphenyl)methyl]amino}-1H,3H-furo[3,4-c]quinolin-7-yl)methyl]carbamoyl]pyridin-2-yl}-2-(trifluoromethyl)piperazine-1-carboxylate (**Int. 31,** 120 mg, 0.143 mmol, 86%, m/z [M+H]⁺: 836.30).

¹H NMR (400 MHz, DMSO-*d*₆) δ 8.16 (d, *J=* 2.4 Hz, 1H), 7.77 (s, 1H), 7.56 (dd, *J=* 9.1, 2.4 Hz, 1H), 7.47 - 7.40 (m, 2H), 7.20 - 7.11 (m, 2H), 7.00 (t, *J* = 5.7 Hz, 1H), 6.73 (d, *J* = 9.0 Hz, 1H), 6.56 (d, *J=* 2.4 Hz, 1H), 6.43 (dd, *J=* 8.4, 2.4 Hz, 1H), 5.31 (d, *J=* 3.7 Hz, 2H), 5.04 (s, 2H), 4.95 (s, 2H), 4.66 (d, *J=* 14.7 Hz, 1H), 4.57 (d, *J=* 5.6 Hz, 2H), 4.16 (m, 2H), 3.95 (m, 2H), 3.83 (s, 3H), 3.69 (d, *J* = 26.8 Hz, 6H), 3.01 (d, *J=* 10.0 Hz, 2H), 1.43 (s, 9H).

¹⁹F NMR (377 MHz, DMSO-*d*₆) δ -70.07 (d, *J=* 8.7 Hz).

### N-({4-Amino-1H,3H-furo[3,4-c]quinolin-7-yl}methyl)-N-(3-chloro-1-methyl-1H-pyrazol-4-yl)-6-[3-(trifluoromethyl)piperazin-1-yl]pyridine-3-carboxamide (Example 7)

To a solution of tert-butyl 4-{5-[(3-chloro-1-methyl-1H-pyrazol-4-yl)[(4-{[(2,4-imethoxyphenyl)methyl]amino}-1H,3H-furo[3,4-c]quinolin-7-yl)methyl]carbamoyl]pyridin-2-yl}-2-(trifluoromethyl)piperazine-1-carboxylate (**Int. 31**, 0.120 g, 0.130 mmol, 1.0 eq.) in DCM (1.5 mL), TFA (0.5 mL, 6.52 mmol, 50 eq.) was added. Then, the reaction mixture was stirred at RT overnight. The reaction mixture was diluted with DCM, poured into water and a solution of NaOH 0.5 M was added until to get a basic pH. The organic layer was separated. The water phase was extracted three times with DCM. The combined organic layers were washed with a saturated solution of NaHCOs, brine and dried over MgSO₄, filtered and evaporated. The crude product was purified by preparative HPLC to obtain after lyophilization N-({4-amino-1H,3H-furo[3,4-c]quinolin-7-yl}methyl)-N-(3-chloro-1-methyl-1H-pyrazol-4-yl)-6-[3-(trifluoromethyl)piperazin-1-yl]pyridine-3-carboxamide **(Example 7,** 0.005 g, 0.003 mmol, Y=6%, white solid, UPLC long elution (polar long method, buffer type "FA") purity 99.63%).

¹H NMR (400 MHz, DMSO-*d*₆) δ 8.16 (d, J = 2.4 Hz, 1H), 7.78 (s, 1H), 7.55 (dd, J = 9.0, 2.5 Hz, 1H), 7.49 - 7.36 (m, 2H), 7.14 (dd, J = 8.1, 1.7 Hz, 1H), 6.75 (d, J = 9.0 Hz, 1H), 6.46 (s, 2H), 5.29 (t, J = 3.3 Hz, 2H), 4.99 (t, J = 3.4 Hz, 2H), 4.94 (s, 2H), 4.35 (dd, J = 12.5, 3.5 Hz, 1H), 3.97 - 3.82 (m, 1H), 3.67 (s, 3H), 3.50 - 3.27 (m, 1H), 3.09 - 2.87 (m, 4H), 2.77 - 2.62 (m, 1H). ¹⁹F NMR (377 MHz, DMSO-*d*₆) δ -74.05 (d, J = 7.7 Hz).

### Example 8: N-({4-Amino-1H,3H-furo[3,4-c]quinolin-7-yl}methyl)-2-cyclopropyl-N-(3-methoxy-1H-pyrazol-4-yl)pyrimidine-5-carboxamide

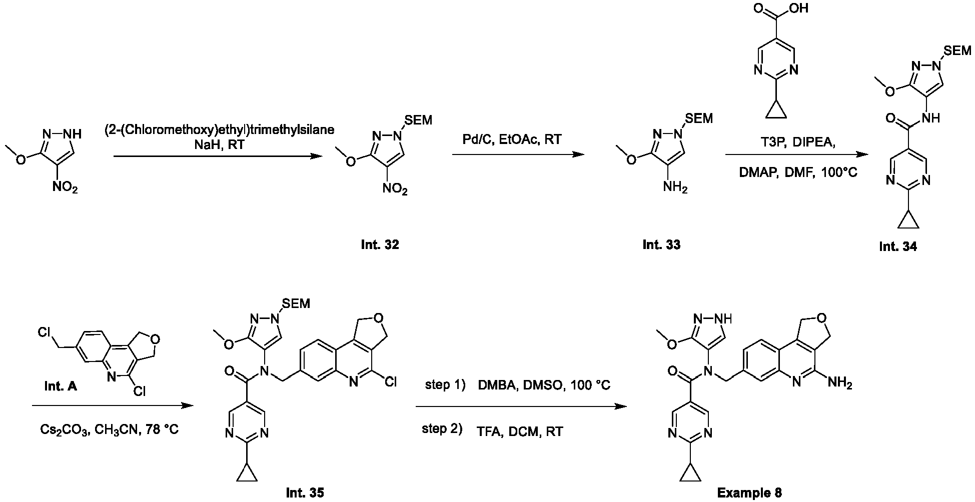

### 3-Methoxy-4-nitro-1-{[2-(trimethylsilyl)ethoxy]methyl}-1H-pyrazole (Int. 32)

A solution of 5-methoxy-4-nitro-1H-pyrazole (1.0 g, 6.988 mmol, 1.0 eq.) in DMF (30.0 mL) at 0°C, NaH (60% in oil) (0.537 g, 14.015 mmol, 2.0 eq.) was added, the reaction mixture was stirred at RT for 1 h. Next [2(chloromethoxy)ethyl]trimethylsilane (1.4 g, 8.397 mmol, 1.202 eq.) was added and the reaction mixture was stirred at RT overnight. The reaction mixture was diluated with water and extracted with EtOAc. The organic layers were washed with brine, dried over Na₂SO₄ and evaporated. The crude product was purified by FCC (silica, 0% to 20% EtOAc gradient in heptane) to give 3-methoxy-4-nitro-1-{[2 (trimethylsilyl)ethoxy]methyl}-1H-pyrazole (**Int. 32**, 0.697 g, 2.346 mmol, 34%, yellow oil).

¹H NMR (400 MHz, DMSO-*d*₆) δ 8.93 (s, 1H), 5.32 (s, 2H), 3.96 (s, 3H), 3.60 (t, 2H), 0.87 (t, 2H), -0.02 (s, 9H).

### 3-Methoxy-1-{[2-(trimethylsilyl)ethoxy]methyl}-1H-pyrazol-4-amine (Int. 33)

A mixture of 3-methoxy-4-nitro-1-{[2-(trimethylsilyl)ethoxy]methyl}-1H-pyrazole (**Int. 32**, 0.64 g, 2.154 mmol, 1.0 eq.) in EtOAc (30.0 mL) was sparged with nitrogen and palladium on carbon (0.071 g, 0.667 mmol, 0.31 eq.) was added. The reaction mixture was hydrogenated in a Parr apparatus (4 bar) at room temperature overnight. The reaction mixture was filtered through Celite^{®}, washed with isopropanol and concentrated. The crude product was purified by FCC (0% to 5% MeOH gradient in DCM) to obtain 3-methoxy-1-{[2-(trimethylsilyl)ethoxy]methyl}-1H-pyrazol-4-amine (**Int.** 33, 0.314 g, 0.671 mmol, 31%, dark red oil, m/z [M+H]⁺: 244.6).

¹H NMR (400 MHz, DMSO-*d*₆) δ 7.04 (s, 1H), 5.06 (s, 2H), 3.77 (s, 3H), 3.53 - 3.49 (m, 2H), 3.45 (t, 2H), 0.80 (t, 2H), -0.03 (s, 9H).

### 2-Cyclopropyl-N-(3-methoxy-1-{[2-(trimethylsilyl)ethoxy]methyl}-1H-pyrazol-4-yl)pyrimidine-5-carboxamide (Int. 34)

DMAP (0.034 g, 0.278 mmol, 0.521 eq.), DIPEA (0.3 mL, 1.722 mmol, 3.224 eq.), 2-cyclopropylpyrimidine-5-carboxylic acid (0.09 g, 0.548 mmol, 1.026 eq.) and 3-methoxy-1-{[2-(trimethylsilyl)ethoxy]methyl}-1H-pyrazol-4-amine (**Int. 33**, 0.25 g, 0.534 mmol, 1.0 eq.) were taken in DMF (2.0 mL) and T3P as 50% solution in EtOAc (0.5 mL, 1.68 mmol, 3.145 eq.) was added dropwise at 0°C. Then, the reaction mixture was stirred at 100°C for 1 h. The mixture was diluted with water and extracted with EtOAc. The organic layers were washed with brine, dried over Na₂SO₄, filtered and evaporated. The crude product was purified by FCC (silica, 0% to 5% MeOH gradient in DCM) to give 2-cyclopropyl-N-(3-methoxy-1-{[2-(trimethylsilyl)ethoxy]methyl}-1H-pyrazol-4-yl)pyrimidine-5-carboxamide (**Int. 34**, 0.221 g, 0.511 mmol, 96%, brown solid, m/z [M+H]⁺: 390.5).

¹H NMR (400 MHz, DMSO-*d*₆) δ 10.16 (s, 1H), 9.05 (s, 2H), 8.10 (s, 1H), 5.25 (s, 2H), 3.88 (s, 3H), 3.54 (t, J = 7.5 Hz, 2H), 2.34 - 2.23 (m, 1H), 1.20 - 1.12 (m, 2H), 1.11 - 1.02 (m, 2H), 0.85 (t, 2H), -0.02 (s, 9H).

### N-({4-Chloro-1H,3H-furo[3,4-c]quinolin-7-yl}methyl)-2-cyclopropyl-N-(3-methoxy-1-{[2-(trimethylsilyl)ethoxy]methyl}-1H-pyrazol-4-yl)pyrimidine-5-carboxamide (Int. 35)

To a solution of 4-chloro-7-(chloromethyl)-1H,3H-furo[3,4-c]quinoline (**Int. A**, 0.168 g, 0.655 mmol, 1.318 eq.) and 2-cyclopropyl-N-(3-methoxy-1-{[2-(trimethylsilyl)ethoxy]methyl}-1H-pyrazol-4-yl)pyrimidine-5-carboxamide (**Int. 34**, 0.215 g, 0.497 mmol, 1.0 eq.) in MeCN (2.5 mL) was added Cs₂CO₃ (0.328 g, 1.007 mmol, 2.027 eq.). The reaction mixture was stirred at 78°C for 1 h. The reaction mixture was diluted with a saturated solution of Na₂CO₃, extracted with EtOAc (2x) and the combined organic layers were washed with brine and dried over Na₂SO₄, filtered and evaporated. The crude product was purified by FCC (0% to 5% MOH gradient in DCM) to give N-({4-chloro-1H,3H-furo[3,4-c]quinolin-7-yl}methyl)-2-cyclopropyl-N-(3-methoxy-1-{[2-(trimethylsilyl)ethoxy]methyl}-1H-pyrazol-4-yl)pyrimidine-5-carboxamide (**Int. 35**, 0.26 g, 0.403 mmol, 81%, white solid, m/z [M+H]⁺: 608.2).

¹H NMR (400 MHz, DMSO-*d*₆) δ 8.60 (s, 2H), 7.91 (s, 1H), 7.84 (d, J = 8.4 Hz, 1H), 7.73 (s, 1H), 7.67 (dd, J = 8.5, 1.6 Hz, 1H), 5.54 (t, J = 3.2 Hz, 2H), 5.22 (t, J = 3.1 Hz, 2H), 5.09 (s, 2H), 5.04 (s, 2H), 3.59 (s, 3H), 3.17 (t, J = 7.9 Hz, 2H), 2.21 - 2.13 (m, 1H), 1.12 - 1.04 (m, 2H), 1.00 - 0.94 (m, 2H), 0.67 (t, J = 7.9 Hz, 2H), -0.13 (s, 9H).

### N-({4-Amino-1H,3H-furo[3,4-c]quinolin-7-yl}methyl)-2-cyclopropyl-N-(3-methoxy-1H-pyrazol-4-yl)pyrimidine-5-carboxamide (Example 8)

### Step 1:

To a solution of N-({4-chloro-1H,3H-furo[3,4-c]quinolin-7-yl}methyl)-2-cyclopropyl-N-(3-methoxy-1-{[2-(trimethylsilyl)ethoxy]methyl}-1H-pyrazol-4-yl)pyrimidine-5-carboxamide (**Int. 35**, 0.255 g, 0.395 mmol, 1.0 eq.) in DMSO (2.0 mL) 1-(2,4-dimethoxyphenyl)methanamine (0.7 g, 4.186 mmol, 10.605 eq.) was added. The reaction was stirred at 100°C overnight. The reaction was cooled down to room temperature, diluted with water, extracted with EtOAc (2x) and the combined organic layers were washed with brine and dried over Na₂SO₄, filtered and evaporated. The crude product was purified by FCC (0% to 20% MeCN gradient in DCM).

### Step 2:

To a solution of the product in DCM (2.0 mL), trifluoroacetic acid (1.6 mL, 20.908 mmol, 52.962 eq.) was added at 0°C. The reaction was stirred at room temperature overnight. Then, the reaction mixture was diluted with DCM and quenched with a saturated solution of Na₂CO₃. The crude product was extracted with DCM and then organic phases were combined, dried over Na₂SO₄, filtered and evaporated. The crude product was purified by FCC (0% to 10% MeOH gradient in DCM) and next by preparative HPLC (basic conditions) to give: N-({4-amino-1H,3H-furo[3,4-c]quinolin-7-yl}methyl)-2-cyclopropyl-N-(3-methoxy-1H-pyrazol-4-yl)pyrimidine-5-carboxamide (0.03 g, 0.066 mmol, 53%, white solid, UPLC long elution (polar long method, buffer type "FA") purity 99.99%, m/z [M+H]⁺: 458.4).

¹H NMR (400 MHz, DMSO-*d*₆) δ 11.92 (s, 1H), 8.54 (s, 2H), 7.44 (d, J = 8.2 Hz, 1H), 7.42 - 7.36 (m, 2H), 7.14 (dd, J = 8.2, 1.8 Hz, 1H), 6.46 (s, 2H), 5.30 (t, J = 3.3 Hz, 2H), 4.99 (t, J = 3.4 Hz, 2H), 4.95 (s, 2H), 3.56 (s, 3H), 2.21 - 2.11 (m, 1H), 1.12 - 1.03 (m, 2H), 1.00 - 0.91 (m, 2H).

### Example 9: N-({4-Amino-1H,3H-furo[3,4-c]quinolin-7-yl}methyl)-N-(3-methoxy-1-methyl-1H-pyrazol-4-yl)-6-[2-(trifluoromethyl)piperazin-1-yl]pyridine-3-carboxamide

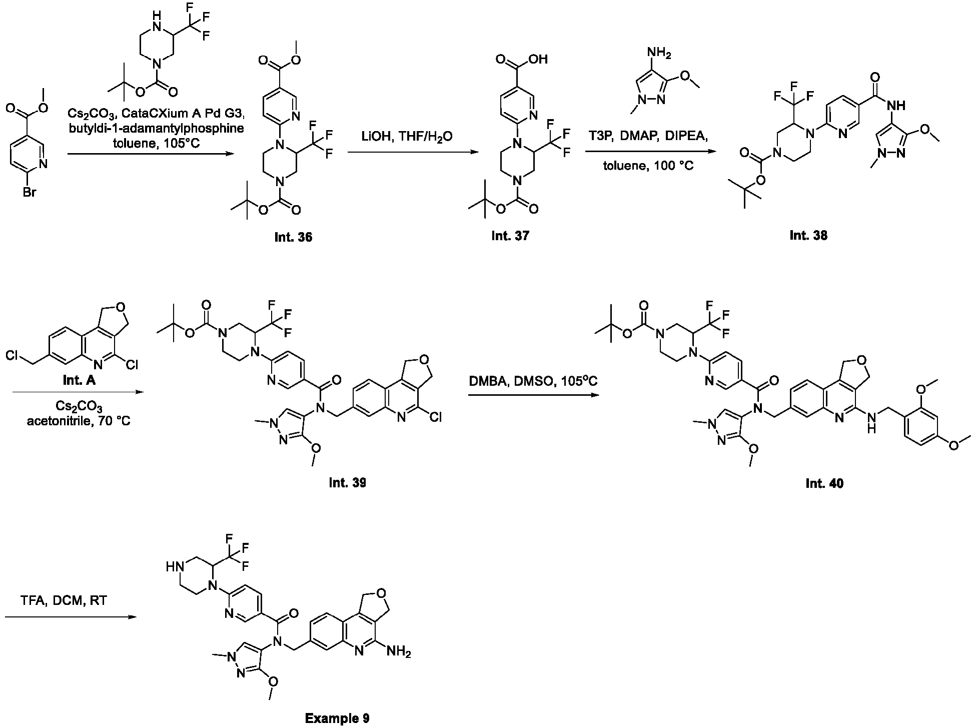

### tert-Butyl 4-[5-(methoxycarbonyl)pyridin-2-yl]-3-(trifluoromethyl)piperazine-1-carboxylate (Int. 36)

A mixture of methyl 6-bromopyridine-3-carboxylate (0.51 g, 2.361 mmol, 2.001 eq.), tert-butyl 3-(trifluoromethyl)piperazine-1-carboxylate (0.3 g, 1.18 mmol, 1.0 eq.), bis(adamantan-1-yl)(butyl)phosphane (0.051 g, 0.142 mmol, 0.121 eq.) and Cs₂CO₃ (1.153 g, 3.539 mmol, 2.999 eq.) in toluene (11.0 mL) was purge with Ar for 15 min. Then, cataCXium A Pd G3 (0.086 g, 0.118 mmol, 0.1 eq.) was added and the reaction mixture was stirred at 105°C for 20 h. Then, the reaction mixture was cooled down to RT, EtOAc was added and the mixture was filtered through a pad of Celite^{®} and the filtrate was concentrate. The residue was purified by FCC (0% to 60% EtOAc gradient in hexane) to obtain tert-butyl 4-[5-(methoxycarbonyl)pyridin-2-yl]-3-(trifluoromethyl)piperazine-1-carboxylate (**Int. 36**, 0.41 g, 0.863 mmol, 73%, light yellow solid).

¹H NMR (400 MHz, DMSO-*d*₆) δ 8.71 (d, J = 2.3 Hz, 1H), 8.09 (dd, J = 9.0, 2.4 Hz, 1H), 7.05 (d, J = 9.1 Hz, 1H), 5.65 - 5.50 (m, 1H), 4.32 (d, J = 14.8 Hz, 1H), 4.23 (d, J = 13.2 Hz, 1H), 4.12 - 3.90 (m, 1H), 3.82 (s, 3H), 3.45 - 2.88 (m, 3H), 1.42 (s, 9H).

¹⁹F NMR (377 MHz, DMSO \-*d*₆) δ -68.44 (d, J = 8.8 Hz), -68.82.

### 6-{4-[(tert-Butoxy)carbonyl]-2-(trifluoromethyl)piperazin-1-yl}pyridine-3-carboxylic acid (Int. 37)

To a solution of tert-butyl 4-[5-(methoxycarbonyl)pyridin-2-yl]-3-(trifluoromethyl)piperazine-1-carboxylate (**Int. 36**, 0.359 g, 0.756 mmol, 1.0 eq.) in THF (30 mL), a solution of LiOH monohydrate (0.089 g, 2.121 mmol, 2.806 eq.) in water (3 mL) was added and the reaction mixture was stirred at 55°C for 9 h. The reaction mixture was concentrated, diluted with water and extracted twice with EtOAc. The aqueous layer was adjusted to pH 3 with 1 M HCl, and the resulting precipitate was filtered, dried to obtain 6-{4-[(tert-butoxy)carbonyl]-2-(trifluoromethyl)piperazin-1-yl}pyridine-3-carboxylic acid (**Int. 37**, 0.3 g, 0.751 mmol, 99%, white solid, m/z [M+H]⁺: 376.1).

¹H NMR (400 MHz, DMSO-*d*₆) δ 12.77 (s, 1H), 8.68 (d, J = 2.3 Hz, 1H), 8.06 (dd, J = 8.9, 2.4 Hz, 1H), 7.02 (d, J = 9.0 Hz, 1H), 5.67 - 5.48 (m, 1H), 4.32 (d, J = 14.8 Hz, 1H), 4.26 - 4.18 (m, 1H), 4.12 - 3.87 (m, 1H), 3.25 - 2.86 (m, 3H), 1.42 (s, 9H).

¹⁹F NMR (377 MHz, DMSO) δ -68.42 (d, J = 8.9 Hz), -68.81.

### tert-Butyl 4-{5-[(3-methoxy-1-methyl-1H-pyrazol-4-yl)carbamoyl]pyridin-2-yl}-3-(trifluoromethyl)piperazine-1-carboxylate (Int. 38)

3-Methoxy-1-methyl-1H-pyrazol-4-amine hydrochloride (0.122 g, 0.746 mmol, 0.999 eq.), 6-{4-[(tert-butoxy)carbonyl]-2-(trifluoromethyl)piperazin-1-yl}pyridine-3-carboxylic acid (**Int. 37**, 0.298 g, 0.746 mmol, 1.0 eq.) and DMAP (0.004 g, 0.033 mmol, 0.044 eq.) were taken in toluene (20 mL), followed by an addition of DIPEA (0.45 mL, 2.583 mmol, 3.461 eq.). Then T3P as 50% solution in EtOAc (0.89 mL, 1.495 mmol, 2.003 eq.) was added slowly and the reaction mixture was stirred at 100°C for 2 h. Then, the reaction mixture was diluted with EtOAc and neutralized with a saturated solution of NaHCOs. The organic phase was washed with a saturated solution of NaHCOs, brine, dried over Na₂SO₄, filtered and evaporated to obtain tert-butyl 4-{5-[(3-methoxy-1-methyl-1H-pyrazol-4-yl)carbamoyl]pyridin-2-yl}-3-(trifluoromethyl)piperazine-1-carboxylate

(**Int. 38**, 0.383 g, 0.617 mmol, 83%, light yellow solid, m/z [M+H]⁺: 485.3).

¹H NMR (400 MHz, DMSO-*d*₆) δ 9.55 (s, 1H), 8.72 (d, J = 2.4 Hz, 1H), 8.15 (dd, J = 9.0, 2.5 Hz, 1H), 7.78 (s, 1H), 7.02 (d, J = 9.0 Hz, 1H), 5.61 - 5.51 (m, 1H), 4.32 (d, J = 14.6 Hz, 1H), 4.20 (d, J = 12.7 Hz, 1H), 4.12 - 3.91 (m, 1H), 3.83 (s, 3H), 3.69 (s, 3H), 3.44 - 2.89 (m, 3H), 1.42 (s, 9H). ¹⁹F NMR (377 MHz, DMSO-*d*₆) δ -68.14 - -68.42, -68.60 - -68.83.

### tert-Butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-3-(trifluoromethyl)piperazine-1-carboxylate (Int. 39)

To tert-butyl 4-{5-[(3-methoxy-1-methyl-1H-pyrazol-4-yl)carbamoyl]pyridin-2-yl}-3-(trifluoromethyl)piperazine-1-carboxylate (**Int. 38**, 0.38 g, 0.612 mmol, 1.0 eq.) in MeCN (40 mL) cesium carbonate (0.339 g, 1.04 mmol, 1.701 eq.) and potassium iodide (0.01 g, 0.06 mmol, 0.098 eq.) were added. The mixture was stirred for 10-15 min at RT, then 4-chloro-7-(chloromethyl)-1H,3H-furo[3,4-c]quinoline (**Int. A,** 0.209 g, 0.814 mmol, 1.331 eq.) was added and the reaction mixture was stirred at 70°C for 8 h. Then, the reaction mixture was cooled down, diluted with EtOAc, the organic phase was washed with water, brine, dried over Na₂SO₄, filtered and evaporated. The residue was purified by FCC (0% to 5% MeOH gradient in DCM) to obtain tert-butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-3-(trifluoromethyl)piperazine-1-carboxylate (**Int 39,** 0.232 g, 0.314 mmol, 51%, yellow solid, m/z [M+H]⁺: 702.6).

¹H NMR (400 MHz, DMSO-*d*₆) δ 8.23 (d, J = 2.4 Hz, 1H), 7.90 (d, J = 1.6 Hz, 1H), 7.83 (d, J = 8.4 Hz, 1H), 7.72 - 7.61 (m, 2H), 7.46 (s, 1H), 6.86 (d, J = 9.0 Hz, 1H), 5.54 (t, J = 3.1 Hz, 2H), 5.51 - 5.38 (m, 1H), 5.23 (t, J = 3.1 Hz, 2H), 5.03 (s, 2H), 4.28 (d, J = 14.7 Hz, 1H), 4.14 - 3.89 (m, 2H), 3.58 (s, 3H), 3.51 (s, 3H), 3.25 - 2.85 (m, 3H), 1.41 (s, 9H).

¹⁹F NMR (377 MHz, DMSO-*d*₆) δ -68.30, -68.70.

### tert-Butyl 4-(5-{[(4-{[(2,4-dimethoxyphenyl)methyl]amino}-1H,3H-furo[3,4-c]quinolin-7-yl)methyl](3-methoxy-1-methyl-1H-pyrazol-4-yl)carbamoyl}pyridin-2-yl)-3-(trifluoromethyl)piperazine-1-carboxylate (Int. 40)

A solution of tert-butyl 4-{5-[({4-chloro-1H,3H-furo[3,4-c]quinolin-7-yl}methyl)(3-methoxy-1-methyl-1H-pyrazol-4-yl)carbamoyl]pyridin-2-yl}-3-(trifluoromethyl)piperazine-1-carboxylate (**Int. 39**, 0.229 g, 0.31 mmol, 1.0 eq.) and 1-(2,4-dimethoxyphenyl)methanamine (0.389 g, 2.326 mmol, 7.508 eq.) in DMSO (6.0 mL) was stirred at 105°C for 38 h. Then, the reaction mixture was cooled down, diluted with EtOAc and the organic layer was washed four times with brine, dried over Na₂SO₄, filtered and evaporated. The residue was purified by FCC (0% to 5% MeOH gradient in DCM) to obtain tert-butyl 4-(5-{[(4-{[(2,4-dimethoxyphenyl)methyl]amino}-1H,3H-furo[3,4-c]quinolin-7-yl)methyl](3-methoxy-1-methyl-1H-pyrazol-4-yl)carbamoyl}pyridin-2-yl)-3-(trifluoromethyl)piperazine-1-carboxylate (**Int. 40,** 0.183 g, 0.202 mmol, 65%, yellow solid, m/z [M+H]⁺: 833.9).

¹H NMR (400 MHz, DMSO-*d*₆) δ 8.19 (d, J = 2.4 Hz, 1H), 7.62 (dd, J = 8.9, 2.4 Hz, 1H), 7.48 - 7.37 (m, 2H), 7.35 (s, 1H), 7.19 - 7.09 (m, 2H), 6.99 (t, J = 5.7 Hz, 1H), 6.84 (d, J = 8.9 Hz, 1H), 6.57 (d, J = 2.4 Hz, 1H), 6.43 (dd, J = 8.4, 2.4 Hz, 1H), 5.51 - 5.39 (m, 1H), 5.34 - 5.28 (m, 2H), 5.08 - 5.02 (m, 2H), 4.90 (s, 2H), 4.58 (d, J = 5.7 Hz, 2H), 4.28 (d, J = 14.6 Hz, 1H), 4.13 - 3.87 (m, 2H), 3.83 (s, 3H), 3.73 (s, 3H), 3.56 (s, 3H), 3.49 (s, 3H), 3.27 - 2.84 (m, 3H), 1.41 (s, 9H).

¹⁹F NMR (377 MHz, DMSO-*d*₆) δ -68.29, -68.70.

### N-({4-Amino-1H,3H-furo[3,4-c]quinolin-7-yl}methyl)-N-(3-methoxy-1-methyl-1H-pyrazol-4-yl)-6-[2-(trifluoromethyl)piperazin-1-yl]pyridine-3-carboxamide (Example 9)

To a solution of tert-butyl 4-(5-{[(4-{[(2,4-dimethoxyphenyl)methyl]amino}-1H,3H-furo[3,4-c]quinolin-7-yl)methyl](3-methoxy-1-methyl-1H-pyrazol-4-yl)carbamoyl}pyridin-2-yl)-3-(trifluoromethyl)piperazine-1-carboxylate (**Int. 40,** 0.18 g, 0.199 mmol, 1.0 eq.) in DCM (10 mL) and TFA (2.5 mL, 32.647 mmol, 164.197 eq.) was added. The reaction mixture was stirred at RT for 16 h. Then, the reaction mixture was diluted with DCM (80 mL) and carefully basified with a saturated solution of NaHCOs. After 30 minutes of vigorous stirring the organic layer was separated, washed carefully with a saturated solution of NaHCOs, dried over anhydrous Na₂SO₄, filtered and evaporated. The crude was purified by preparative HPLC (basic conditions) and lyophilized to obtain N-({4-amino-1H,3H-furo[3,4-c]quinolin-7-yl}methyl)-N-(3-methoxy-1-methyl-1H-pyrazol-4-yl)-6-[2-(trifluoromethyl)piperazin-1-yl]pyridine-3-carboxamide **(Example 9,** 0.051 g, 0.088 mmol, 44%, white solid, UPLC long elution (Polar long method, buffer type "FA") purity 99.99%, m/z [M+H]⁺: 583.30).

¹H NMR (400 MHz, DMSO-*d*₆) δ 8.17 (d, J = 2.4 Hz, 1H), 7.57 (dd, J = 9.0, 2.5 Hz, 1H), 7.47 - 7.39 (m, 2H), 7.33 (s, 1H), 7.13 (dd, J = 8.2, 1.7 Hz, 1H), 6.79 (d, J = 9.0 Hz, 1H), 6.45 (s, 2H), 5.35 - 5.22 (m, 3H), 5.00 (t, J = 3.4 Hz, 2H), 4.96 - 4.83 (m, 2H), 3.95 (d, J = 12.9 Hz, 1H), 3.60 (s, 3H), 3.50 (s, 3H), 3.25 (d, J = 13.7 Hz, 1H), 3.05 (t, J = 12.5 Hz, 1H), 2.97 (d, J = 12.6 Hz, 1H), 2.87 (d, J = 13.5 Hz, 1H), 2.64 - 2.54 (m, 1H), 2.41 - 2.24 (m, 1H).

¹⁹F NMR (377 MHz, DMSO-*d*₆) δ -66.38 (d, J = 8.7 Hz).

### Biological assays and data

As stated above, the compounds of the present invention are PRMT5 modulators and are useful in treating diseases by PRMT5 activity regulation. The biological activity of the compounds of the present invention can be determined by any appropriate test to determine the activity of the compound as PRMT5 modulator, as well as cell lines and *in vivo* models, in particular in presence or absence of MTA as exemplified below.

### Biochemical assay for recombinant PRMT5/MEP50 complex in presence or absence of MTA

Four mixes were prepared on ice:
Mix 1 - containing 3x concentrated PRMT5/MEP50 enzyme (in-house production) in 1x buffer (50 mM TRIS pH 8.0, 50 mM KCI, 15 mM MgCl₂, 1 mM EDTA) including 3x carrier protein (BSA, final conc. 0.005%) and reducing agent (DTT, final conc. 1 mM)
Mix 2 - 1× buffer including 3x carrier protein and reducing agent
Mix 3 - containing 3x concentrated Histone H4 (*N*-terminal Peptide, Biotinylated; EpiCypher, #12-0029) in 1× buffer;
Mix 4 - containing 3x concentrated SAM in 1x buffer.

Briefly, 5 µL of Mix 4 were added on a plate with 1% of DMSO or MTA at 0.8 µM (dispensed with Tecan D300e or Echo acoustic dispenser). Then using a MultiFlo FX dispenser, 5 µL of Mix 1 and Mix 2 were added to the appropriate wells and the plate was pre-incubated for 20 minutes at RT.

After that, the tested compounds at 10 concentrations, each one in duplicate, were added and normalized to 3% of DMSO (using a Tecan D300e or an Echo acoustic dispenser) and incubated again for 20 minutes at RT.

Then, 5 µL of Mix 3 were added to all the wells except the low control wells (buffer 1x was added instead to the low control wells). The plate was incubated at RT for 60 min. After 60 min, 5 µL of TFA were added to all wells using DragonFly Discovery liquid dispenser and the plate was incubated at RT for 5 minutes.

After 5 minutes, 5 µL of MTase-Glo Reagent (Promega #V7601 or #V7602) 1X was added using a MultiFlo FX dispenser and the plate was incubated at RT for 30 min. Next, 15 µL of MTaseGlo Detection Solution was added to all wells and the plate was incubated at RT for 30 min. Afterwards, the luminescence signal was recorded using plate reader.

The data were analyzed in GraphPad Prism^{®}. IC₅₀, Hill slope and efficacy parameters were determined by fitting a variable slope sigmoidal function.

### HCT116 MTAP Knockout model

The HCT116 cell line was purchased from PHE ECACC (Acc No: 91091005). The HCT116 MTAP KO cell line was generated using a CRISPR/Cas9 system and sgRNA targeting the MTAP gene. HCT116 cell line was co-transfected with MTAP CRISPR/Cas9 KO and MTAP HDR plasmids (Santa Cruz Biotechnology, sc-406223, and sc-406223-HDR, respectively), followed by puromycin selection. Knockout of the MTAP gene after clonal selection was validated using western blotting (Antibody Cell Sig., 4158). HCT116 WT and HCT116 MTAP knockout cells were propagated in RPMI 1640 medium supplemented with 10% FBS and 1 mM sodium pyruvate in a humidified 5% CO₂ tissue culture incubator.

### SDMA ELISA assay:

On Day 0, HCT116 parental and HCT116 MTAP KO cells were seeded in 96-well plate in RPMI 1640 medium containing 10% FBS and 1% sodium pyruvate and incubated overnight in a humidified, 5% CO₂ tissue culture incubator.

On Day 1, the cells were treated with DMSO vehicle control or a dose response of test compounds dispensed to wells at defined concentration using a Tecan D300e digital dispenser (n=2) normalized with DMSO.

On Day 4, 10 µl of alamarBlue reagent (amount equal to 5% of the well volume) were added to each well and the cells were incubated at 37 °C in a 5% CO₂ incubator for 5 h. The fluorescence intensity was measured with a plate reader at 540 nm excitation wavelength and 590 nm emission wavelength. The culture medium was removed and the cells were washed with PBS. Then the PBS was completely removed. 50 µL of Lysis Buffer (PathScan^{®} Sandwich ELISA Lysis Buffer diluted 2x in PBS including Halt Protease & Phosphatase Inhibitor Cocktail (100x), ThermoScientific) was added to each well and the plate was incubated on ice for 15 minutes followed by freezing of the plate at -80 °C.

On Day 5, the lysates were thawed, diluted, 100 µl was transferred to an ELISA plate (Thermo Scientific, 442404) and incubated for 2 h at RT. Next, the lysate was removed and each well was washed three times with 200 µL of the Wash Buffer (PBS with 0.05% Tween 20). 100 µL of the Blocking Buffer (PBS with 0.05% Tween 20 and 5% BSA) were added to each well and incubated for 2 h at RT at 250 rpm. Next the wells were washed three times with Wash Buffer and 40 µL of the SDMA antibody (Cell Signaling #13222S; 1:1000, Wash Buffer with 1% BSA) were added to each well. The plate was sealed and incubated on the rocking platform overnight at 4 °C.

On Day 6 antibodies were removed and each well was washed five times with 200 µL of the Wash Buffer. 40 µL of HRP-linked anti-rabbit IgG antibody (Cell Signaling #7074S; 1:2000, Wash Buffer with 1% BSA) were added to each well and the plate was incubated for 2 h at RT protected from light. Next, antibodies were removed and each well was washed five times with 200 µL of the Wash Buffer. 100 µL of Substrate Solution (Invitrogen 00-4201-56) were added to each well and plate was incubated for 10 min. at RT. Then, 50 µL of Stop Solution (aq. 1 M H₂SO₄) were added and the plate was mixed gently. The absorbance at 450 nm was measured. For data analysis, dose-response curves were determined and the relevant parameters were calculated (EC₅₀, HillSlope, efficacy and %effect for the 2 highest tested compound concentrations) by fitting a variable-slope sigmoidal function [EC₅₀ inhibition; normalization: 0% POS CTL, 100% VEH CTL] using the GraphPad Prism^{®} software. Outliers were excluded from the graphs.

Biochemical (with and without MTA) and cellular potency (in HCT116-WT and MTAP KO cell lines)

Compounds are classified according to their IC₅₀/EC₅₀ values in the assays described above in three groups:
A = IC₅₀/EC₅₀ ≤ 0.1 µM; B = 0.1 µM < IC₅₀/EC₅₀ ≤ 1 µM; C = 30 µM ≥ IC₅₀/EC₅₀ > 1 µM

| Example No. | MTase-Glo | MTase-Glo + MTA | SDMA ELISA, HCT116-WT | SDMA ELISA, HCT116-KO |
|---|---|---|---|---|
| **Example 1** | A | A | A | B |
| **Example 2** | A | A | A | A |
| **Example 3** | A | A | A | A |
| **Example 4** | A | A | A | A |
| **Example 5** | A | A | A | A |
| **Example 6** | A | A | A | A |
| **Example 7** | A | A | A | A |
| **Example 8** | A | A | A | B |
| **Example 9** | | | A | A |

### References:

Blanc RS., Richard S. Arginine Methylation: The Coming of Age. Mol Cell. 2017 Jan 5;65(1):8-24. doi: 10.1016/j.molcel.2016.11.003.
Koh, C.M., Bezzi, M. & Guccione, E. The Where and the How of PRMT5. Curr Mol Bio Rep 1, 19-28 (2015). doi:10.1007/s40610-015-0003-5.
Wu Q, Schapira M, Arrowsmith CH, Barsyte-Lovejoy D. Protein arginine methylation: from enigmatic functions to therapeutic targeting. Nat Rev Drug Discov. 2021 Jul;20(7):509-530. doi: 10.1038/s41573-021-00159-8.
Gao J, Aksoy BA, Dogrusoz U, Dresdner G, Gross B, Sumer SO, Sun Y, Jacobsen A, Sinha R, Larsson E, Cerami E, Sander C, Schultz N. Integrative analysis of complex cancer genomics and clinical profiles using the cBioPortal. Sci Signal. 2013 Apr 2;6(269):pl1. doi: 10.1126/scisignal.2004088.
Marjon K, Cameron MJ, Quang P, Clasquin MF, Mandley E, Kunii K, McVay M, Choe S, Kernytsky A, Gross S, Konteatis Z, Murtie J, Blake ML, Travins J, Dorsch M, Biller SA, Marks KM. MTAP Deletions in Cancer Create Vulnerability to Targeting of the MAT2A/PRMT5/RIOK1 Axis. Cell Rep. 2016 Apr 19;15(3):574-587. doi: 10.1016/j.celrep.2016.03.043.
Firestone RS, Schramm VL. The Transition-State Structure for Human MAT2A from Isotope Effects. J Am Chem Soc. 2017 Oct 4;139(39):13754-13760. doi: 10.1021/jacs.7b05803.
Kryukov GV, Wilson FH, Ruth JR, Paulk J, Tsherniak A, Marlow SE, Vazquez F, Weir BA, Fitzgerald ME, Tanaka M, Bielski CM, Scott JM, Dennis C, Cowley GS, Boehm JS, Root DE, Golub TR, Clish CB, Bradner JE, Hahn WC, Garraway LA. MTAP deletion confers enhanced dependency on the PRMT5 arginine methyltransferase in cancer cells. Science. 2016 Mar 11;351(6278):1214-8. doi: 10.1126/science.aad5214.
Mavrakis KJ, McDonald ER 3rd, Schlabach MR, Billy E, Hoffman GR, deWeck A, Ruddy DA, Venkatesan K, Yu J, McAllister G, Stump M, deBeaumont R, Ho S, Yue Y, Liu Y, Yan-Neale Y, Yang G, Lin F, Yin H, Gao H, Kipp DR, Zhao S, McNamara JT, Sprague ER, Zheng B, Lin Y, Cho YS, Gu J, Crawford K, Ciccone D, Vitari AC, Lai A, Capka V, Hurov K, Porter JA, Tallarico J, Mickanin C, Lees E, Pagliarini R, Keen N, Schmelzle T, Hofmann F, Stegmeier F, Sellers WR. Disordered methionine metabolism in MTAP/CDKN2A-deleted cancers leads to dependence on PRMT5. Science. 2016 Mar 11;351(6278):1208-13. doi: 10.1126/science.aad5944.

## Claims

1. A compound of formula (la*) 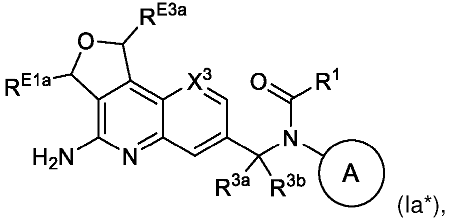 or a salt, stereoisomer, atropisomer, rotamer, tautomer, or N-oxide thereof; wherein
A is a moiety selected from 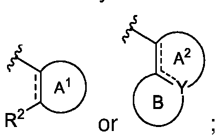
wherein
the wavy line in each case marks the connection to the N-atom of the remainder of the molecule;
and wherein
A¹ is a 5- or 6-membered saturated, partially or fully unsaturated, or aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned rings is independently unsubstituted or substituted with one or more, same or different substituents R^{A1};
A² is a 5- or 6-membered saturated, partially or fully unsaturated, or aromatic carbocyclic or heterocyclic ring, wherein the aforementioned heterocyclic ring comprises one or more, same or different heteroatoms selected from O, N, or S, wherein said N- and/or S-atoms are independently oxidized or non-oxidized, and wherein each substitutable carbon or heteroatom in the aforementioned rings is independently unsubstituted or substituted with one or more, same or different substituents R^{A2};
and wherein the dashed lines in the rings A¹ and A² denote that an additional bond may be present, so that a double bond is formed;
and wherein
B is a fused morpholine ring, wherein each substitutable carbon atom of said morpholine ring is independently unsubstituted or substituted with one or more, same or different substituents R^{B};
Y is C;
X³ is CH, CR^{X3}, or N;
R¹ is a 6-membered aromatic carbocyclyl or heterocyclyl, wherein the aforementioned heterocyclic ring comprises one or more, same or different heteroatoms selected from O, N, or S, wherein said N- and/or S-atoms are independently oxidized or non-oxidized, and wherein the aforementioned rings are independently unsubstituted or substituted with one or more, preferably one, same or different substituents R^{Y};
R² is halogen, CN, S(=O)₂R^{S}, S(=O)(=NH)R^{S}, C(=O)R^{C}, NHC(=O)R^{C}, C(=O)NR^{N4}R^{N5}, C₁-C₄-alkoxy, C₁-C₄-haloalkyl, C₁-C₄-haloalkoxy, C₁-C₆-hydroxyalkyl;
R^{3a} is H, or D;
R^{3b} is H, or D;
and wherein
R^{A1} is halogen, CN, OH, C₁-C₄-alkyl, C₁-C₄-alkoxy, C₁-C₄-haloalkyl, or 3- to 7-membered saturated carbocyclyl or heterocyclyl, wherein the aforementioned heterocyclyl ring comprises one or more, same or different heteroatoms selected from O, N, or S, wherein said N- and/or S-atoms are independently oxidized or non-oxidized; and wherein each substitutable carbon or heteroatom in the aforementioned rings is independently unsubstituted or substituted with one or more, same or different substituents R^{A3};
R^{A2} is halogen, CN, OH, C₁-C₄-alkyl, C₁-C₄-alkoxy, or C₁-C₄-haloalkyl;
R^{A3} is halogen, CN, OH, C₁-C₄-alkyl, C₁-C₄-alkoxy, or C₁-C₄-haloalkyl;
or two R^{A3} attached to the same atom form cyclopropyl;
R^{B} is halogen, CN, OH, C₁-C₄-alkyl, or C₁-C₄-haloalkyl;
or two R^{B} attached to the same atom form an oxo group;
R^{C} is H, or C₁-C₄-alkyl;
R^{E1a} is H, C₁-C₂-alkyl, or C₁-C₂-haloalkyl;
R^{E3a} is H, C₁-C₂-alkyl, or C₁-C₂-haloalkyl;
R^{N4} is H, or C₁-C₄-alkyl;
R^{N5} is H, or C₁-C₄-alkyl;
R^{S} is C₁-C₄-alkyl, C₁-C₄-alkoxy, C₁-C₄-haloalkyl, C₃-C₅-cycloalkyl, or NR^{N4}R^{N5};
R^{X3} is halogen;
R^{Y} is halogen, CN, OH, NR^{N4}R^{N5}, C₁-C₄-alkyl, C₁-C₄-alkoxy, C₁-C₄-haloalkyl, C₁-C₄-haloalkoxy, C₁-C₄-hydroxyalkyl, C₁-C₄-alkoxy-C₁-C₄-alkoxy, or 3- to 10-membered saturated, partially or fully unsaturated, or aromatic carbocyclyl, carbocyclyl-C₁-C₂-alkyl, carbocyclyloxy, heterocyclyl, heterocyclyloxy, or heterocyclyl-C₁-C₂-alkyl, or 5- to 10-membered saturated carbobicyclyl or hetereobicyclyl, wherein the aforementioned heterocyclyl or heterobicyclyl rings comprise one or more, same or different heteroatoms selected from O, N, or S, wherein said N- and/or S-atoms are independently oxidized or non-oxidized, and wherein each substitutable carbon or heteroatom in the aforementioned groups is independently unsubstituted or substituted with one or more, same or different substituents R^{Z};
or two R^{Y} attached to the same atom form an oxo group;
or two R^{Y} together with the atoms to which they are bonded form a fused 5- or 6-membered saturated, partially or fully unsaturated, or aromatic carbocyclyl, or heterocyclyl, wherein the aforementioned heterocyclyl ring comprises one or more, same or different heteroatoms selected from O, N, or S, wherein said N- and/or S-atoms are independently oxidized or non-oxidized;
R^{Z} is halogen, CN, C₁-C₄-alkyl, C₁-C₄-haloalkyl, C₁-C₄-alkoxy, or 3- to 6-membered saturated carbocyclyl, carbocyclyloxy, heterocyclyl, or heterocyclyloxy, wherein the aforementioned heterocyclyl rings comprise one or more, same or different heteroatoms selected from O, N, or S, wherein said N- and/or S-atoms are independently oxidized or non-oxidized;
or two R^{Z} attached to the same atom form an oxo group or cyclopropyl;
with the proviso that one of the following i) to iv) is true:
i) A is 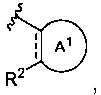 wherein A¹ is substituted with at least one substituent R^{A1}; wherein one of said at least one substituent R^{A1} is piperazinyl, piperidinyl, or 1,4-diazepane, wherein each substitutable carbon atom of the piperazinyl, piperidinyl, or 1,4-diazepane is independently unsubstituted or substituted with one or more, same or different substituents R^{A3};
ii) A is 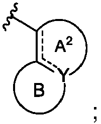 wherein
B is a fused morpholine ring, wherein each substitutable carbon atom of said morpholine ring is independently unsubstituted or substituted with one or more, same or different substituents R^{B};
iii) R¹ is substituted with at least one substituent R^{Y};
wherein one of said at least one substituent R^{Y} is piperazinyl or 1,4-diazepane, wherein each substitutable carbon atom of the piperazinyl or 1,4-diazepane is independently unsubstituted or substituted with one or more, same or different substituents R^{Z};
iv) A is

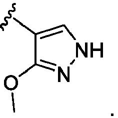

2. The compound according to claim 1, wherein
i) A is 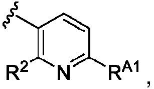 wherein
R^{A1} is piperazinyl, wherein each substitutable carbon atom of the piperazinyl is independently unsubstituted or substituted with one or more, same or different substituents RA3;
wherein preferably
R^{A3} is CF₃;
or
ii) A is 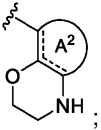 or
iii) R¹ is 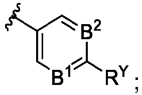 wherein
B¹ is N or CH;
B² is N or CH; and
R^{Y} is piperazinyl or 1,4-diazepane, wherein each substitutable carbon atom of the aforementioned piperazinyl or 1,4-diazepane is independently unsubstituted or substituted with one or more, same or different substituents R^{Z};
wherein preferably
R^{Z} is F or CF₃,
or two R^{Z} attached to the same carbon atom form cyclopropyl; or
iv) A is

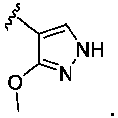

3. The compound according to claim 1 or 2, wherein the compound is a compound of formula (la*-1) 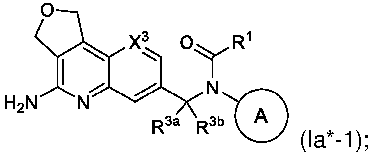 wherein
X³ is CH.

4. The compound according to any one of claims 1 to 3, wherein
R² is halogen, S(=O)₂R^{S}, C₁-C₂-alkoxy, or C₁-C₂-haloalkyl;
and wherein
R^{S} is C₁-alkyl.

5. The compound according to any one of claims 1 to 4, wherein
R² is Cl, CF₃, or OCH₃.

6. The compound according to any one of claims 1 to 5, wherein
A¹ is pyridinyl or pyrazolyl;
wherein the pyridinyl or pyrazolyl is independently unsubstituted or substituted with one or more, same or different, substituents R^{A1}; wherein
R^{A1} is C₁-C₂-alkyl or piperazinyl, piperidinyl, or 1,4-diazepane, wherein each substitutable carbon atom of the piperazinyl, piperidinyl, or 1,4-diazepane, is independently unsubstituted or substituted with one or more, same or different substituents R^{A3};
and
A² is unsubstituted phenyl; and
B is a fused unsubstituted morpholine ring, wherein each substitutable carbon atom of said morpholine ring is independently unsubstituted or substituted with one or more, same or different substituents R^{B}.

7. The compound according to any one of claims 1 to 6, wherein
A is a moiety selected from the group consisting of 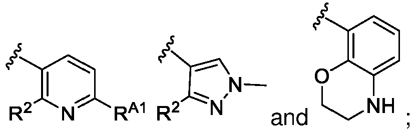
wherein
R^{A1} is piperazinyl, wherein each substitutable carbon atom of the piperazinyl is independently unsubstituted or substituted with one or more, same or different substituents R^{A3};
and wherein preferably
R^{A3} is CF₃.

8. The compound according to any one of claims 1 to 7, wherein
A is a moiety selected from the group consisting of

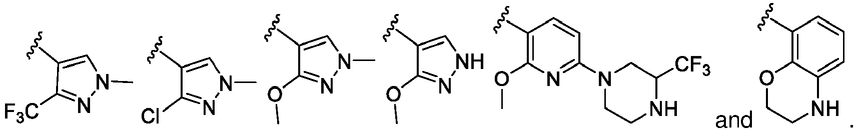

9. The compound according to any one of claims 1 to 8, wherein
R¹ is 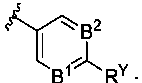
wherein
B¹ is N;
B² is CH or N; and
R^{Y} is F, CN, CF₃, cyclopropyl, piperazinyl, or 1,4-diazepane, wherein each substitutable carbon atom of the aforementioned piperazinyl or 1,4-diazepane is independently unsubstituted or substituted with one or more, same or different substituents R^{Z};
wherein
R^{Z} is F or CF₃,
or two R^{Z} attached to the same carbon atom form cyclopropyl.

10. The compound according to any one of claims 1 to 9, wherein
A is 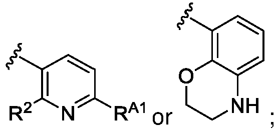
wherein
R^{A1} piperazinyl or 1,4-diazepane, wherein each substitutable carbon atom of the aforementioned piperazinyl or 1,4-diazepane is independently unsubstituted or substituted with one or more, same or different substituents R^{A3}; and
R¹ is 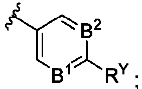
wherein
B¹ is CH or N;
B² is CH or N; and
R^{Y} is F, CN, CF₃, or cyclopropyl;
and wherein preferably
R^{A3} is CF₃;
or
A is 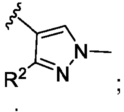 and
R¹ is 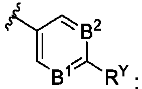
wherein
B¹ is CH or N;
B² is CH or N; and
R^{Y} piperazinyl or 1,4-diazepane, wherein each substitutable carbon atom of the aforementioned piperazinyl or 1,4-diazepane is independently unsubstituted or substituted with one or more, same or different substituents R^{Z};
wherein
R^{Z} is F or CF₃,
or two R^{Z} attached to the same carbon atom form cyclopropyl.

11. The compound according to any one of claims 1 to 10, wherein
R¹ is

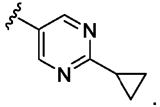

12. The compound according to any one of claims 1 to 10, wherein
R¹ is 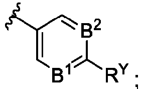
wherein
B¹ is N;
B² is CH; and
R^{Y} is piperazinyl or 1,4-diazepane, wherein each substitutable carbon atom of the aforementioned piperazinyl or 1,4-diazepane is independently unsubstituted or substituted with one or more, same or different substituents R^{Z};
wherein
R^{Z} is F or CF₃,
or two R^{Z} attached to the same carbon atom form cyclopropyl.

13. The compound according to any one of claims 1 to 12, wherein the compound of formula (I) is selected from the group consisting of:
N-({4-amino-1H,3H-furo[3,4-c]quinolin-7-yl}methyl)-N-[1-methyl-3-(trifluoromethyl)-1H-pyrazol-4-yl]-6-(piperazin-1-yl)pyridine-3-carboxamide;
N-({4-amino-1 H,3H-furo[3,4-c]quinolin-7-yl}methyl)-6-{4,7-diazaspiro[2.5]octan-7-yl}-N-[1-methyl-3-(trifluoromethyl)-1H-pyrazol-4-yl]pyridine-3-carboxamide;
N-({4-amino-1H,3H-furo[3,4-c]quinolin-7-yl}methyl)-2-cyclopropyl-N-{2-methoxy-6-[3-(trifluoromethyl)piperazin-1-yl]pyridin-3-yl}pyrimidine-5-carboxamide;
N-({4-amino-1H,3H-furo[3,4-c]quinolin-7-yl}methyl)-2-cyclopropyl-N-(3,4-dihydro-2H-1,4-benzoxazin-8-yl)pyrimidine-5-carboxamide;
N-({4-amino-1H,3H-furo[3,4-c]quinolin-7-yl}methyl)-N-(3-methoxy-1-methyl-1H-pyrazol-4-yl)-6-[3-(trifluoromethyl)piperazin-1-yl]pyridine-3-carboxamide;
N-({4-amino-1H,3H-furo[3,4-c]quinolin-7-yl}methyl)-6-(6,6-difluoro-1,4-diazepan-1-yl)-N-(3-methoxy-1-methyl-1H-pyrazol-4-yl)pyridine-3-carboxamide;
N-({4-amino-1H,3H-furo[3,4-c]quinolin-7-yl}methyl)-N-(3-chloro-1-methyl-1H-pyrazol-4-yl)-6-[3-(trifluoromethyl)piperazin-1-yl]pyridine-3-carboxamide;
N-({4-amino-1H,3H-furo[3,4-c]quinolin-7-yl}methyl)-2-cyclopropyl-N-(3-methoxy-1H-pyrazol-4-yl)pyrimidine-5-carboxamide; and
N-({4-amino-1H,3H-furo[3,4-c]quinolin-7-yl}methyl)-N-(3-methoxy-1-methyl-1H-pyrazol-4-yl)-6-[2-(trifluoromethyl)piperazin-1-yl]pyridine-3-carboxamide.

14. A pharmaceutical composition comprising a pharmaceutically effective amount of the compound according to any one of claims 1 to 13 and optionally a pharmaceutically acceptable carrier, diluent or excipient.

15. A compound according to any one of claims 1 to 13 or a pharmaceutical composition according to claim 14 for use in medicine.

16. A compound according to any one of claims 1 to 13 or a pharmaceutical composition according to claim 14 for use in the treatment of a disease selected from the group consisting of cancer and pre-cancerous syndromes.
